(19) Europäisches Patentamt
European Patent Office
Office européen des brevets

(11) EP 1 916 236 A1

(12) **EUROPEAN PATENT APPLICATION**
published in accordance with Art. 153(4) EPC

(43) Date of publication:
**30.04.2008 Bulletin 2008/18**

(21) Application number: **06766979.6**

(22) Date of filing: **20.06.2006**

(51) Int Cl.:
***C07C 237/42*** *(2006.01)* ***A01N 37/24*** *(2006.01)*
***A01N 41/10*** *(2006.01)* ***A61P 7/04*** *(2006.01)*
***C07C 317/40*** *(2006.01)* ***C07C 323/42*** *(2006.01)*
***C07D 213/82*** *(2006.01)*

(86) International application number:
**PCT/JP2006/312318**

(87) International publication number:
**WO 2006/137395 (28.12.2006 Gazette 2006/52)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **23.06.2005 JP 2005182854**
**12.07.2005 JP 2005203137**

(71) Applicant: **Mitsui Chemicals, Inc.**
**Tokyo 105-7117 (JP)**

(72) Inventors:
- **YOSHIDA, Kei, c/o Mitsui Chemicals, Inc. Mobara-shi, Chiba 297-0017 (JP)**
- **INOMATA, Shinichi, c/o Mitsui Chemicals, Inc. Mobara-shi, Chiba 297-0017 (JP)**
- **DAIDO, Hidenori, c/o Mitsui Chemicals, Inc. Mobara-shi, Chiba 297-0017 (JP)**
- **KAWAHARA, N., c/o Mitsui Chemicals Crop Life, Inc. Tokyo 103-0027 (JP)**
- **NOMURA, Michikazu, c/o Mitsui Chemicals, Inc. Mobara-shi, Chiba 297-0017 (JP)**
- **KOBAYASHI, Y., c/o Mitsui Chemicals Crop Life, Inc Mobara-shi, Chiba 297-0017 (JP)**

(74) Representative: **Stuart, Ian Alexander et al**
**Mewburn Ellis LLP**
**York House**
**23 Kingsway**
**London WC2B 6HP (GB)**

# (54) AMIDE DERIVATIVE, PESTICIDE CONTAINING SUCH COMPOUND AND USE THEREOF

(57) The present invention is to provide a compound represented by the general formula (1) exhibiting a high insecticidal effect and an insecticide comprising the compound as an active ingredient.

The compound represented by the general formula (1) and an insecticide comprising the compound as an active ingredient,

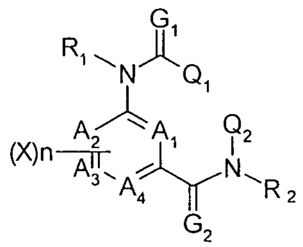

(1)

wherein, in the formula, $A_1$, $A_2$, $A_3$ and $A_4$ each represent a carbon atom or the like; $R_1$ and $R_2$ are each a hydrogen atom, an alkyl group or the like; $G_1$ and $G_2$ are each an oxygen atom or the like; X represents a hydrogen atom, a halogen atom or the like; n is an integer of 0 to 4; $Q_1$ represents a substituted phenyl group, a substituted heterocyclic group or the like; $Q_2$ represents a phenyl group having a haloalkyl sulfur group or the like, a heterocyclic group or the like.

EP 1 916 236 A1

**Description**

TECHNICAL FIELD

The present invention relates to a new amidederivative, an insecticide containing the compound as an active ingredient, an intermediate in the production of the compound, and method for applacation thereof as insecticide.

BACKGROUND ART

In WO 2000 / 55120 and US Patent No. 6548514, a compound similar to the compound of the present invention for medicine use was described. But activity against insects was never described therein and it is obvious that such compounds are not included in the scope of the claims of the present invention.

In WO 2000 / 7980, a compound similar to the compound of the present invention for medicine use was described. But activity against insects was never described therein and it is obvious that such a compound is not included in the scope of the claims of the present invention.

In US Patent Laid-Open No. 2002-032238, a compound similar to the compound of the present invention for medicine use was described. But activity against insects was never described therein. Furthermore, it is obvious that such a compound is not included in the scope of the claims of the present invention.

DISCLOSURE OF THE INVENTION

The present invention provides a new compound exhibiting a wide spectrum of insecticidal properties against insect pests of various crops and further having a high insecticidal effect on insect pests having a resistance which has recently become a serious problem as well, an insecticide containing the compound as an active ingredient, and an application method of the insecticide.

In order to solve the above objects, the present inventors have repeatedly conducted an extensive study and as a result, have found a new use of a compound of the present invention as an insecticide because the compound of the present invention is a novel compound which is not described in any documents and has an excellent insecticidal effect. The compound of the present invention is found not only to exhibit an insecticidal effect on various insect pests, but also an excellent insecticidal effect on insect pests that are difficult to control such as LEPIDOPTERA, THYSANOPTERA and the like which have become a worldwide problem. Furthermore, they have found a new preparation method and a usual intermediate for the production of the compound of the present invention. Thus, the present invention has been completed.

That is, the present invention is specified by the following matters.

[1] A compound represented by the general formula (1),

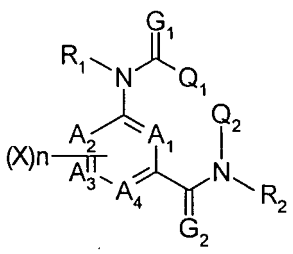

(1)

wherein, in the formula, $A_1$, $A_2$, $A_3$ and $A_4$ each independently represents a carbon atom, a nitrogen atom or an oxidized nitrogen atom;

$R_1$ and $R_2$ are each independently a hydrogen atom, a C1-C4 alkyl group or a C1-C4 alkylcarbonyl group;
$G_1$ and $G_2$ are each independently an oxygen atom or a sulfur atom;
Xs are each independently a hydrogen atom, a halogen atom or a trifluoromethyl group;
n is an integer of 0 to 4;
$Q_1$ is a phenyl group,
a substituted phenyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6

halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group),
a heterocyclic group (The heterocyclic group herein represents a pyridyl group, a pyridine N-oxide group, a pyrimidinyl group, a pyridazyl group, a pyrazyl group, a furyl group, a thienyl group, an oxazolyl group, an isoxazolyl group, an oxadiazolyl group, a thiazolyl group, an isothiazolyl group, an imidazolyl group, a triazolyl group, a pyrrole group, a pyrazolyl group or a tetrazolyl group.), or
a substituted heterocyclic group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a Cl-C3 haloalkylthio group, a Cl-C3 alkylsulfinyl group, a Cl-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group) (The heterocyclic group represents the same as those described above.); and
$Q_2$ is represented by the general formula (2) or (3),

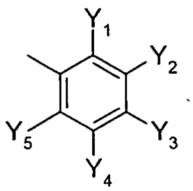

(2)

wherein, in the formula, $Y_1$ represents a C1-C4 alkoxy group, a C1-C4 haloalkoxy group, a C1-C3 haloalkylthio group, a C1-C3 haloalkylsulfinyl group or a C1-C3 haloalkylsulfonyl group; $Y_5$ represents a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_3$ represents a C2-C6 perfluoroalkyl group, a C1-C6 perfluoroalkylthio group, a C1-C6 perfluoroalkylsulfinyl group or a C1-C6 perfluoroalkylsulfonyl group; and $Y_2$ and $Y_4$ each independently represent a hydrogen atom, a halogen atom or a C1-C4 alkyl group; or

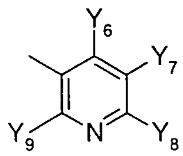

(3)

wherein, in the formula, $Y_6$ and $Y_9$ each independently represents a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_8$ represents a C1-C4 haloalkoxy group, a C2-C6 perfluoroalkyl group, a C1-C6 perfluoroalkylthio group, a C1-C6 perfluoroalkylsulfinyl group or a C1-C6 perfluoroalkylsulfonyl group; and $Y_7$ represents a hydrogen atom, a halogen atom or a C1-C4 alkyl group (Herein, at least one of $Y_6$ and $Y_9$ must represent a Cl-C4 alkoxy group, a C1-C4 haloalkoxy group, a C1-C3 haloalkylthio group, a C1-C3 haloalkylsulfinyl group or a C1-C3 haloalkylsulfonyl group.);

[2] a compound represented by the general formula (1),

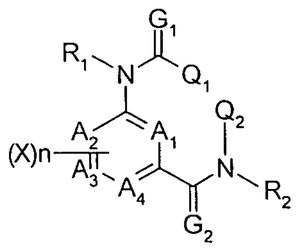

(1)

wherein, in the formula, $A_1$, $A_2$, $A_3$ and $A_4$ each independently represents a carbon atom, a nitrogen atom or an oxidized nitrogen atom;

$R_1$ and $R_2$ are each independently a hydrogen atom, a C1-C4 alkyl group or a C1-C4 alkylcarbonyl group;
$G_1$ and $G_2$ are each independently an oxygen atom or a sulfur atom;
Xs are each independently a hydrogen atom, a halogen atom or a trifluoromethyl group;
n represents an integer of 0 to 4;
$Q_1$ is a phenyl group,
a substituted phenyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group),
a heterocyclic group (The heterocyclic group herein represents a pyridyl group, a pyridine N-oxide group, a pyrimidinyl group, a pyridazyl group, a pyrazyl group, a furyl group, a thienyl group, an oxazolyl group, an isoxazolyl group, an oxadiazolyl group, a thiazolyl group, an isothiazolyl group, an imidazolyl group, a triazolyl group, a pyrrole group, a pyrazolyl group or a tetrazolyl group.), or
a substituted heterocyclic group having one or more substituents which may be the same or different (the substituents selected from a halogen atom, a C1-C4 alkyl group, a C1-C4.haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group) (The heterocyclic group represents the same as those described above.); and
$Q_2$ is represented by the general formula (2) or (3),

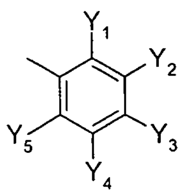

(2)

wherein, in the formula, $Y_1$ represents a C1-C4 alkoxy group, a C1-C4 haloalkoxy group, a C1-C3 haloalkylthio group, a C1-C3 haloalkylsulfinyl group or a C1-C3 haloalkylsulfonyl group; $Y_5$ represents a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_3$ represents a C2-C6 perfluoroalkyl group, a C1-C6 perfluoroalkylthio group, a C1-C6 perfluoroalkylsulfinyl group or a C1-C6 perfluoroalkylsulfonyl group; and $Y_2$ and $Y_4$ each independently represents a hydrogen atom, a halogen atom or a C1-C4 alkyl group, or

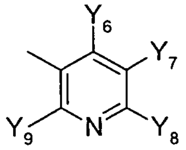 (3)

wherein, in the formula, $Y_6$ and $Y_9$ each independently represents a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_8$ represents a C1-C4 haloalkoxy group, a C2-C6 perfluoroalkyl group, a C1-C6 perfluoroalkylthio group, a C1-C6 perfluoroalkylsulfinyl group or a C1-C6 perfluoroalkylsulfonyl group; and $Y_7$ represents a hydrogen atom, a halogen atom or a C1-C4 alkyl group (Herein, at least one of $Y_6$ and $Y_9$ must represent a C1-C4 alkoxy group, a C1-C4 haloalkoxy group, a C1-C3 haloalkylthio group, a C1-C3 haloalkylsulfinyl group or a C1-C3 haloalkylsulfonyl group.),
whereas such compounds are excluded, wherein $Y_1$ represents a trifluoromethylthio group; $Y_2$ and $Y_4$ represent hydrogen atoms; $Y_3$ represents a heptafluoroisopropyl group; $Y_5$ represents a bromine atom; X represents a hydrogen atom; $G_1$ and $G_2$ represent oxygen atoms; $R_1$ and $R_2$ represent hydrogen atoms; and $Q_1$ represents an unsubstituted phenyl group;

[3] the compound as described in [1] or [2], represented by the general formula (1a) in which $A_1$, $A_2$, $A_3$ and $A_4$ in the general formula (1) are all carbon atoms,

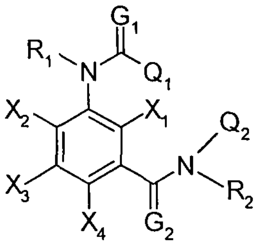 (1a)

wherein, in the formula, when any one of $R_1$ and $R_2$ is a hydrogen atom, the other one is a C1-C4 alkyl group or a C1-C4 alkylcarbonyl group, or both of $R_1$ and $R_2$ are C1-C4 alkyl groups or C1-C4 alkylcarbonyl groups;

$G_1$ and $G_2$ are each independently an oxygen atom or a sulfur atom;
$X_1$, $X_2$, $X_3$ and $X_4$ are each independently a hydrogen atom, a halogen atom or a trifluoromethyl group;
$Q_1$ is a phenyl group,
a substituted phenyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group),
a heterocyclic group (The heterocyclic group herein represents a pyridyl group, a pyridine N-oxide group, a pyrimidinyl group, a pyridazyl group, a pyrazyl group, a furyl group, a thienyl group, an oxazolyl group, an isoxazolyl group, an oxadiazolyl group, a thiazolyl group, an isothiazolyl group, an imidazolyl group, a triazolyl group, a pyrrole group, a pyrazolyl group or a tetrazolyl group.), or
a substituted heterocyclic group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino

group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group) (The heterocyclic group represents the same as those described above.); and

$Q_2$ is represented by the general formula (2) or (3),

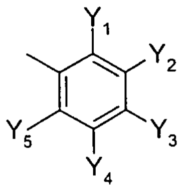

(2)

wherein, in the formula, $Y_1$ represents a C1-C3 haloalkylthio group, a C1-C3 haloalkylsulfinyl group or a C1-C3 haloalkylsulfonyl group; $Y_5$ represents a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_3$ represents a C2-C6 perfluoroalkyl group, a C1-C6 perfluoroalkylthio group, a C1-C6 perfluoroalkylsulfinyl group or a C1-C6 perfluoroalkylsulfonyl group; and $Y_2$ and $Y_4$ each independently represents a hydrogen atom, a halogen atom or a C1-C4 alkyl group, or

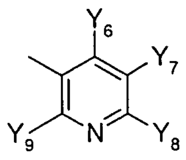

(3)

wherein, in the formula, $Y_6$ and $Y_9$ each independently represents a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_8$ represents a C1-C4 haloalkoxy group, a C2-C6 perfluoroalkyl group, a C1-C6 perfluoroalkylthio group, a C1-C6 perfluoroalkylsulfinyl group or a C1-C6 perfluoroalkylsulfonyl group; and $Y_7$ represents a hydrogen atom, a halogen atom or a C1-C4 alkyl group (Herein, at least one of $Y_6$ and $Y_9$ must represent a C1-C3 haloalkylthio group, a C1-C3 haloalkylsulfinyl group or a C1-C3 haloalkylsulfonyl group.);

[4] the compound as described in [3], wherein $Q_2$ is represented by the general formula (2),

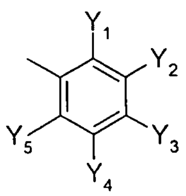

(2)

wherein, in the formula, $Y_1$ represents a C1-C3 haloalkylthio group, a C1-C3 haloalkylsulfinyl group or a C1-C3 haloalkylsulfonyl group; $Y_5$ represents a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_3$ represents a C2-C6 perfluoroalkyl group, a C1-C6 perfluoroalkylthio group, a C1-C6 perfluoroalkylsulfinyl group or a C1-C6 perfluoroalkylsulfonyl group; and $Y_2$ and $Y_4$ represent each independently a hydrogen atom, a halogen atom or a C1-C4 alkyl group;

[5] the compound as described in [4], wherein $Q_1$ represents a phenyl group,

a substituted phenyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group),
a pyridyl group, or
a substituted pyridyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, aC1-C4 alkylcarbonyl group, a C1-C4 alkylcarbonyloxy group, a C1-C4 alkoxycarbonyl group, an acetylamino group and a phenyl group);

[6] the compound as described in [1] or [2], represented by the general formula (1a) in which $A_1$, $A_2$, $A_3$ and $A_4$ in the general formula (1) are all carbon atoms,

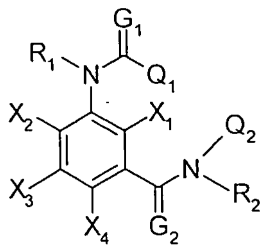 (1a)

wherein, in the formula, $R_1$ and $R_2$ are each independently a hydrogen atom, a C1-C4 alkyl group or a C1-C4 alkylcarbonyl group;

$G_1$ and $G_2$ are each independently an oxygen atom or a sulfur atom;
$X_1$, $X_2$, $X_3$ and $X_4$ are each independently a hydrogen atom, a halogen atom or a trifluoromethyl group;
$Q_1$ is a substituted phenyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group),
a heterocyclic group (The heterocyclic group herein represents a pyridyl group, a pyridine N-oxide group, a pyrimidinyl group, a pyridazyl group, a pyrazyl group, a furyl group, a thienyl group, an oxazolyl group, an isoxazolyl group, an oxadiazolyl group, a thiazolyl group, an isothiazolyl group, an imidazolyl group, a triazolyl group, a pyrrole group, a pyrazolyl group or a tetrazolyl group.), or
a substituted heterocyclic group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group) (The heterocyclic group represents the same as those described above.); and
$Q_2$ is represented by the general formula (2) or (3),

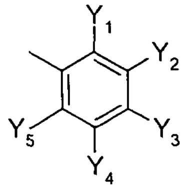

(2)

wherein, in the formula, $Y_1$ represents a C1-C3 haloalkylthio group, a C1-C3 haloalkylsulfinyl group or a C1-C3 haloalkylsulfonyl group;. $Y_5$ represents a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_3$ represents a C2-C6 perfluoroalkyl group, a C1-C6 perfluoroalkylthio group, a C1-C6 perfluoroalkylsulfinyl group or a C1-C6 perfluoroalkylsulfonyl group; and $Y_2$ and $Y_4$ each independently represent a hydrogen atom, a halogen atom or a C1-C4 alkyl group, or

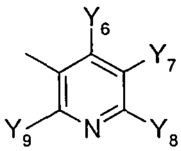

(3)

wherein, in the formula, $Y_6$ and $Y_9$ each independently represent a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_8$ represents a C1-C4 haloalkoxy group, a C2-C6 perfluoroalkyl group, a C1-C6 perfluoroalkylthio group, a C1-C6 perfluoroalkylsulfinyl group or a C1-C6 perfluoroalkylsulfonyl group; and $Y_7$ represents a hydrogen atom, a halogen atom or a C1-C4 alkyl group (Herein, at least one of $Y_6$ and $Y_9$ must represent a C1-C3 haloalkylthio group, a C1-C3 haloalkylsulfinyl group or a C1-C3 haloalkylsulfonyl group.);

[7] the compound as described in [6], wherein $Q_1$ represents a substituted phenyl group having one or more substituents which may be the same or different (the substituents selected from a halogen atom, a C1-C4 -alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group),

a pyridyl group, or
a substituted pyridyl group having one or more substituents which may be the same or different (the substituents selected a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group); and
$Q_2$ is represented by the general formula (2),

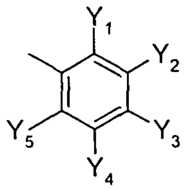

(2)

wherein, in the formula, $Y_1$ represents a C1-C3 haloalkylthio group, a C1-C3 haloalkylsulfinyl group or a C1-C3 haloalkylsulfonyl group; $Y_5$ represents a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_3$ represents a C2-C6 perfluoroalkyl group, a C1-C6 perfluoroalkylthio group, a C1-C6 perfluoroalkylsulfinyl group or a C1-C6 perfluoroalkylsulfonyl group; and $Y_2$ and $Y_4$ each independently represent a hydrogen atom, a halogen atom or a C1-C4 alkyl group;

[8] the compound as described in [1] or [2], represented by the general formula (1a) in which $A_1$, $A_2$, $A_3$ and $A_4$ in the general formula (1) are all carbon atoms,

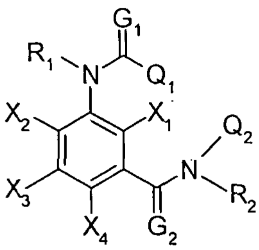

(1a)

wherein, in the formula, $R_1$ and $R_2$ are each independently a hydrogen atom, a C1-C4 alkyl group or a C1-C4 alkylcarbonyl group;

$G_1$ and $G_2$ are each independently an oxygen atom or a sulfur atom;
$X_1$, $X_2$, $X_3$ and $X_4$ are each independently a hydrogen atom, a halogen atom or a trifluoromethyl group;
$Q_1$ is a phenyl group,
a substituted phenyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group),
a heterocyclic group (The heterocyclic group herein represents a pyridyl group, a pyridine N-oxide group, a pyrimidinyl group, a pyridazyl group, a pyrazyl group, a furyl group, a thienyl group, an oxazolyl group, an isoxazolyl group, an oxadiazolyl group, a thiazolyl group, an isothiazolyl group, an imidazolyl group, a triazolyl group, a pyrrole group, a pyrazolyl group or a tetrazolyl group.), or
a substituted heterocyclic group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group) (The heterocyclic group represents the same as those described above.); and
$Q_2$ is represented by the general formula (2) or (3),

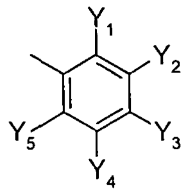

(2)

wherein, in the formula, $Y_1$ represents a C1-C3 haloalkylthio group, a C1-C3 haloalkylsulfinyl group or a C1-C3 haloalkylsulfonyl group; $Y_5$ represents a fluorine atom, a chlorine atom, an iodine atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_3$ represents a C2-C6 perfluoroalkyl group, a C1-C6 perfluoroalkylthio group, a C1-C6 perfluoroalkylsulfinyl group or a C1-C6 perfluoroalkylsulfonyl group; and $Y_2$ and $Y_4$ each independently represent a hydrogen atom, a halogen atom or a C1-C4 alkyl group, or

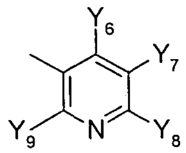

(3)

wherein, in the formula, $Y_6$ and $Y_9$ each independently representa halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_8$ represents a C1-C4 haloalkoxy group, a C2-C6 perfluoroalkyl group, a C1-C6 perfluoroalkylthio group, a C1-C6 perfluoroalkylsulfinyl group or a C1-C6 perfluoroalkylsulfonyl group; and $Y_7$ represents a hydrogen atom, a halogen atom or a C1-C4 alkyl group (Herein, at least one of $Y_6$ and $Y_9$ must represent a C1-C3 haloalkylthio group, a C1-C3 haloalkylsulfinyl group or a C1-C3 haloalkylsulfonyl group.);

[9] the compound as described in [8], wherein $Q_1$ represents a phenyl group,

a substituted phenyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group),
a pyridyl group, or
a substituted pyridyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group); and
$Q_2$ is represented by the general formula (2),

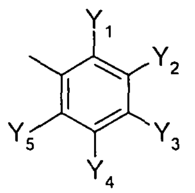

(2)

wherein, in the formula, $Y_1$ represents a C1-C3 haloalkylthio group, a C1-C3 haloalkylsulfinyl group or a C1-C3 haloalkylsulfonyl group; $Y_5$ represents a fluorine atom, a chlorine atom, an iodine atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_3$ represents a C2-C6 perfluoroalkyl group, a C1-C6 perfluoroalkylthio group, a C1-C6 perfluoroalkylsulfinyl group or a C1-C6 perfluoroalkylsulfonyl group; and $Y_2$ and $Y_4$ each independently represent a hydrogen atom, a halogen atom or a C1-C4 alkyl group;

[10] the compound as described in [1] or [2], represented by the general formula (1a) in which $A_1$, $A_2$, $A_3$ and $A_4$ in the general formula (1) are all carbon atoms,

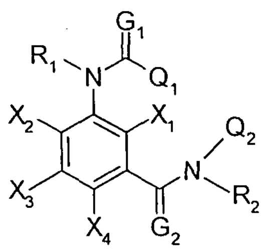

(1a)

wherein, in the formula, $R_1$ and $R_2$ are each independently a hydrogen atom, a C1-C4 alkyl group or a C1-C4 alkylcarbonyl group;

$G_1$ and $G_2$ are each independently an oxygen atom or a sulfur atom;
$X_1$, $X_2$, $X_3$ and $X_4$ are each independently a hydrogen atom, a halogen atom or a trifluoromethyl group;
$Q_1$ is a phenyl group,
a substituted phenyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group),
a heterocyclic group (The heterocyclic group herein represents a pyridyl group, a pyridine N-oxide group, a pyrimidinyl group, a pyridazyl group, a pyrazyl group, a furyl group, a thienyl group, an oxazolyl group, an isoxazolyl group, an oxadiazolyl group, a thiazolyl group, an isothiazolyl group, an imidazolyl group, a triazolyl group, a pyrrole group, a pyrazolyl group or a tetrazolyl group.), or
a substituted heterocyclic group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group) (The heterocyclic group represents the same as those described above.); and
$Q_2$ is represented by the general formula (2) or (3),

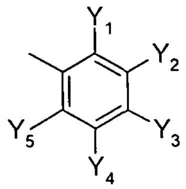

(2)

wherein, in the formula, $Y_1$ represents a C2-C3 haloalkylthio group, a C1-C3 haloalkylsulfinyl group or a C1-C3 haloalkylsulfonyl group; $Y_5$ represents a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_3$ represents a C2-C6 perfluoroalkyl group, a C1-C6 perfluoroalkylthio group, a C1-C6 perfluoroalkylsulfinyl group or a C1-C6 perfluoroalkylsulfonyl group; and $Y_2$ and $Y_4$ each independently represents a hydrogen atom, a halogen atom or a C1-C4 alkyl group; or

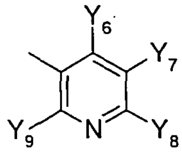

(3)

wherein, in the formula, $Y_6$ and $Y_9$ each independently represent a halogen atom, a C1-C4 alkyl group, a C1-C4 haloalkyl group, a C1-C4 alkoxy group, a C1-C4 haloalkoxy group, a C1-C3 alkylthio group, a C2-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_8$ represents a C1-C4 haloalkoxy group, a C2-C6 perfluoroalkyl group, a C1-C6 perfluoroalkylthio group, a C1-C6 perfluoroalkylsulfinyl group or a C1-C6 perfluoroalkylsulfonyl group; and $Y_7$ represents a hydrogen atom, a halogen atom or a C1-C4 alkyl group (Herein, at least one of $Y_6$ and $Y_9$ must represent a C2-C3 haloalkylthio group, a C1-C3 haloalkylsulfinyl group or a C1-C3 haloalkylsulfonyl group.);

[11] the compound as described in [10], wherein $Q_1$ represents a phenyl group,

a substituted phenyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group),
a pyridyl group, or
a substituted pyridyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group); and
$Q_2$ is represented by the general formula (2),

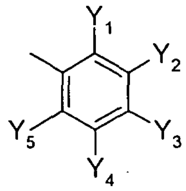

(2)

wherein, in the formula, $Y_1$ represents a C2-C3 haloalkylthio group, a C1-C3 haloalkylsulfinyl group or a C1-C3 haloalkylsulfonyl group; $Y_5$ represents a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_3$ represents a C2-C6 perfluoroalkyl group, a C1-C6 perfluoroalkylthio group, a C1-C6 perfluoroalkylsulfinyl group or a C1-C6 perfluoroalkylsulfonyl group; and $Y_2$ and $Y_4$ each independently represents a hydrogen atom, a halogen atom or a C1-C4 alkyl group;

[12] the compound as described in [1], represented by the general formula (1),

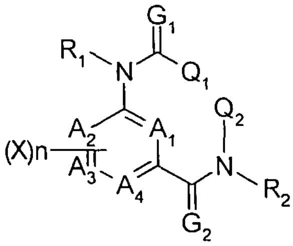

(1)

wherein, in the formula, $A_1$, $A_2$, $A_3$ and $A_4$ each independently represents a carbon atom, a nitrogen atom or an oxidized nitrogen atom;
when any one of $R_1$ and $R_2$ is a hydrogen atom, the other one is a C1-C4 alkyl group or a C1-C4 alkylcarbonyl group, or both of $R_1$ and $R_2$ are C1-C4 alkyl groups or C1-C4 alkylcarbonyl groups;

$G_1$ and $G_2$ are each independently an oxygen atom or a sulfur atom;
Xs are each independently a hydrogen atom, a halogen atom or a trifluoromethyl group;
n represents an integer of 0 to 4;
$Q_1$ is a phenyl group,
a substituted phenyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group),
a heterocyclic group (The heterocyclic group herein represents a pyridyl group, a pyridine N-oxide group, a pyrimidinyl group, a pyridazyl group, a pyrazyl group, a furyl group, a thienyl group, an oxazolyl group, an isoxazolyl group, an oxadiazolyl group, a thiazolyl group, an isothiazolyl group, an imidazolyl group, a triazolyl group, a pyrrole group, a pyrazolyl group or a tetrazolyl group.), or
a substituted heterocyclic group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group) (The heterocyclic group

represents the same as those described above.); and
$Q_2$ is represented by the general formula (2) or (3),

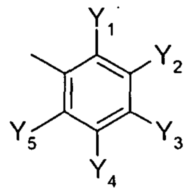

(2)

wherein, in the formula, $Y_1$ represents a C1-C4 alkoxy group or a C1-C4 haloalkoxy group; $Y_5$ represents a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_3$ represents a C2-C6 perfluoroalkyl group, a C1-C6 perfluoroalkylthio group, a C1-C6 perfluoroalkylsulfinyl group or a C1-C6 perfluoroalkylsulfonyl group; and $Y_2$ and $Y_4$ each independently represents a hydrogen atom, a halogen atom or a C1-C4 alkyl group, or

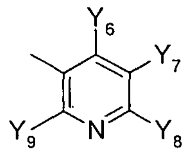

(3)

wherein, in the formula, $Y_6$ and $Y_9$ each independently represents a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_8$ represents a C1-C4 haloalkoxy group, a C2-C6 perfluoroalkyl group, a C1-C6 perfluoroalkylthio group, a C1-C6 perfluoroalkylsulfinyl group or a C1-C6 perfluoroalkylsulfonyl group; and $Y_7$ represents a hydrogen atom, a halogen atom or a C1-C4 alkyl group (Herein, at least one of $Y_6$ and $Y_9$ must represent a C1-C4 alkoxy group or a C1-C4 haloalkoxy group.);

[13] the compound as described in [12], represented by the general formula (1a) in which $A_1$, $A_2$, $A_3$ and $A_4$ in the general formula (1) are all carbon atoms,

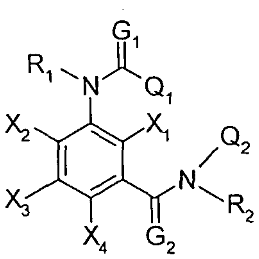

(1a)

wherein, in the formula, when any one of $R_1$ and $R_2$ is a hydrogen atom, the other one is a C1-C4 alkyl group or a C1-C4 alkylcarbonyl groups or both $R_1$ and $R_2$ are C1-C4 alkyl groups or C1-C4 alkylcarbonyl groups;

$G_1$ and $G_2$ are each independently an oxygen atom or a sulfur atom;
$X_1$, $X_2$, $X_3$ and $X_4$ are each independently a hydrogen atom, a halogen atom or a trifluoromethyl group;
$Q_1$ is a phenyl group,
a substituted phenyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3

haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group),

a heterocyclic group (The heterocyclic group herein represents a pyridyl group, a pyridine N-oxide group, a pyrimidinyl group, a pyridazyl group, a pyrazyl group, a furyl group, a thienyl group, an oxazolyl group, an isoxazolyl group, an oxadiazolyl group, a thiazolyl group, an isothiazolyl group, an imidazolyl group, a triazolyl group, a pyrrole group, a pyrazolyl group or a tetrazolyl group.), or

a substituted heterocyclic group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group) (The heterocyclic group represents the same as those described above.); and

$Q_2$ is represented by the general formula (2),

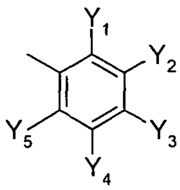

(2)

wherein, in the formula, $Y_1$ represents a C1-C4 haloalkoxy group; $Y_5$ represents a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_3$ represents a C2-C6 perfluoroalkyl group, a C1-C6 perfluoroalkylthio group, a C1-C6 perfluoroalkylsulfinyl group or a C1-C6 perfluoroalkylsulfonyl group; and $Y_2$ and $Y_4$ each independently represents a hydrogen atom, a halogen atom or a C1-C4 alkyl group;

[14] the compound as described in [13], wherein $Q_1$ represents a phenyl group,

a substituted phenyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group),

a pyridyl group, or

a substituted pyridyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group);

[15] a compound represented by the general formula (4),

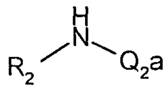
(4)

wherein, in the formula, $R_2$ represents a hydrogen atom, a C1-C4 alkyl group or a C1-C4 alkylcarbonyl group; and

$Q_2a$ is represented by the general formula (2), (3) or (5),

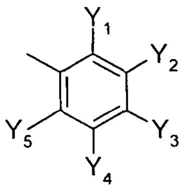
(2)

wherein, in the formula, $Y_1$, $Y_2$, $Y_3$, $Y_4$ and $Y_5$ represent the same as those described in [1],

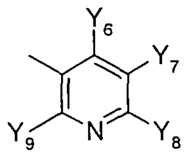
(3)

wherein, in the formula, $Y_6$, $Y_7$, $Y_8$ and $Y_9$ represent the same as those described in [1], or

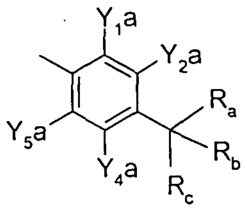
(5)

wherein, in the formula, $R_a$ and $R_b$ are each independently a fluorine atom or a C1-C4 perfluoroalkyl group; $R_c$ is a hydroxy group, -O-$R_d$ ($R_d$ represents a C1-C3 alkyl group, a C1-C3 haloalkyl group, a C1-C3 alkylsulfonyl group, a C1-C3 haloalkylsulfonyl group, an arylsulfonyl group, a C1-C4 alkylcarbonyl group or a C1-C4 haloalkylcarbonyl group.), a chlorine atom, a bromine atom or an iodine atom; $Y_1a$ represents a C1-C4 alkoxy group, a C1-C4 haloalkoxy group, a C1-C3 haloalkylthio group, a C1-C3 haloalkylsulfinyl group or a C1-C3 haloalkylsulfonylgroup; $Y_5a$ represents a hydrogen atom, a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; and $Y_2a$ and $Y_4a$ each independently represents a hydrogen atom, a halogen atom or a C1-C4 alkyl group;

[16] a process for preparing the compound as described in any one of [1] to [14] and [21], which comprises reacting the compound represented by the general formula (4) as described in [15] with a compound represented by the general formula (6),

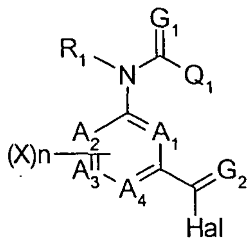

(6)

wherein, in the formula, $A_1$, $A_2$, $A_3$ and $A_4$ each independently represents a carbon atom, a nitrogen atom or an oxidized nitrogen atom;

$R_1$ represents a hydrogen atom, a C1-C4 alkyl group or a C1-C4 alkylcarbonyl group;
$G_1$ and $G_2$ each independently represent an oxygen atom or a sulfur atom;
X represents a hydrogen atom, a halogen atom or a trifluoromethyl group;
n represents an integer of 0 to 4;
$Q_1$ represents the same as those described in [1] ; and
Hal represents a chlorine atom or a bromine atom;

[17] a compound represented by the general formula (7),

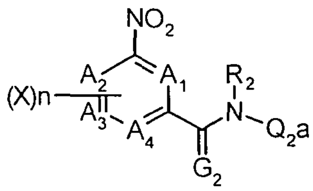

(7)

wherein, in the formula, $A_1$, $A_2$, $A_3$ and $A_4$ each independently represents a carbon atom, a nitrogen atom or an oxidized nitrogen atom;

$R_2$ is a hydrogen atom, a C1-C4 alkyl group or a C1-C4 alkylcarbonyl group;
$G_2$ is an oxygen atom or a sulfur atom;
X is a hydrogen atom, a halogen atom or a trifluoromethyl group;
n represents an integer of 0 to 4; and
$Q_2$a represents the same as those described in [15];

[18] a process for preparing the compound as descried in [17], which comprises reacting a compound represented by the general formula (8) with the compound represented by the general formula (4) as described in [15],

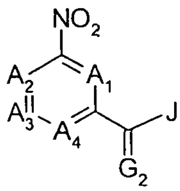

(8)

wherein, in the formula, $A_1$, $A_2$, $A_3$ and $A_4$ each independently represents a carbon atom, a nitrogen atom or an oxidized nitrogen atom;

$G_2$ is an oxygen atom or a sulfur atom; and

J represents a halogen atom or a hydroxy group;

[19] a compound represented by the general formula (9),

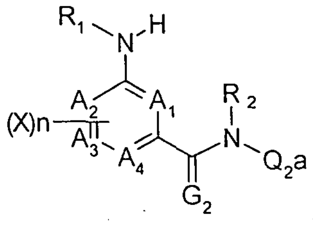
(9)

wherein, in the formula, $A_1$, $A_2$, $A_3$ and $A_4$ each independently represent a carbon atom, a nitrogen atom or an oxidized nitrogen atom;

$R_1$ and $R_2$ are each independently a hydrogen atom, a C1-C4 alkyl group or a C1-C4 alkylcarbonyl group;
$G_2$ is an oxygen atom or a sulfur atom;
Xs are a hydrogen atom, a halogen atom or a trifluoromethyl group;
n represents an integer of 0 to 4; and
$Q_2a$ represents the same as those described in [15];

[20] a process for preparing the compound represented by the general formula (9) as described in [19], which comprises reacting the compound represented by the general formula (7) as described in [17] in the presence of a suitable reducing agent;
[21] a compound represented by the general formula (10),

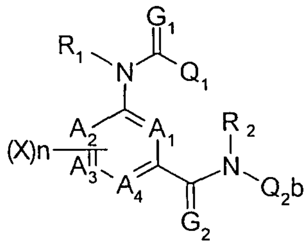
(10)

wherein, in the formula, $A_1$, $A_2$, $A_3$ and $A_4$ each independently represents a carbon atom, a nitrogen atom or an oxidized nitrogen atom;

$R_1$ and $R_2$ are each independently a hydrogen atom, a C1-C4 alkyl group or a C1-C4 alkylcarbonyl group;
$G_1$ and $G_2$ are each independently an oxygen atom or a sulfur atom;
X is a hydrogen atom, a halogen atom or a trifluoromethyl group;
n represents an integer of 0 to 4;
$Q_1$ represents the same as those described in [1] ; and
$Q_2b$ is represented by the general formula (5),

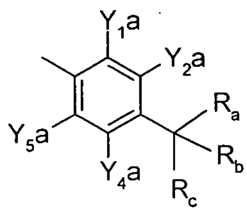
(5)

wherein, in the formula, $R_a$ and $R_b$ are each independently a fluorine atom or a C1-C4 perfluoroalkyl group; $R_c$ is a hydroxy group, $-O-R_d$ ($R_d$ represents a C1-C3 alkyl group, a C1-C3 haloalkyl group, a C1-C3 alkylsulfonyl group, a C1-C3 haloalkylsulfonyl group, an arylsulfonyl group, a C1-C4 alkylcarbonyl group or a C1-C4 haloalkylcarbonyl group.), a chlorine atom, a bromine atom or an iodine atom; $Y_1a$ represents a C1-C4 alkoxy group, a

C1-C4 haloalkoxy group, a C1-C3 haloalkylthio group, a C1-C3 haloalkylsulfinyl group or a C1-C3 haloalkylsulfonyl group; $Y_5$a represents a hydrogen atom, a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; and $Y_2$a and $Y_4$a each independently represent a hydrogen atom, a halogen atom or a C1-C4 alkyl group;

[22] a process for preparing the compound as described in any one of [1] to [14] and [21], which comprises reacting the compound represented by the general formula (9) as described in [19] with a compound represented by the general formula (11) or (12),

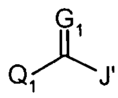

(11)

wherein, in the formula, $G_1$ represents an oxygen atom or a sulfur atom; J' represents a halogen atom or a hydroxy group; and $Q_1$ represents the same as those described [1], or

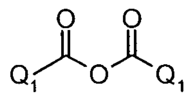

(12)

wherein, in the formula, $Q_1$ represents the same as those described [1];
[23] a process for preparing a compound represented by the general formula (14),

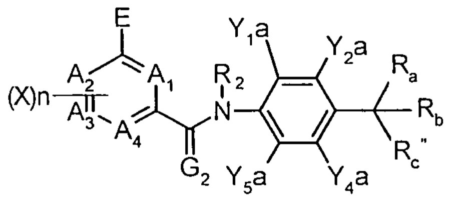

(14)

wherein, in the formula, $A_1$, $A_2$, $A_3$ and $A_4$ each independently represents a carbon atom, a nitrogen atom or an oxidized nitrogen atom; $R_2$ is a hydrogen atom, a C1-C4 alkyl group or a C1-C4 alkylcarbonyl group; $G_2$ is an oxygen atom or a sulfur atom; X is a hydrogen atom, a halogen atom or a trifluoromethyl group; n represents an integer of 0 to 4; $Y_1$a, $Y_2$a, $Y_4$a, $Y_5$a, $R_a$ and $R_b$ each represent the same as those described in [15] ; $R_{c''}$ represents a chlorine atom, a bromine atom or an iodine atom; E represents a nitro group, -NH-($R_1$) ($R_1$ represents a hydrogen atom, a C1-C4 alkyl group or a C1-C4 alkylcarbonyl group.), -N($R_1$)-C(= $G_1$)$Q_1$ ($R_1$ represents a hydrogen atom, a C1-C4 alkyl group or a C1-C4 alkylcarbonyl group; $G_1$ represents an oxygen atom or a sulfur atom; and $Q_1$ represents the same as those described in [1],
which comprises reacting a compound represented by the general formula (13) with a suitable halogenating agent,

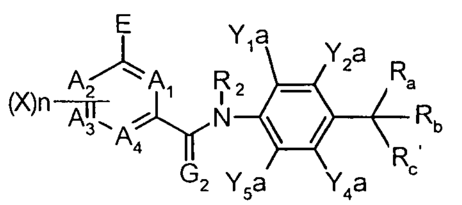

(13)

wherein, in the formula, $A_1$, $A_2$, $A_3$ and $A_4$ each independently represent a carbon atom, a nitrogen atom or an oxidized nitrogen atom; $R_2$ is a hydrogen atom, a C1-C4 alkyl group or a C1-C4 alkylcarbonyl group; $G_2$ is an oxygen atom or a sulfur atom; X is a hydrogen atom, a halogen atom or a trifluoromethyl group; n represents an integer of

0 to 4; $Y_1a$, $Y_2a$, $Y_4a$, $Y_5a$, $R_a$ and $R_b$ each represent the same as those described in [15]; $R_{c'}$ represents a hydroxy group, -O-$R_d$ ($R_d$ represents a C1-C3 alkyl group, a C1-C3 haloalkyl group, a C1-C3 alkylsulfonyl group, a C1-C3 haloalkylsulfonyl group, an arylsulfonyl group, a C1-C4 alkylcarbonyl group or a C1-C4 haloalkylcarbonyl group.); and E represents a nitro group, -NH-($R_1$) ($R_1$ represents a hydrogen atom, a C1-C4 alkyl group or a C1-C4 alkylcarbonyl group.) or -N($R_1$)-C(= $G_1$)$Q_1$ ($R_1$ represents a hydrogen atom, a C1-C4 alkyl group or a C1-C4 alkylcarbonyl group; $G_1$ represents an oxygen atom or a sulfur atom; and $Q_1$ represents the same as those described in [1].);

[24] a process for preparing a compound represented by the general formula (15), which comprises reacting the compound represented by the general formula (13) or the compound represented by the general formula (14) as described in [23] with a suitable fluorinating agent,

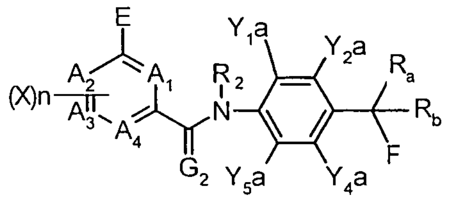
(15)

wherein, in the formula, $A_1$, $A_2$, $A_3$, $A_4$, X, n, $R_2$, $G_2$, $Y_1a$, $Y_2a$, $Y_4a$, $Y_5a$, $R_a$, $R_b$ and E represent the same as those described in [23];

[25] an insecticide comprising the compound as described in any one of [1] to [14] as an active ingredient; and

[26] a method for application of a chemical, which comprises applying an effective amount of the compound as described in any one of [1] to [14] on useful crops or soil for the purpose of protecting useful crops from harmful organisms.

The compound of the present invention exhibits a control effect on various insect pests, protects usual crops, and greatly contributes to the reduction of environmental load with a small amount of chemicals.

BEST MODE FOR CARRYING OUT THE INVENTION

A compound of the present invention is represented by the general formula (1);

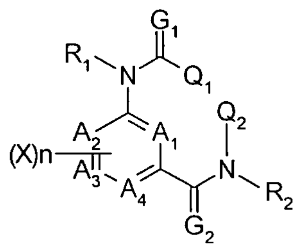
(1)

wherein, in the formula, $A_1$, $A_2$, $A_3$ and $A_4$ each independently represents a carbon atom, a nitrogen atom or an oxidized nitrogen atom;

$R_1$ and $R_2$ are each independently a hydrogen atom, a C1-C4 alkyl group or a C1-C4 alkylcarbonyl group;
$G_1$ and $G_2$ are each independently an oxygen atom or a sulfur atom;
Xs are each independently a hydrogen atom, a halogen atom or a trifluoromethyl group;
n is an integer of 0 to 4;
$Q_1$ is a phenyl group,
a substituted phenyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl

group, an acetylamino group and a phenyl group),
a heterocyclic group (The heterocyclic group herein represents a pyridyl group, a pyridine N-oxide group, a pyrimidinyl group, a pyridazyl group, a pyrazyl group, a furyl group, a thienyl group, an oxazolyl group, an isoxazolyl group, an oxadiazolyl group, a thiazolyl group, an isothiazolyl group, an imidazolyl group, a triazolyl group, a pyrrole group, a pyrazolyl group or a tetrazolyl group.), or
a substituted heterocyclic group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group) (The heterocyclic group represents the same as those described above.); and
$Q_2$ is represented by the general formula (2) or (3),

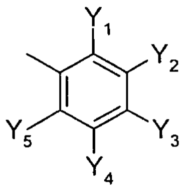
(2)

wherein, in the formula, $Y_1$ represents a C1-C4 alkoxy group, a C1-C4 haloalkoxy group, a C1-C3 haloalkylthio group, a C1-C3 haloalkylsulfinyl group or a C1-C3 haloalkylsulfonyl group; $Y_5$ represents a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_3$ represents a C2-C6 perfluoroalkyl group, a C1-C6 perfluoroalkylthio group, a C1-C6 perfluoroalkylsulfinyl group or a C1-C6 perfluoroalkylsulfonyl group; and $Y_2$ and $Y_4$ each independently represent a hydrogen atom, a halogen atom or a C1-C4 alkyl group; or

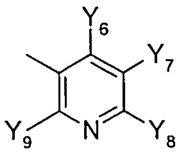
(3)

wherein, in the formula, $Y_6$ and $Y_9$ each independently represents a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_8$ represents a C1-C4 haloalkoxy group, a C2-C6 perfluoroalkyl group, a C1-C6 perfluoroalkylthio group, a C1-C6 perfluoroalkylsulfinyl group or a C1-C6 perfluoroalkylsulfonyl group; and $Y_7$ represents a hydrogen atom, a halogen atom or a C1-C4 alkyl group (Herein, at least one of $Y_6$ and $Y_9$ must represent a C1-C4 alkoxy group, a C1-C4 haloalkoxy group, a C1-C3 haloalkylthio group, a C1-C3 haloalkylsulfinyl group or a C1-C3 haloalkylsulfonyl group.).

A compound of the present invention may be represented by the general formula (10);

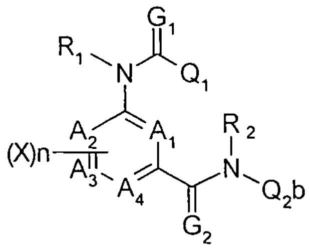

(10)

wherein, in the formula, $A_1$, $A_2$, $A_3$ and $A_4$ each independently represent a carbon atom, a nitrogen atom or an oxidized nitrogen atom;

$R_1$ and $R_2$ are each independently a hydrogen atom, a C1-C4 alkyl group or a C1-C4 alkylcarbonyl group;
$G_1$ and $G_2$ are each independently an oxygen atom or a sulfur atom;
X is a hydrogen atom, a halogen atom or a trifluoromethyl group;
n represents an integer of 0 to 4;
$Q_1$ is a phenyl group,
a substituted phenyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group),
a heterocyclic group (The heterocyclic group herein represents a pyridyl group, a pyridine N-oxide group, a pyrimidinyl group, a pyridazyl group, a pyrazyl group, a furyl group, a thienyl group, an oxazolyl group, an isoxazolyl group, an oxadiazolyl group, a thiazolyl group, an isothiazolyl group, an imidazolyl group, a triazolyl group, a pyrrole group, a pyrazolyl group or a tetrazolyl group.), or
a substituted heterocyclic group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group) (The heterocyclic group represents the same as those described above.); and
$Q_2b$ is represented by the general formula (5),

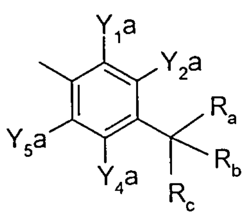

(5)

wherein, in the formula, $R_a$ and $R_b$ are each independently a fluorine atom or a C1-C4 perfluoroalkyl group; $R_c$ is a hydroxy group, -O-$R_d$ ($R_d$ represents a C1-C3 alkyl group, a C1-C3 haloalkyl group, a C1-C3 alkylsulfonyl group, a C1-C3 haloalkylsulfonyl group, an arylsulfonyl group, a C1-C4 alkylcarbonyl group or a C1-C4 haloalkylcarbonyl group.), a chlorine atom, a bromine atom or an iodine atom; $Y_1a$ represents a C1-C4 alkoxy group, a C1-C4 haloalkoxy group, a C1-C3 haloalkylthio group, a C1-C3 haloalkylsulfinyl group or a C1-C3 haloalkylsulfonyl group; $Y_5a$ represents a hydrogen atom, a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; and $Y_2a$

and $Y_4$a each independently represent a hydrogen atom, a halogen atom or a C1-C4 alkyl group.

Wordings used in general formulae such as the general formula (1) of the present invention and the like have the respective meanings as described hereinafter in terms of definitions.

The term "halogen atom" represents a fluorine atom, a chlorine atom, a bromine atom or an iodine atom.

In expression of "Ca-Cb (a and b represent an integer of not less than 1)," for example, "C1-C3" refers to 1 to 3 carbon atoms in the carbon chain, "C2-C6" refers to 2 to 6 carbon atoms in the carbon chain, and "C1-C4" refers to 1 to 4 carbon atoms in the carbon chain.

"n-" refers to normal, "i-" refers to iso, "s-" refers to secondary and "t-" refers to tertiary.

Furthermore, the term "C1-C3 alkyl group" refers to a straight or branched alkyl group having 1 to 3 carbon atoms such as methyl, ethyl, n-propyl, i-propyl or the like.

The term "C1-C4 alkyl group" refers to a straight or branched alkyl group having 1 to 4 carbon atoms such as n-butyl, s-butyl, i-butyl, t-butyl, cyclopropylmethyl or the like in addition to "C1-C3 alkyl group."

The term "C1-C6 alkyl group" refers to a straight or branched alkyl group having 1 to 6 carbon atoms such as n-pentyl, 2-pentyl, 3-pentyl, neopentyl, n-hexyl, 2-hexyl, 4-methyl-2-pentyl, 3-methyl-n-pentyl, cyclobutylmethyl or the like in addition to "C1-C4 alkyl group."

The term "C1-C3 haloalkyl group" refers to a straight or branched alkyl group having 1 to 3 carbon atoms which is substituted with one or more halogen atoms which may be the same or different, such as monofluoromethyl, difluoromethyl, trifluoromethyl, monochloromethyl, dichloromethyl, trichloromethyl, monobromomethyl, dibromomethyl, tribromomethyl, bromodifluoromethyl, 1-fluoroethyl, 2-fluoroethyl, 2,2-difluoroethyl, 2,2,2-trifluoroethyl, 1-chloroethyl, 2-chloroethyl, 2,2-dichloroethyl, 2,2,2-trichloroethyl, 1-bromoethyl, 2-bromoethyl, 2,2-dibromoethyl, 2,2,2-tribromoethyl, 2-iodoethyl, pentafluoroethyl, 3-fluoro-n-propyl, 3-chloro-n-propyl, 3-bromo-n-propyl, 1,3-difluoro-2-propyl, 1,3-dichloro-2-propyl, 1,1,1-trifluoro-2-propyl, 1-chloro-3-fluoro-2-propyl, 1,1,1,3,3,3-hexafluoro-2-propyl, 1,1,1,3,3,3-hexafluoro-2-chloro-2-propyl, 2,2,3,3,3-pentafluoro-n-propyl, heptafluoro-i-propyl, heptafluoro-n-propyl or the like.

The term "C1-C4 haloalkyl group" refers to a straight or branched alkyl group having 1 to 4 carbon atoms which is substituted with one or more halogen atoms which may be the same or different, such as 4-fluoro-n-butyl, 4-chloro-n-butyl, nonafluoro-n-butyl, nonafluoro-2-butyl or the like in addition to "C1-C3 haloalkyl group."

The "C2-C4 alkenyl group" refers to an alkenyl group of 2 to 4 carbon atoms having a double bond in the carbon chain such as vinyl, allyl, 2-butenyl, 3-butenyl or the like, and "C2-C4 haloalkenyl group" refers to a straight or branched alkenyl group of 2 to 4 carbon atoms having a double bond in the carbon chain which is substituted with one or more halogen atoms which may be the same or different, such as 3,3-difluoro-2-propenyl, 3,3-dichloro-2-propenyl, 3,3-dibromo-2-propenyl, 2,3-dibromo-2-propenyl, 4,4-difluoro-3-butenyl, 3,4,4-tribromo-3-butenyl or the like.

The term "C2-C4 alkynyl group" refers to a straight or branched alkynyl group of 2 to 4 carbon atoms having a triple bond in the carbon chain such as propargyl, 1-butyn-3-yl, 1-butyn-3-methyl-3-yl or the like, and "C2-C4 haloalkynyl group" refers to a straight or branched alkenyl group of 2 to 4 carbon atoms having a triple bond in the carbon chain which is substituted with one or more halogen atoms which may be the same or different.

The term "C3-C6 cycloalkyl group" refers to a cycloalkyl group of 3 to 6 carbon atoms having a ring structure, such as cyclopropyl, cyclobutyl, cyclopentyl, 2-methylcyclopentyl, 3-methylcyclopentyl, cyclohexyl or the like, and "C3-C6 halocycloalkyl group" refers to a cycloalkyl group of 3 to 6 carbon atoms having a ring structure which is substituted with one or more halogen atoms which may be the same or different, such as 2,2,3,3-tetrafluorocyclobutyl, 2-chlorocyclohexyl, 4-chlorocyclohexyl or the like.

The term "C1-C3 alkoxy group" refers to a straight or branched alkoxy group having 1 to 3 carbon atoms such as methoxy, ethoxy, n-propyloxy, isopropyloxy or the like, and "C1-C4 alkoxy group" refers to a straight or branched alkoxy group having 1 to 4 carbon atoms such as n-butyloxy, isobutyloxy, s-butyloxy, t-butyloxy or the like, in addition to "C1-C3 alkoxy group."

The term "C1-C3 haloalkoxy group" refers to a straight or branched haloalkoxy group having 1 to 3 carbon atoms which is substituted with one or more halogen atoms which may be the same or different, such as trifluoromethoxy, difluoromethoxy, monofluoromethoxy, pentafluoroethoxy, 1,1,1,3,3,3-hexafluoro-2-propyloxy, 2-fluoroethoxy, 2,2-difluoroethoxy, 2,2,2-trifluoroethoxy, 2-chloroethoxy, 2,2-dichloroethoxy, 2,2,2-trichloroethoxy, 3-fluoro-n-propyloxy, 2,2,3,3-tetrafluoro-n-propyloxy, 2,2,3,3,3-pentafluoro-n-propyloxy, 1,3-difluoro-2-propyloxy, 1-chloro-3-fluoro-2-propyloxy, 1,1,2,3,3,3-hexafluoropropyloxy, 2,2,2-trifluoro-1-trifluoromethylethoxy, 2-trifluoromethoxy-1,1,2-trifluoroethoxy, 1,1,2,2-tetrafluoroethoxy or the like, and "C1-C4 haloalkoxy group" refers to a straight or branched haloalkoxy group having 1 to 4 carbon atoms which is substituted with one or more halogen atoms which may be the same or different, such as 1,1,1,3,3,4,4,4-octafluoro-2-butyloxy or the like, in addition to "C1-C3 haloalkoxy group."

The term "C1-C3 alkylthio group" refers to a straight or branched alkylthio group having 1 to 3 carbon atoms such as methylthio, ethylthio, n-propylthio, i-propylthio, cyclopropylthio or the like, "C1-C4 alkylthio group" refers to a straight or branched alkylthio group having 1 to 4 carbon atoms such as n-butylthio, i-butylthio, s-butylthio, t-butylthio, cyclopropylmethylthio or the like, in addition to "C1-C3 alkylthio group," "C1-C3 haloalkylthio group" refers to a straight

or branched alkylthio group having 1 to 3 carbon atoms which is substituted with one or more halogen atoms which may be the same or different, such as trifluoromethylthio, pentafluoroethylthio, 2-fluoroethylthio, 2,2,2-trifluoroethylthio, heptafluoro-n-propylthio, heptafluoro-i-propylthio, 1,1,2,3,3,3-hexafluoropropylthio, 2,2,2-trifluoro-1-trifluoromehylethylthio, 2-trifluoromethoxy-1,1,2-trifluoroethylthio, 1,1,2,2-tetrafluoroethylthio or the like, and "C1-C4 haloalkylthio group" refers to a straight or branched alkylthio group having 1 to 4 carbon atoms which is substituted with one or more halogen atoms which may be the same or different, such as nonafluoro-n-butylthio, nonafluoro-s-butylthio, 4,4,4-trifluoro-n-butylthio or the like, in addition to "C1-C3 haloalkylthio group."

The term "C1-C3 alkylsulfinyl group" refers to a straight or branched alkylsulfinyl group having 1 to 3 carbon atoms, such as methylsulfinyl, ethylsulfinyl, n-propylsulfinyl, i-propylsulfinyl, cyclopropylsulfinyl or the like, and "C1-C3 haloalkylsulfinyl group" refers to a straight or branched alkylsulfinyl group having 1 to 3 carbon atoms which is substituted with one or more halogen atoms which may be the same or different, such as trifluoromethylsulfinyl, pentafluoroethylsulfinyl, 2,2,2-trifluoroethylsulfinyl, heptafluoro-n-propylsulfinyl, heptafluoro-i-propylsulfinyl, 1,1,2,3,3,3-hexafluoropropylsulfinyl, 2,2,2-trifluoro-1-trifluoromethylethylsulfinyl, 2-trifluoromethoxy-1,1,2-trifluoroethylsulfinyl, 1,1,2,2-tetrafluoroethylsulfinyl or the like.

The term "C1-C3 alkylsulfonyl group" refers to a straight or branched alkylsulfonyl group having 1 to 3 carbon atoms, such as methylsulfonyl, ethylsulfonyl, n-propylsulfonyl, i-propylsulfonyl, cyclopropylsulfonyl or the like, and "C1-C3 haloalkylsulfonyl group" refers to a straight or branched alkylsulfonyl group having 1 to 3 carbon atoms which is substituted with one or more halogen atoms which may be the same or different, such as trifluoromethylsulfonyl, pentafluoroethylsulfonyl, 2,2,2-trifluoroethylsulfonyl, heptafluoro-n-propylsulfonyl, heptafluoro-i-propylsulfonyl, 1,1,2,3,3,3-hexafluoropropylsulfonyl, 2,2,2-trifluoro-1-trifluoromethylethylsulfonyl, 2-trifluoromethoxy-1,1,2-trifluoroethylsulfonyl, 1,1,2,2-tetrafluoroethylsulfonyl or the like.

The term "arylsulfonyl group" refers to an arylsulfonyl group of 6 to 14 carbon atoms having an aromatic ring, such as phenylsulfonyl, p-toluenesulfonyl, 1-naphthylsulfonyl, 2-naphthylsulfonyl, anthrylsulfonyl, phenanthrylsulfonyl, acenaphthylenylsulfonyl or the like.

The term "C1-C4 alkylamino group" refers to a straight, branched or cyclic alkylamino group having 1 to 4 carbon atoms, such as methylamino, ethylamino, n-propylamino, i-propylamino, n-butylamino, cyclopropylamino, 2,2,2-trifluoroethylamino or the like, and "di C1-C4 alkylamino group" refers to a straight or branched amino group which is substituted with two alkyl groups having 1 to 4 carbon atoms which may be the same or different, such as dimethylamino, diethylamino, N-ethyl-N-methylamino or the like.

The term "C1-C4 alkylcarbonyl group" refers to a straight, branched or cyclic alkylcarbonyl group having 1 to 4 carbon atoms, such as formyl, acetyl, propionyl, isopropylcarbonyl, cyclopropylcarbonyl or the like.

The term "C1-C4 haloalkylcarbonyl group" refers to a straight or branched alkylcarbonyl group having 1 to 4 carbon atoms which is substituted with one or more halogen atoms which may be the same or different, such as fluoroacetyl, difluoroacetyl, trifluoroacetyl, chloroacetyl, dichloroacetyl, trichloroacetyl, bromoacetyl, iodoacetyl, 3,3,3-trifluoropropionyl, 2,2,3,3,3-pentafluoropropionyl or the like.

The term "C1-C4 alkylcarbonyloxy group" refers to a straight or branched alkylcarbonyloxy group having 1 to 4 carbon atoms, such as acetoxy, propionyloxy or the like.

The term "C1-C4 alkoxycarbonyl group" refers to a straight or branched alkoxycarbonyl group having 1 to 4 carbon atoms, such as methoxycarbonyl, ethoxycarbonyl, isopropyloxycarbonyl or the like.

The term "C1-C4 perfluoroalkyl group" refers to a straight or branched alkyl group having 1 to 4 carbon atoms which is all substituted with fluorine atoms, such as trifluoromethyl, pentafluoroethyl, heptafluoro-n-propyl, heptafluoro-i-propyl, nonafluoro-n-butyl, nonafluoro-2-butyl, nonafluoro-i-butyl or the like, and "C2-C6 perfluoroalkyl group" refers to a straight or branched alkyl group having 2 to 6 carbon atoms which is all substituted with fluorine atoms, such as pentafluoroethyl, heptafluoro-n-propyl, heptafluoro-i-propyl, nonafluoro-n-butyl, nonafluoro-2-butyl, nonafluoro-i-butyl, perfluoro-n-pentyl, perfluoro-n-hexyl or the like.

The term "C1-C6 perfluoroalkylthio group" refers to a straight or branched alkylthio group having 1 to 6 carbon atoms which is all substituted with fluorine atoms, such as trifluoromethylthio, pentafluoroethylthio, heptafluoro-n-propylthio, heptafluoro-i-propylthio, nonafluoro-n-butylthio, nonafluoro-2-butylthio, nonafluoro-i-butylthio, perfluoro-n-pentylthio, perfluoro-n-hexylthio or the like.

The term "C1-C6 perfluoroalkylsulfinyl group" refers to a straight or branched alkylsulfinyl group having 1 to 6 carbon atoms which is all substituted with fluorine atoms, such as trifluoromethylsulfinyl, pentafluoroethylsulfinyl, heptafluoro-n-propylsulfinyl, heptafluoro-i-propylsulfinyl, nonafluoro-n-butylsulfinyl, nonafluoro-2-butylsulfinyl, nonafluoro-i-butylsulfinyl, perfluoro-n-pentylsulfinyl, perfluoro-n-hexylsulfinyl or the like.

The term "C1-C6 perfluoroalkylsulfonyl group" refers to a straight or branched alkylsulfonyl group having 1 to 6 carbon atoms which is all substituted with fluorine atoms, such as trifluoromethylsulfonyl, pentafluoroethylsulfonyl, heptafluoro-n-propylsulfonyl, heptafluoro-i-propylsulfonyl, nonafluoro-n-butylsulfonyl, nonafluoro-2-butylsulfonyl, nonafluoro-i-butylsulfonyl, perfluoro-n-pentylsulfonyl, perfluoro-n-hexylsulfonyl or the like.

The compound represented by the general formula (1) of the present invention contains one or more asymmetric

carbon atoms or asymmetric centers in its structural formula in some cases and has two or more optical isomers in some cases. The present invention also includes all of the respective optical isomers and mixtures consisting of these isomers in any ratio. Furthermore, the compound represented by the general formula (1) of the present invention has two or more geometrical isomers derived from a carbon-carbon double bond in its structural formula in some cases. The present invention also includes all of the respective geometrical isomers and mixtures consisting of these isomers in any ratio.

Preferable examples of the substituents or atoms in the compounds represented by the general formula (1) and the like of the present invention are listed below.

$A_1$ is preferably a carbon atom, a nitrogen atom or an oxidized nitrogen atom and at the same time, $A_2$, $A_3$ and $A_4$ are all carbon atoms, and more preferably all of $A_1$, $A_2$, $A_3$ and $A_4$ are carbon atoms as $A_1$, $A_2$, $A_3$, $A_4$,.

$R_1$ is preferably a hydrogen atom, a C1-C4 alkyl group or an acetyl group, and more preferably a hydrogen atom, a methyl group or an ethyl group.

$R_2$ is preferably a hydrogen atom, a C1-C4 alkyl group or an acetyl group, and more preferably a hydrogen atom, a methyl group or an ethyl group.

Both $G_1$ and $G_2$ are preferably oxygen atoms.

X is preferably a hydrogen atom or a halogen atom, and more preferably a hydrogen atom or a fluorine atom.

n is preferably 0, 1 or 2, and more preferably 0 or 1.

$X_1$ is preferably a hydrogen atom or a halogen atom, and more preferably a hydrogen atom or a fluorine atom.

$X_2$ is preferably a hydrogen atom or a fluorine atom, and more preferably a hydrogen atom.

$X_3$ and $X_4$ are preferably hydrogen atoms.

$Q_1$ is preferably a phenyl group, a substituted phenyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group and an acetylamino group, a phenyl group), a pyridyl group, or a substituted pyridyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group and an acetylamino group, a phenyl group), and more preferably a phenyl group, a substituted phenyl group having 1 to 3 substituents which may be the same or different (the substituents selected from a fluorine atom, a chlorine atom, a bromine atom, an iodine atom, a methyl group, a trifluoromethyl group, a methoxy group, a trifluoromethoxy group, a methylthio group, a methylsulfinyl group, a methylsulfonyl group, a trifluoromethylthio group, a trifluoromethylsulfinyl group, a trifluoromethylsulfonyl group, a methylamino group, a dimethylamino group, a cyano group and a nitro group), a pyridyl group, or a substituted pyridyl group having one or two substituents which may be the same or different (the substituents selected from a fluorine atom, a chlorine atom, a bromine atom, an iodine atom, a methyl group, a trifluoromethyl group, a methoxy group, a trifluoromethoxy group, a methylthio group, a methylsulfinyl group, a methylsulfonyl group, a trifluoromethylthio group, a trifluoromethylsulfinyl group, a trifluoromethylsulfonyl group, a methylamino group, a dimethylamino group, a cyano group and a nitro group).

$Q_2$ is preferably a substituted phenyl group or a substituted pyridyl group represented by the general formula (2) or (3), wherein $Y_1$ of general formula (2) is preferably a trifluoromethylthio group, a pentafluoroethylthio group, a heptafluoro-n-propylthio group, a heptafluoro-i-propylthio group, a trifluoromethylsulfinyl group, a pentafluoroethylsulfinyl group, a heptafluoro-n-propylsulfinyl group, a heptafluoro-i-propylsulfinyl group, a trifluoromethylsulfonyl group, a pentafluoroethylsulfonyl group, a heptafluoro-n-propylsulfonyl group, a heptafluoro-i-propylsulfonyl group, a methoxy group, an ethoxy group, a trifluoromethoxy group, a pentafluoroethoxy group, a 2-fluoroethoxy group, a 2,2-difluoroethoxy group, a 2,2,2-trifluoroethoxy group or a 2,2,2-trichloroethoxy group, and more preferably a trifluoromethylthio group, a pentafluoroethylthio group, a trifluoromethylsulfinyl group, a pentafluoroethylsulfinyl group, a trifluoromethylsulfonyl group, a pentafluoroethylsulfonyl group, a trifluoromethoxy group, a 2-fluoroethoxy group, a 2,2-difluoroethoxy group or a 2,2,2-trifluoroethoxy group; $Y_5$ of general formula (2) is preferably a fluorine atom, a chlorine atom, a bromine atom, an iodine atom, a methyl group, an ethyl group, an n-propyl group, an i-propyl group, an n-butyl group, a 2-butyl group, a trifluoromethyl group, a methylthio group, a methylsulfinyl group, a methylsulfonyl group, a trifluoromethylthio group, a pentafluoroethylthio group, a heptafluoro-i-propylthio group, a trifluoromethylsulfinyl group, a pentafluoroethylsulfinyl group, a heptafluoro-n-propylsulfinyl group, a heptafluoro-i-propylsulfinyl group, a trifluoromethylsulfonyl group, a pentafluoroethylsulfonyl group, a heptafluoro-n-propylsulfonyl group, a heptafluoro-i-propylsulfonyl group, a cyano group,

a methoxy group, an ethoxy group, an n-propyloxy group, a trifluoromethoxy group, a pentafluoroethoxy group, a 2-fluoroethoxy group, a 2,2-difluoroethoxy group, a 2,2,2-trifluoroethoxy group or a 2,2,2-trichloroethoxy group; $Y_6$ and $Y_9$ of general formula (3) are preferably each independently a fluorine atom, a chlorine atom, a bromine atom, an iodine atom, a methyl group, an ethyl group, an n-propyl group, an i-propyl group, an n-butyl group, a 2-butyl group, a trifluoromethyl group, a methylthio group, a methylsulfinyl group, a methylsulfonyl group, a trifluoromethylthio group, a pentafluoroethylthio group, a heptafluoro-i-propylthio group, a trifluoromethylsulfinyl group, a pentafluoroethylsulfinyl group, a heptafluoro-n-propylsulfinyl group, a heptafluoro-i-propylsulfinyl group, a trifluoromethylsulfonyl group, a pentafluoroethylsulfonyl group, a heptafluoro-n-propylsulfonyl group, a heptafluoro-i-propylsulfonyl group, a cyano group, a methoxy group, an ethoxy group, an n-propyloxy group, a trifluoromethoxy group, a pentafluoroethoxy group, a 2-fluoroethoxy group, a 2,2-difluoroethoxy group, a 2,2,2-trifluoroethoxy group or a 2,2,2-trichloroethoxy group; any one of $Y_6$ and $Y_9$ must be a trifluoromethylthio group, a pentafluoroethylthio group, a heptafluoro-n-propylthio group, a heptafluoro-i-propylthio group, a trifluoromethylsulfinyl group, a pentafluoroethylsulf-inyl group, a heptafluoro-n-propylsulfinyl group, a heptafluoro-i-propylsulfinyl group, a trifluoromethylsulfonyl group, a pentafluoroethylsulfonyl group, a heptafluoro-n-propylsulfonyl group, a heptafluoro-i-propylsulfonyl group, a methoxy group, an ethoxy group, a trifluoromethoxy group, a pentafluoroethoxy group, a 2-fluoroethoxy group, a 2,2-difluoroethoxy group, a 2,2,2-trifluoroethoxy group or a 2,2,2-trichloroethoxy group, and more preferably any one of $Y_6$ and $Y_9$ must be a trifluoromethylthio group, a pentafluoroethylthio group, a trifluoromethylsulfinyl group, a pentafluoroethylsulfinyl group, a trifluoromethylsulfonyl group, a pentafluoroethylsulfonyl group, a trifluoromethoxy group, a 2-fluoroethoxy group, a 2,2-difluoroethoxy group, a 2,2,2-trifluoroethoxy group or a 2,2,2-trichloroethoxy group; $Y_2$, $Y_4$ and $Y_7$ of general formula (3) are preferably each independently a hydrogen atom, a halogen atom or a methyl group, and more preferably a hydrogen atom; $Y_3$ is preferably a pentafluoroethyl group, a heptafluoro-n-propyl group, a heptafluoro-i-propyl group, a nonafluoro-n-butyl group, a nonafluoro-2-butyl group, a nonafluoro-i-butyl group, a trifluoromethylthio group, a pentafluoroethylthio group, a heptafluoro-n-propylthio group, a heptafluoro-i-propylthio group, a nonafluoro-n-butylthio group, a nonafluoro-2-butylthio group, a trifluoromethylsulfinyl group, a pentafluoroethylsulfinyl group, a heptafluoro-n-propylsulfinyl group, a heptafluoro-i-propylsulfinyl group, a nonafluoro-n-butylsulfinyl group, a nonafluoro-2-butylsulfinyl group, a trifluoromethylsulfonyl group, a pentafluoroethylsulfonyl group, a heptafluoro-n-propylsulfonyl group, a heptafluoro-i-propylsulfonyl group, a nonafluoro-n-butylsulfonyl group or a nonafluoro-2-butyl sulfonyl group, a pentafluoroethoxy group, 1, 1, 1, 3, 3, 3-hexafluoro-i-propyloxy group, 1,1,2,3,3,3-hexafluoropropyloxy group, 2,2,2-trifluoro-1-trifluoromethylethoxy group, 2-trifluoromethoxy-1,1,2-trifluoroethoxy group, 1,1,2,2-tetrafluoroethoxy group; and $Y_8$ is preferably a pentafluoroethyl group, a heptafluoro-n-propyl group, a heptafluoro-i-propyl group, a nonafluoro-n-butyl group, a nonafluoro-2-butyl group, a nonafluoro-i-butyl group, a trifluoromethylthio group, a pentafluoroethylthio group, a heptafluoro-n-propylthio group, a heptafluoro-i-propylthio group, a nonafluoro-n-butylthio group, a nonafluoro-2-butylthio group, a trifluoromethylsulfinyl group, a pentafluoroethylsulfinyl group, a heptafluoro-n-propylsulfinyl group, a heptafluoro-i-propylsulfinyl group, a nonafluoro-n-butylsulfinyl group, a nonafluoro-2-butylsulfinyl group, a trifluoromethylsulfonyl group, a pentafluoroethylsulfonyl group, a heptafluoro-n-propylsulfonyl group, a heptafluoro-i-propylsulfonyl group, a nonafluoro-n-butylsulfonyl group, a nonafluoro-2-butylsulfonyl group, a pentafluoroethoxy group, a 1,1,1,3,3,3-hexafluoro-i-propyloxy group, a 1,1,2,3,3,3-hexafluoropropyloxy group, a 2,2,2-trifluoro-1-trifluoromethylethoxy group, a 2-trifluoromethoxy-1,1,2-trifluoroethoxy group or a 1,1,2,2-tetrafluoroethoxy group.

L of after-mentioned general formula is preferably a chlorine atom, a bromine atom or a hydroxy group.

$R_1$a is preferably a hydrogen atom, a C1-C4 alkyl group or an acetyl group, and more preferably a hydrogen atom, a methyl group or an ethyl group.

$R_2$a is preferably a hydrogen atom, a C1-C4 alkyl group or an acetyl group, and more preferably a hydrogen atom, a methyl group or an ethyl group.

Both $G_1$a and $G_2$a are preferably oxygen atoms.

$X_1$a is preferably a hydrogen atom or a halogen atom, and more preferably a hydrogen atom or a fluorine atom.

$X_2$a is preferably a hydrogen atom or a fluorine atom, and more preferably a hydrogen atom.

$X_3$a and $X_4$a are preferably hydrogen atoms.

$Y_1$a is preferably a trifluoromethylthio group, a pentafluoroethylthio group, a heptafluoro-n-propylthio group, a heptafluoro-i-propylthio group, a trifluoromethylsulfinyl group, a pentafluoroethylsulfinyl group, a heptafluoro-n-propylsulfinyl group, a heptafluoro-i-propylsulfinyl group, a trifluoromethylsulfonyl group, a pentafluoroethylsulfonyl group, a heptafluoro-n-propylsulfonyl group, a heptafluoro-i-propylsulfonyl group, a methoxy group, an ethoxy group, a trifluoromethoxy group, a pentafluoroethoxy group, a 2-fluoroethoxy group, a 2,2-difluoroethoxy group, a 2,2,2-trifluoroethoxy group or a 2,2,2-trichloroethoxy group, and more preferably a trifluoromethylthio group, a pentafluoroethylthio group, a trifluoromethylsulfinyl group, a pentafluoroethylsulfinyl group, a trifluoromethylsulfonyl group, a pentafluoroethylsulfonyl group, a trifluoromethoxy group, a 2-fluoroethoxy group, a 2,2-difluoroethoxy group or a 2,2,2-trifluoroethoxy group.

$Y_2$a and $Y_4$a are each preferably a hydrogen atom, a halogen atom, a methyl group, and more preferably a hydrogen atom.

$Y_5$a is preferably a fluorine atom, a chlorine atom, a bromine atom, an iodine atom, a methyl group, an ethyl

group, an n-propyl group, an i-propyl group, an n-butyl group, a 2-butyl group, a trifluoromethyl group, a methylthio group, a methylsulfinyl group, a methylsulfonyl group, a trifluoromethylthio group, a pentafluoroethylthio group, a heptafluoro-i-propylthio group, a trifluoromethylsulfinyl group, a pentafluoroethylsulfinyl group, a heptafluoro-n-propylsulfinyl group, a heptafluoro-i-propylsulfinyl group, a trifluoromethylsulfonyl group, a pentafluoroethylsulfonyl group, a heptafluoro-n-propylsulfonyl group, a heptafluoro-i-propylsulfonyl group, a cyano group, a methoxy group, an ethoxy group, an n-propyloxy group, a trifluoromethoxy group, a pentafluoroethoxy group, a 2-fluoroethoxy group, a 2,2-difluoroethoxy group, a 2,2,2-trifluoroethoxy group or a 2,2,2-trichloroethoxy group.

$R_a$ and $R_b$ are each preferably a fluorine atom, a trifluoromethyl group, a pentafluoroethyl group or a heptafluoro-n-propyl group, and more preferably a fluorine atom, a trifluoromethyl group or a pentafluoroethyl group.

$R_c$ is preferably a hydroxy group, a chlorine atom, a bromine atom, an iodine atom, a methoxy group, an ethoxy group, a methylsulfonyloxy group, a trifluoromethylsulfonyloxy group, a phenylsulfonyloxy group, a p-toluenesulfonyloxy group, an acetoxy group or a trifluoroacetoxy group, more preferably a hydroxy group, a chlorine atom, a bromine atom, a methoxy group, a methylsulfonyloxy group, a trifluoromethylsulfonyloxy group, a phenylsulfonyloxy group or a p-toluenesulfonyloxy group, and further preferably a hydroxy group, a chlorine atom or a bromine atom.

$R_{c'}$ is preferably a hydroxy group.

$R_{c''}$ is preferably a chlorine atom or a bromine atom.

J and J' are each preferably a hydroxy group, a chlorine atom or a bromine atom, and more preferably a chlorine atom.

The compounds of present invention represented the general formula (1) may contain the following compounds I to V.

(The compound I)

The compound I is represented by the general formula (1a) in which $A_1$, $A_2$, $A_3$ and $A_4$ in the general formula (1) are all carbon atoms,

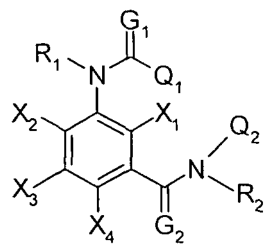
(1a)

wherein, in the formula (1a), when any one of $R_1$ and $R_2$ is a hydrogen atom, the other one is a C1-C4 alkyl group or a C1-C4 alkylcarbonyl group, or both of $R_1$ and $R_2$ are C1-C4 alkyl groups or C1-C4 alkylcarbonyl groups;

$G_1$ and $G_2$ are each independently an oxygen atom or a sulfur atom;
$X_1$, $X_2$, $X_3$ and $X_4$ are each independently a hydrogen atom, a halogen atom or a trifluoromethyl group;
$Q_1$ is a phenyl group,
a substituted phenyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group),
a heterocyclic group (The heterocyclic group herein represents a pyridyl group, a pyridine N-oxide group, a pyrimidinyl group, a pyridazyl group, a pyrazyl group, a furyl group, a thienyl group, an oxazolyl group, an isoxazolyl group, an oxadiazolyl group, a thiazolyl group, an isothiazolyl group, an imidazolyl group, a triazolyl group, a pyrrole group, a pyrazolyl group or a tetrazolyl group.), or
a substituted heterocyclic group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio

group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group) (The heterocyclic group represents the same as those described above.); and

$Q_2$ is represented by the general formula (2) or (3),

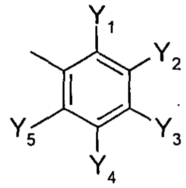

(2)

wherein, in the formula, $Y_1$ represents a Cl-C3 haloalkylthio group, a C1-C3 haloalkylsulfinyl group or a C1-C3 haloalkylsulfonyl group; $Y_5$ represents a halogen atom, aCl-C4 alkyl group, a Cl-C4 haloalkyl group, a C1-C4 alkoxy group, a C1-C4 haloalkoxy group, a C1-C3 alkylthio group, a Cl-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_3$ represents a C2-C6 perfluoroalkyl group, a C1-C6 perfluoroalkylthio group, a C1-C6 perfluoroalkylsulfinyl group or a C1-C6 perfluoroalkylsulfonyl group; and $Y_2$ and $Y_4$ each independently represents a hydrogen atom, a halogen atom or a C1-C4 alkyl group, or

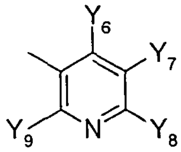

(3)

wherein, in the formula, $Y_6$ and $Y_9$ each independently represents a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_8$ represents a C1-C4 haloalkoxy group, a C2-C6 perfluoroalkyl group, a C1-C6 perfluoroalkylthio group, a C1-C6 perfluoroalkylsulfinyl group or a C1-C6 perfluoroalkylsulfonyl group; and $Y_7$ represents a hydrogen atom, a halogen atom or a C1-C4 alkyl group (Herein, at least one of $Y_6$ and $Y_9$ must represent a C1-C3 haloalkylthio group, a C1-C3 haloalkylsulfinyl group or a C1-C3 haloalkylsulfonyl group.).

As the compound I represented by the general formula (1a), a compound containing a following group may be used,

wherein, in the formula (1a), $Q_2$ is represented by the general formula (2),

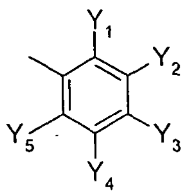

(2)

wherein, in the formula, $Y_1$ represents a C1-C3 haloalkylthio group, a C1-C3 haloalkylsulfinyl group or a C1-C3 haloalkylsulfonyl group; $Y_5$ represents a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_3$ represents a C2-C6 perfluoroalkyl group, a C1-C6 perfluoroalkylthio group, a C1-C6 perfluoroalkylsulfinyl group or a C1-C6 perfluoroalkylsulfonyl group; and $Y_2$ and $Y_4$ represent each independently a hydrogen atom, a halogen atom or a C1-C4

alkyl group.

Furthermore, as the compound I represented by the general formula (1a), a compound containing a following group may be used,
wherein, in the general formula (1a), $Q_2$ represents the above-mentioned group,

$Q_1$ represents a phenyl group,
a substituted phenyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group),
a pyridyl group, or
a substituted pyridyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group).

(The compound II)

The compound II is represented by the general formula (1a) in which $A_1$, $A_2$, $A_3$ and $A_4$ in the general formula (1) are all carbon atoms,

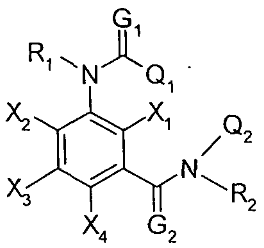 (1a)

wherein, in the formula (1a), $R_1$ and $R_2$ are each independently a hydrogen atom, a C1-C4 alkyl group or a C1-C4 alkylcarbonyl group;

$G_1$ and $G_2$ are each independently an oxygen atom or a sulfur atom;
$X_1$, $X_2$, $X_3$ and $X_4$ are each independently a hydrogen atom, a halogen atom or a trifluoromethyl group;
$Q_1$ is a substituted phenyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group),
a heterocyclic group (The heterocyclic group herein represents a pyridyl group, a pyridine N-oxide group, a pyrimidinyl group, a pyridazyl group, a pyrazyl group, a furyl group, a thienyl group, an oxazolyl group, an isoxazolyl group, an oxadiazolyl group, a thiazolyl group, an isothiazolyl group, an imidazolyl group, a triazolyl group, a pyrrole group, a pyrazolyl group or a tetrazolyl group.), or
a substituted heterocyclic group having one or more substituents which may be the same or different (the substituents selected from a halogen atom, a C1-C4 alkyl group, a C1-C4 haloalkyl group, a C2-C4 alkenyl group, a C2-C4

haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group) (The heterocyclic group represents the same as those described above.); and

$Q_2$ is represented by the general formula (2) or (3),

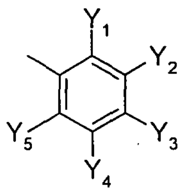

(2)

wherein, in the formula, $Y_1$ represents a C1-C3 haloalkylthio group, a C1-C3 haloalkylsulfinyl group or a C1-C3 haloalkylsulfonyl group; $Y_5$ represents a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_3$ represents a C2-C6 perfluoroalkyl group, a C1-C6 perfluoroalkylthio group, a C1-C6 perfluoroalkylsulfinyl group or a C1-C6 perfluoroalkylsulfonyl group; and $Y_2$ and $Y_9$ each independently represent a hydrogen atom, a halogen atom or a C1-C4 alkyl group, or

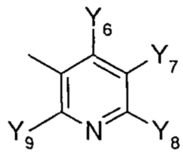

(3)

wherein, in the formula, $Y_6$ and $Y_9$ each independently represent a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_8$ represents a C1-C4 haloalkoxy group, a C2-C6 perfluoroalkyl group, a C1-C6 perfluoroalkylthio group, a C1-C6 perfluoroalkylsulfinyl group or a C1-C6 perfluoroalkylsulfonyl group; and $Y_7$ represents a hydrogen atom, a halogen atom or a C1-C4 alkyl group (Herein, at least one of $Y_6$ and $Y_9$ must represent a C1-C3 haloalkylthio group, a C1-C3 haloalkylsulfinyl group or a C1-C3 haloalkylsulfonyl group.).

As the compound II represented by the general formula (1a), a compound containing a following group may be used,

wherein, in the formula (1a), $Q_1$ represents a substituted phenyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group),

a pyridyl group, or

a substituted pyridyl group having one or more substituents which may be the same or different (the substituents selected a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy

group, a C1-C4 alkylcarbonyl group, a C1-C4 alkylcarbonyloxy group, a C1-C4 alkoxycarbonyl group, an acetylamino group and a phenyl group); and

$Q_2$ is represented by the general formula (2),

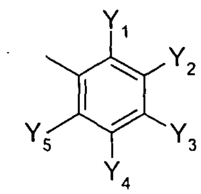

(2)

wherein, in the formula, $Y_1$ represents a C1-C3 haloalkylthio group, a C1-C3 haloalkylsulfinyl group or a C1-C3 haloalkylsulfonyl group; $Y_5$ represents a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_3$ represents a C2-C6 perfluoroalkyl group, a C1-C6 perfluoroalkylthio group, a C1-C6 perfluoroalkylsulfinyl group or a C1-C6 perfluoroalkylsulfonyl group; and $Y_2$ and $Y_4$ each independently represent a hydrogen atom, a halogen atom or a C1-C4 alkyl group

(The compound III)

The compound III is represented by the general formula (1a) in which $A_1$, $A_2$, $A_3$ and $A_4$ in the general formula (1) are all carbon atoms,

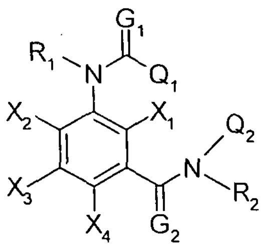

(1a)

wherein, in the formula, $R_1$ and $R_2$ are each independently a hydrogen atom, a C1-C4 alkyl group or a C1-C4 alkylcarbonyl group;

$G_1$ and $G_2$ are each independently an oxygen atom or a sulfur atom;

$X_1$, $X_2$, $X_3$ and $X_4$ are each independently a hydrogen atom, a halogen atom or a trifluoromethyl group;

$Q_1$ is a phenyl group,

a substituted phenyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group),

a heterocyclic group (The heterocyclic group herein represents a pyridyl group, a pyridine N-oxide group, a pyrimidinyl group, a pyridazyl group, a pyrazyl group, a furyl group, a thienyl group, an oxazolyl group, an isoxazolyl group, an oxadiazolyl group, a thiazolyl group, an isothiazolyl group, an imidazolyl group, a triazolyl group, a pyrrole group, a pyrazolyl group or a tetrazolyl group.), or

a substituted heterocyclic group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio

group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group) (The heterocyclic group represents the same as those described above.); and
$Q_2$ is represented by the general formula (2) or (3),

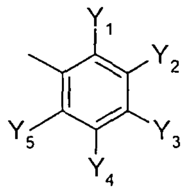
(2)

wherein, in the formula, $Y_1$ represents a C1-C3 haloalkylthio group, a C1-C3 haloalkylsulfinyl group or a C1-C3 haloalkylsulfonyl group; $Y_5$ represents a fluorine atom, a chlorine atom, an iodine atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_3$ represents a C2-C6 perfluoroalkyl group, a C1-C6 perfluoroalkylthio group, a Cl-C6 perfluoroalkylsulfinyl group or a C1-C6 perfluoroalkylsulfonyl group; and $Y_2$ and $Y_4$ each independently represent a hydrogen atom, a halogen atom or a C1-C4 alkyl group, or

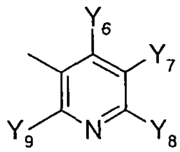
(3)

wherein, in the formula, $Y_6$ and $Y_9$ each independently representa halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_8$ represents a C1-C4 haloalkoxy group, a C2-C6 perfluoroalkyl group, a C1-C6 perfluoroalkylthio group, a C1-C6 perfluoroalkylsulfinyl group or a C1-C6 perfluoroalkylsulfonyl group; and $Y_7$ represents a hydrogen atom, a halogen atom or a C1-C4 alkyl group (Herein, at least one of $Y_6$ and $Y_9$ must represent a C1-C3 haloalkylthio group, a C1-C3 haloalkylsulfinyl group or a C1-C3 haloalkylsulfonyl group.).

As the compound III represented by the general formula (1a), a compound containing a following group may be used,
wherein, in the formula (1a), $Q_1$ represents a phenyl group,

a substituted phenyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group),
a pyridyl group, or
a substituted pyridyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group

group, an acetylamino group and a phenyl group); and

$Q_2$ is represented by the general formula (2),

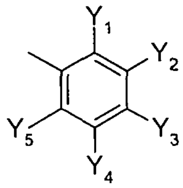

(2)

wherein, in the formula, $Y_1$ represents a C1-C3 haloalkylthio group, a C1-C3 haloalkylsulfinyl group or a C1-C3 haloalkylsulfonyl group; $Y_5$ represents a fluorine atom, a chlorine atom, an iodine atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_3$ represents a C2-C6 perfluoroalkyl group, a C1-C6 perfluoroalkylthio group, a C1-C6 perfluoroalkylsulfinyl group or a C1-C6 perfluoroalkylsulfonyl group; and $Y_2$ and $Y_4$ each independently represent a hydrogen atom, a halogen atom or a C1-C4 alkyl group.

(The compound IV)

The compound IV is represented by the general formula (1a) in which $A_1$, $A_2$, $A_3$ and $A_4$ in the general formula (1) are all carbon atoms,

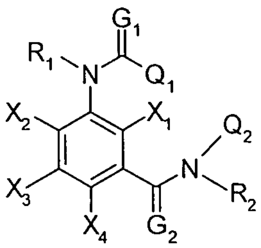

(1a)

wherein, in the formula, $R_1$ and $R_2$ are each independently a hydrogen atom, a C1-C4 alkyl group or a C1-C4 alkylcarbonyl group;

$G_1$ and $G_2$ are each independently an oxygen atom or a sulfur atom;

$X_1$, $X_2$, $X_3$ and $X_4$ are each independently a hydrogen atom, a halogen atom or a trifluoromethyl group;

$Q_1$ is a phenyl group,

a substituted phenyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group),

a heterocyclic group (The heterocyclic group herein represents a pyridyl group, a pyridine N-oxide group, a pyrimidinyl group, a pyridazyl group, a pyrazyl group, a furyl group, a thienyl group, an oxazolyl group, an isoxazolyl group, an oxadiazolyl group, a thiazolyl group, an isothiazolyl group, an imidazolyl group, a triazolyl group, a pyrrole group, a pyrazolyl group or a tetrazolyl group.), or

a substituted heterocyclic group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro

group, a hydroxy group, a C1-C4 alkylcarbonyl group, a C1-C4 alkylcarbonyloxy group, a C1-C4 alkoxycarbonyl group, an acetylamino group and a phenyl group) (The heterocyclic group represents the same as those described above.); and

$Q_2$ is represented by the general formula (2) or (3),

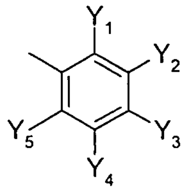

(2)

wherein, in the formula, $Y_1$ represents a C2-C3 haloalkylthio group, a C1-C3 haloalkylsulfinyl group or a C1-C3 haloalkylsulfonyl group; $Y_5$ represents a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_3$ represents a C2-C6 perfluoroalkyl group, a C1-C6 perfluoroalkylthio group, a C1-C6 perfluoroalkylsulfinyl group or a C1-C6 perfluoroalkylsulfonyl group; and $Y_2$ and $Y_4$ each independently represents a hydrogen atom, a halogen atom or a C1-C4 alkyl group; or

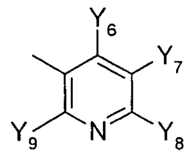

(3)

wherein, in the formula, $Y_6$ and $Y_9$ each independently represent a halogen atom, a C1-C4 alkyl group, a C1-C4 haloalkyl group, a C1-C4 alkoxy group, a C1-C4 haloalkoxy group, a C1-C3 alkylthio group, a C2-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_8$ represents a C1-C4 haloalkoxy group, a C2-C6 perfluoroalkyl group, a C1-C6 perfluoroalkylthio group, a C1-C6 perfluoroalkylsulfinyl group or a C1-C6 perfluoroalkylsulfonyl group; and $Y_7$ represents a hydrogen atom, a halogen atom or a C1-C4 alkyl group (Herein, at least one of $Y_6$ and $Y_9$ must represent a C2-C3 haloalkylthio group, a C1-C3 haloalkylsulfinyl group or a C1-C3 haloalkylsulfonyl group.).

As the compound IV represented by the general formula (1a), a compound containing a following group may be used,

wherein, in the formula (1a), $Q_1$ represents a phenyl group,

a substituted phenyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group),

a pyridyl group, or

a substituted pyridyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group); and

$Q_2$ is represented by the general formula (2),

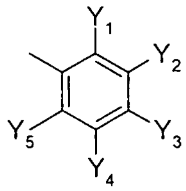

(2)

wherein, in the formula, $Y_1$ represents a C2-C3 haloalkylthio group, a C1-C3 haloalkylsulfinyl group or a C1-C3 haloalkylsulfonyl group; $Y_5$ represents a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_3$ represents a C2-C6 perfluoroalkyl group, a C1-C6 perfluoroalkylthio group, a C1-C6 perfluoroalkylsulfinyl group or a C1-C6 perfluoroalkylsulfonyl group; and $Y_2$ and $Y_4$ each independently represents a hydrogen atom, a halogen atom or a C1-C4 alkyl group.

(The compound V)

The compound V is represented by the general formula (1),

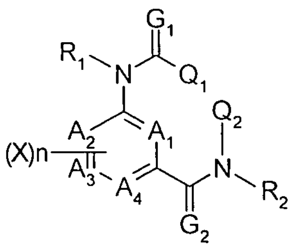

(1)

wherein, in the formula, $A_1$, $A_2$, $A_3$ and $A_4$ each independently represents a carbon atom, a nitrogen atom or an oxidized nitrogen atom;
when any one of $R_1$ and $R_2$ is a hydrogen atom, the other one is a C1-C4 alkyl group or a C1-C4 alkylcarbonyl group, or both of $R_1$ and $R_2$ are C1-C4 alkyl groups or C1-C4 alkylcarbonyl groups;

$G_1$ and $G_2$ are each independently an oxygen atom or a sulfur atom;
Xs are each independently a hydrogen atom, a halogen atom or a trifluoromethyl group;
n represents an integer of 0 to 4;
$Q_1$ is a phenyl group,
a substituted phenyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group),
a heterocyclic group (The heterocyclic group herein represents a pyridyl group, a pyridine N-oxide group, a pyrimidinyl group, a pyridazyl group, a pyrazyl group, a furyl group, a thienyl group, an oxazolyl group, an isoxazolyl group, an oxadiazolyl group, a thiazolyl group, an isothiazolyl group, an imidazolyl group, a triazolyl group, a pyrrole group, a pyrazolyl group or a tetrazolyl group.), or
a substituted heterocyclic group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro

group, a hydroxy group, a C1-C4 alkylcarbonyl group, a C1-C4 alkylcarbonyloxy group, a C1-C4 alkoxycarbonyl group, an acetylamino group and a phenyl group) (The heterocyclic group represents the same as those described above.); and

$Q_2$ is represented by the general formula (2) or (3),

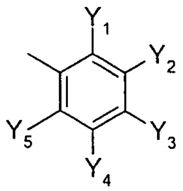
(2)

wherein, in the formula, $Y_1$ represents a C1-C4 alkoxy group or a C1-C4 haloalkoxy group; $Y_5$ represents a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_3$ represents a C2-C6 perfluoroalkyl group, a C1-C6 perfluoroalkylthio group, a C1-C6 perfluoroalkylsulfinyl group or a C1-C6 perfluoroalkylsulfonyl group; and $Y_2$ and $Y_4$ each independently represents a hydrogen atom, a halogen atom or a C1-C4 alkyl group, or

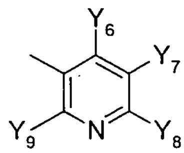
(3)

wherein, in the formula, $Y_6$ and $Y_9$ each independently represents a halogen atom, a C1μ-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_8$ represents a C1-C4 haloalkoxy group, a C2-C6 perfluoroalkyl group, a C1-C6 perfluoroalkylthio group, a C1-C6 perfluoroalkylsulfinyl group or a C1-C6 perfluoroalkylsulfonyl group; and $Y_7$ represents a hydrogen atom, a halogen atom or a C1-C4 alkyl group (Herein, at least one of $Y_6$ and $Y_9$ must represent a C1-C4 alkoxy group or a C1-C4 haloalkoxy group.).

As the compound V represented by the general formula (1), the compound represented by the general formula (1a) which $A_2$, $A_3$ and $A_4$ in the general formula (1) are all carbon atoms may be used,

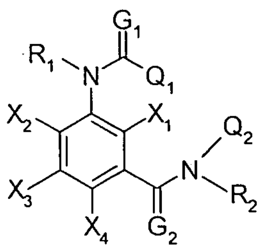
(1a)

wherein, in the formula, when any one of $R_1$ and $R_2$ is a hydrogen atom, the other one is a C1-C4 alkyl group or a C1-C4 alkylcarbonyl group, or both $R_1$ and $R_2$ are C1-C4 alkyl groups or C1-C4 alkylcarbonyl groups;

$G_1$ and $G_2$ are each independently an oxygen atom or a sulfur atom;

$X_1$, $X_2$, $X_3$ and $X_4$ are each independently a hydrogen atom, a halogen atom or a trifluoromethyl group;

$Q_1$ is a phenyl group,

a substituted phenyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio

group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group),

a heterocyclic group (The heterocyclic group herein represents a pyridyl group, a pyridine N-oxide group, a pyrimidinyl group, a pyridazyl group, a pyrazyl group, a furyl group, a thienyl group, an oxazolyl group, an isoxazolyl group, an oxadiazolyl group, a thiazolyl group, an isothiazolyl group, an imidazolyl group, a triazolyl group, a pyrrole group, a pyrazolyl group or a tetrazolyl group.), or

a substituted heterocyclic group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group) (The heterocyclic group represents the same as those described above.); and

$Q_2$ is represented by the general formula (2),

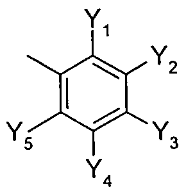
(2)

wherein, in the formula, $Y_1$ represents a C1-C4 haloalkoxy group; $Y_5$ represents a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_3$ represents a C2-C6 perfluoroalkyl group, a C1-C6 perfluoroalkylthio group, a C1-C6 perfluoroalkylsulfinyl group or a C1-C6 perfluoroalkylsulfonyl group; and $Y_2$ and $Y_4$ each independently represents a hydrogen atom, a halogen atom or a C1-C4 alkyl group.

As the compound V represented by the general formula (1a), a compound containing a following group may be used,
wherein, in the formula (1a), $Q_1$ represents a phenyl group,

a substituted phenyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group),

a pyridyl group, or

a substituted pyridyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group).

Typical preparation methods of the compound of the present invention are illustrated below. The compound of the present invention can be prepared according to the methods, but the preparation method paths are not restricted to

the following preparation methods.

In the following preparation methods, $Q_2a$ is any one of the general formula (2), (3) and (5),

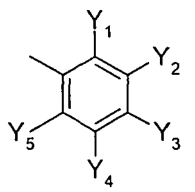

(2)

wherein, in the formula, $Y_1$, $Y_2$, $Y_3$, $Y_4$ and $Y_5$ represent the same as those described in [1],

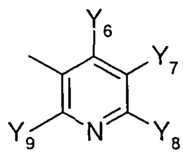

(3)

wherein, in the formula, $Y_6$, $Y_7$, $Y_8$ and $Y_9$ represent the same as those described in [1], and

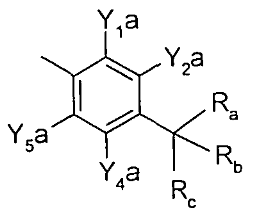

(5)

wherein, in the formula, $Y_1a$, $Y_2a$, $Y_4a$, $Y_5a$, $R_a$, $R_b$ and $R_c$ represent the same as those described in [15]. In addition, $Q_2b$ is represented by the general formula (5) in process for producing the compounds of present invention.

Preparation Method 1

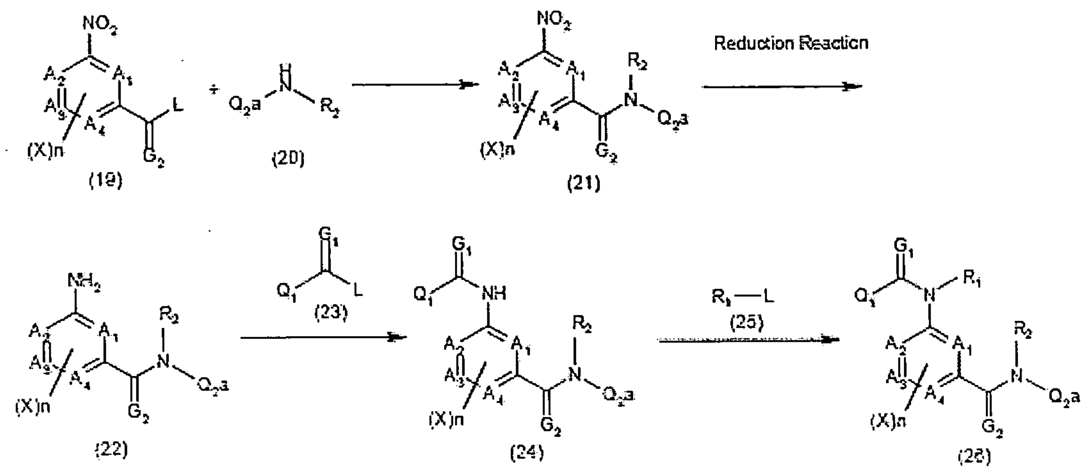


(19) (20) (21) (22) (23) (24) (25) (26)

wherein, in the formula, $A_1$, $A_2$, $A_3$, $A_4$, $G_1$, $G_2$, $R_1$, $R_2$, X, n and $Q_1$ represent the same as those described in [1]; and L represents a functional group having a leaving-group ability such as a halogen atom, a hydroxy group or the like.

1-(i): General formula (19) + General formula (20) → General formula (21)

By reacting an m-nitroaromatic carboxylic acid derivative having a leaving group represented by the general formula (19) with an aromatic amine derivative represented by the general formula (20) in a suitable solvent or without a solvent, an aromatic carboxamide derivative having a nitro group represented by the general formula (21) can be prepared. In the process, a suitable base can also be used.

Solvents may not remarkably hinder the progress of the reaction and examples thereof include water; aromatic hydrocarbons such as benzene, toluene, xylene and the like; halogenated hydrocarbons such as dichloromethane, chloroform, carbon tetrachloride and the like; chained ethers or cyclic ethers such as diethyl ether, dioxane, tetrahydrofuran, 1,2-dimethoxyethane and the like; esters such as ethyl acetate, butyl acetate and the like; alcohols such as methanol, ethanol and the like; ketones such as acetone, methyl isobutyl ketone, cyclohexanone and the like; amides such as dimethylformamide, dimethylacetamide and the like; nitriles such as acetonitrile and the like; 1,3-dimethyl-2-imidazolidinone, sulfolane, dimethylsulfoxide and the like. These solvents can be used singly or in combination of 2 or more kinds.

Furthermore, examples of the base include organic bases such as triethylamine, tri-n-butylamine, pyridine, 4-dimethylaminopyridine and the like; alkali metal hydroxides such as sodium hydroxide, potassium hydroxide and the like; carbonates such as sodium hydrogen carbonate, potassium carbonate and the like; phosphates such as di-potassium mono-hydrogen phosphate, tri-sodium phosphate and the like; alkali metal hydrides such as sodium hydride and the like; and alkali metal alcoholates such as sodium methoxide, sodium ethoxide and the like. These bases may be suitably selected in the range of 0.01 to 50 mole equivalents based on the compound represented by the general formula (19) and used accordingly.

The reaction temperature may be suitably selected in the range of -20°C to the reflux temperature of a solvent in use, while the reaction time may be properly selected in the range of several minutes to 96 hours.

On the compounds represented by the general formula (19), an aromatic carboxylic acid halide derivative can be easily prepared from an aromatic carboxylic acid according to a usual method using a halogenating agent. Examples of the halogenating agent include thionyl chloride, thionyl bromide, phosphorus oxychloride, oxalyl chloride, phosphorus trichloride, phosgene and the like.

On the other hand, a compound represented by the general formula (21) can be prepared from the m-nitroaromatic carboxylic acid in which L in the general formula (19) represents a hydroxy group and a compound represented by the general formula (20) without using a halogenating agent. As a method thereof, an additive such as 1-hydroxybenzotriazole and the like is suitably used according to a method, as described, for example, in Chem. Ber. P. 788 (1970) and a method employing a condensation agent using N,N'-dicyclohexylcarbodiimide can be exemplified. Other condensation agents to be used in this case include, for example, 1-ethyl-3-(3-dimethylaminopropyl)carbodiimide, 1,1'-carbonylbis-1H-imidazole and the like.

Furthermore, as other process for preparing the compound represented by the general formula (21), a mixed anhydride procedure using chloroformates can be cited. Also, the compound represented by the general formula (21) can be prepared according to a method as described in J. Am. Chem. Soc. P. 5012 (1967). Examples of chloroformates to be used in this case include isobutyl chloroformate, isopropyl chloroformate and the like. In addition to chloroformates, diethylacetyl chloride, trimethylacetyl chloride and the like can be cited.

Both the method using a condensation agent and mixed anhydride procedure are not restricted to the solvent, reaction temperature and reaction time as described in the above literatures, and an inert solvent which does not remarkably hinder the suitable progress of the reaction may be used. The reaction temperature and reaction time may be suitably selected as the reaction proceeds.

1-(ii): General formula (21) -> General formula (22)

An aromatic carboxamide derivative having a nitro group represented by the general formula (21) can be made into aromatic carboxamide derivative having an amino group represented by the general formula (22) by the reduction reaction. As the reduction reaction, a method employing the hydrogenation and a method employing a metallic compound (for example, tin(II) chloride (anhydride), iron powder, zinc powder and the like) can be cited.

The former method can be carried out in a proper solvent in the presence of a catalyst, in an atmospheric pressure or under an increased pressure, in a hydrogen atmosphere. Such the catalyst includes, for example, palladium catalysts such as palladium carbon and the like, nickel catalysts such as Raney nickel and the like, platinum catalysts, cobalt catalysts, ruthenium catalysts, rhodium catalysts and the like. The solvent includes, for example, water; alcohols such as methanol, ethanol and the like; aromatic hydrocarbons such as benzene, toluene and the like; chained ethers or cyclic ethers such as ether, dioxane, tetrahydrofuran and the like; and esters such as ethyl acetate and the like. The pressure may be suitably selected in the range of 0.1 to 10 MPa, the reaction temperature may be suitably selected in the range of -20°C to the reflux temperature of a solvent in use, and the reaction time may be properly selected in the

range of several minutes to 96 hours. Then, the compound of the general formula (22) can be more effectively prepared.

As the latter method, a method employing tin(IT) chloride (anhydride) as a metallic compound according to the conditions as described in "Organic Syntheses" Coll. Vol. III P. 453 can be cited.

1-(iii) : General formula (22) + General formula (23) → General formula (24)

By reacting an aromatic carboxamide derivative having an amino group represented by the general formula (22) with a compound represented by the general formula (23) in a suitable solvent or without a solvent, a compound represented by the general formula (24) of the present invention can be prepared. In this process, a suitable base can also be used.

Solvents may not remarkably hinder the progress of the reaction and examples thereof include water; aromatic hydrocarbons such as benzene, toluene, xylene and the like; halogenated hydrocarbons such as dichloromethane, chloroform, carbon tetrachloride and the like; chained ethers or cyclic ethers such as diethyl ether, dioxane, tetrahydrofuran, 1,2-dimethoxyethane and the like; esters such as ethyl acetate, butyl acetate and the like; alcohols such as methanol, ethanol and the like; ketones such as acetone, methyl isobutyl ketone, cyclohexanone and the like; amides such as dimethylformamide, dimethylacetamide and the like; nitriles such as acetonitrile and the like; and inert solvents such as 1,3-dimethyl-2-imidazolidinone and the like. These solvents can be used singly or in combination of 2 or more kinds.

Furthermore, examples of the base include organic bases such as triethylamine, tri-n-butylamine, pyridine, 4-dimethylaminopyridine and the like; alkali metal hydroxides such as sodium hydroxide, potassium hydroxide and the like; carbonates such as sodium hydrogen carbonate, potassium carbonate and the like; phosphates such as di-potassium mono-hydrogen phosphate, tri-sodium phosphate and the like; alkali metal hydrides such as sodium hydride and the like; and alkali metal alcoholates such as sodium methoxide, sodium ethoxide and the like. These bases may be suitably selected in the range of 0.01 to 50 mole equivalents based on the compound represented by the general formula (22) and used accordingly. The reaction temperature may be suitably selected in the range of -20°C to the reflux temperature of a solvent in use, while the reaction time may be properly selected in the range of several minutes to 96 hours. Furthermore, a method employing a condensation agent as described in 1-(i) or a mixed anhydride procedure can also be used.

1-(iv) : General formula (24) + General formula (25) - General formula (26)

By reacting a compound represented by the general formula (24) with an alkyl compound having a leaving group represented by the general formula (25) in a solvent or without a solvent, a compound represented by the general formula (26) can be prepared. Examples of the compound represented by the general formula (25) include alkyl halides such as methyl iodide, ethyl iodide, n-propyl bromide and the like. Furthermore, in this process, a suitable base or solvent can be used. As the base or solvent, the bases or solvents cited in 1-(i) can be used. The reaction temperature and reaction time cited in 1-(i) can be used

Furthermore, the compound represented by the general formula (26) can also be prepared by an alternative method comprising reacting an alkylating agent such as dimethyl sulfate, diethyl sulfate or the like, instead of the compound represented by the general formula (25), with the compound represented by the general formula (24).

Preparation Method 2

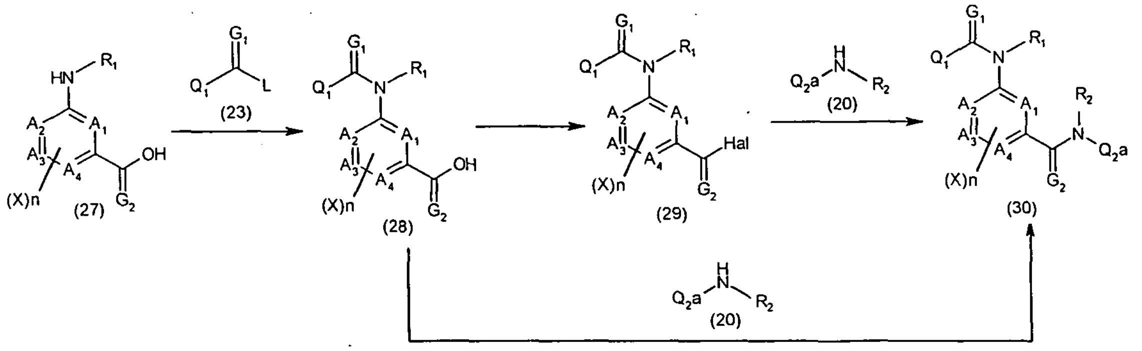

wherein, in the formula, $A_1$, $A_2$, $A_3$, $A_4$, $G_1$, $G_2$, $R_1$, $R_2$, X, n and $Q_1$ represent the same as those described in [1]; L represents a functional group having a leaving-group ability such as a halogen atom, a hydroxyl group or the like; and Hal represents a chlorine atom or a bromine atom.

2-(i): General formula (27) + General formula (23) → General formula (28)

By reacting a carboxylic acid having an amino group represented by the general formula (27) with a compound represented by the general formula (23) according to the conditions suitably selecting a solvent and a base respectively as described in 1-(i) or without a solvent or without a base, a carboxylic acid having an acylamino group represented by the general formula (28) can be prepared. The reaction time and reaction temperature can be suitably selected from the conditions as described in 1-(i).

2-(ii): General formula (28) → General formula (29)

A compound represented by the general formula (29) can be prepared by a known halogenation reaction comprising reacting a compound represented by the general formula (28) with, thionyl chloride, oxalyl chloride, phosgene, phosphorus oxychloride, phosphorus pentachloride, phosphorus trichloride, thionyl bromide, phosphorus tribromide, diethylaminosulfur trifluoride or the like.

2-(iii) : General formula (29) + General formula (20) → General formula (30)

By reacting a compound represented by the general formula (29) with a compound represented by the general formula (20) according to the conditions suitably selecting a solvent and a base respectively as described in 1- (i) or without a solvent or without a base, a compound represented by the general formula (30) can be prepared. The reaction time and reaction temperature can be suitably selected from the conditions as described in 1-(i).

2-(iv): General formula (28) + General formula (20) → General formula (30)

By reacting a compound represented by the general formula (28) with a compound represented by the general formula (20) according to the conditions using a condensation agent or a mixed anhydride procedure as described in 1-(i), a compound represented by the general formula (30) can be prepared.

Preparation Method 3

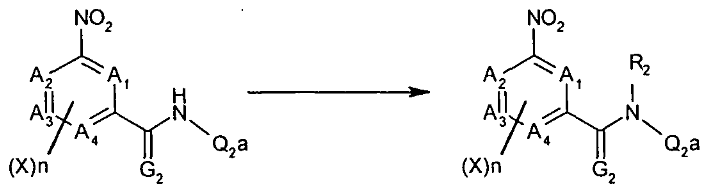

(48) (49)

wherein, in the formula, $A_1$, $A_2$, $A_3$, $A_4$, X, n, $G_2$ and $R_2$ represent the same as those described in [1].

By reacting a compound represented by the general formula (48) with a suitable reactant in a suitable solvent or without a solvent, using a suitable base, a compound represented by the general formula (49) can be prepared.

Solvents may not remarkably hinder the progress of the reaction and examples thereof include aliphatic hydrocarbons such as hexane, cyclohexane, methylcyclohexane and the like; aromatic hydrocarbons such as benzene, xylene, toluene and the like; halogenated hydrocarbons such as dichloromethane, chloroform, carbon tetrachloride, 1,2-dichloroethane and the like; ethers such as diethyl ether, dioxane, tetrahydrofuran, 1,2-dimethoxyethane and the like; amides such as dimethylformamide, dimethylacetamide and the like; nitriles such as acetonitrile, propionitrile and the like; ketones such as acetone, methyl isobutyl ketone, cyclohexanone, methylethyl ketone and the like; esters such as ethyl acetate, butyl acetate and the like; alcohols such as methanol, ethanol and the like; 1,3-dimethyl-2-imidazolidinone, sulfolane, dimethylsulfoxide, water and the like. These solvents can be used singly or in combination of 2 or more kinds.

Examples of the base include organic bases such as triethylamine, tributylamine, pyridine, 4-dimethylaminopyridine and the like; alkali metal hydroxides such as sodium hydroxide, potassium hydroxide and the like; carbonates

such as sodium hydrogen carbonate, potassium carbonate and the like; phosphates such as potassium mono-hydrogen phosphate, tri-sodium phosphate and the like; alkali metal hydrides such as sodium hydride and the like; alkali metal alkoxides such as sodium methoxide, sodium ethoxide and the like; organolithiums such as n-butyl lithium and the like; and Grignard reagents such as ethyl magnesium bromide and the like.

These bases may be suitably selected in the range of 0.01 to 50 mole equivalents based on the compound represented by the general formula (48).

Examples of the reactant include halogenated alkyls such as methyl iodide, ethyl bromide, trifluoromethyl iodide, 2,2,2-trifluoroethyl iodide and the like; halogenated allyls such as allyl iodide and the like; halogenated propargyls such as propargyl bromide and the like; halogenated acyls such as acetyl chloride and the like; acid anhydrides such as trifluoroacetic anhydride and the like; and alkyl sulfuric acids such as dimethyl sulfate, diethyl sulfate and the like. These reactants may be suitably selected in the range of 1 to 5 mole equivalents, based on the compound represented by the general formula (48) or may be used as a solvent. The reaction temperature may be suitably selected in the range of -80°C to the reflux temperature of a solvent in use, while the reaction time may be properly selected in the range of several minutes to 96 hours.

Preparation Method 4

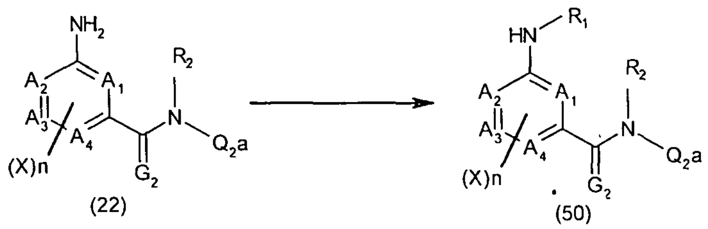

(22) (50)

wherein, in the formula, $A_1$, $A_2$, $A_3$, $A_4$, X, n, $G_2$, $R_1$ and $R_2$ represent the same as those described in [1].

4-(i): General formula (22) → General formula (50)

By reacting a compound represented by the general formula (22) with aldehydes or ketones in a suitable solvent or without a solvent, adding a suitable catalyst and reacting the resultant in a hydrogen atmosphere, a compound represented by the general formula (50) can be prepared.

Solvents may not remarkably hinder the progress of the reaction and examples thereof include aliphatic hydrocarbons such as hexane, cyclohexane, methylcyclohexane and the like; aromatic hydrocarbons such as benzene, xylene, toluene and the like; halogenated hydrocarbons such as dichloromethane, chloroform, carbon tetrachloride, 1,2-dichloroethane and the like; ethers such as diethyl ether, dioxane, tetrahydrofuran, 1,2-dimethoxyethane and the like; amides such as dimethylformamide, dimethylacetamide and the like; nitriles such as acetonitrile, propionitrile and the like; ketones such as acetone, methyl isobutyl ketone, cyclohexanone, methylethyl ketone and the like; esters such as ethyl acetate, butyl acetate and the like; alcohols such as 1,3-dimethyl-2-imidazolidinone, sulfolane, dimethylsulfoxide, methanol, ethanol and the like; water and the like. These solvents can be used singly or in combination of 2 or more kinds.

Examples of the catalyst include palladium catalysts such as palladium carbon and the like, nickel catalysts such as Raney nickel and the like, cobalt catalysts, platinum catalysts, ruthenium catalysts, rhodium catalysts and the like.

Examples of aldehydes include formaldehyde, acetoaldehyde, propionaldehyde, trifluoroacetoaldehyde, difluoroacetoaldehyde, fluoroacetoaldehyde, chloroacetoaldehyde, dichloroacetoaldehyde, trichloroacetoaldehyde, bromoacetoaldehyde and the like.

Examples of ketones include acetone, perfluoroacetone, methylethyl ketone and the like.

The reaction pressure may be suitably selected in the range of 0.1 MPa to 10MPa.

The reaction temperature may be suitably selected in the range of -20°C to the reflux temperature of a solvent in use, while the reaction time may be properly selected in the range of several minutes to 96 hours.

4-(ii) : General formula (22) → General formula (50) (Alternative method 1)

By reacting a compound represented by the general formula (22) with aldehydes or ketones in a suitable solvent or without a solvent, and applying a suitable reducing agent, a compound represented by the general formula (50) can

be prepared.

Solvents may not remarkably hinder the progress of the reaction and examples thereof include aliphatic hydrocarbons such as hexane, cyclohexane, methylcyclohexane and the like; aromatic hydrocarbons such as benzene, xylene, toluene and the like; halogenated hydrocarbons such as dichloromethane, chloroform, carbon tetrachloride, 1,2-dichloroethane and the like; ethers such as diethyl ether, dioxane, tetrahydrofuran, 1,2-dimethoxyethane and the like; amides such as dimethylformamide, dimethylacetamide and the like; nitriles such as acetonitrile, propionitrile and the like; ketones such as acetone, methyl isobutyl ketone, cyclohexanone, methylethyl ketone and the like; esters such as ethyl acetate, butyl acetate and the like; alcohols such as 1,3-dimethyl-2-imidazolidinone, sulfolane, dimethylsulfoxide, methanol, ethanol and the like; water and the like. These solvents can be used singly or in combination of 2 or more kinds.

Examples of the reducing agent include borohydrides such as sodium borohydride, sodium cyanoborohydride, sodium triacetate borohydride and the like.

Examples of aldehydes include formaldehyde, acetoaldehyde, propionaldehyde, trifluoroacetoaldehyde, difluoroacetoaldehyde, fluoroacetoaldehyde, chloroacetoaldehyde, dichloroacetoaldehyde, trichloroacetoaldehyde, bromoacetoaldehyde and the like.

Examples of ketones include acetone, perfluoroacetone, methylethyl ketone and the like.

The reaction temperature may be suitably selected in the range of -20°C to the reflux temperature of a solvent in use, while the reaction time may be properly selected in the range of several minutes to 96 hours.

4- (iii) : General formula (22) → General formula (50) (Alternative method 2)

By reacting a compound represented by the general formula (22) with a formylating agent in a suitable solvent or without a solvent and adding a suitable additive thereto, it is possible to prepare a compound, wherein, in the general formula (50), $R_1$ is a methyl group.

Solvents may not remarkably hinder the progress of the reaction and examples thereof include aliphatic hydrocarbons such as hexane, cyclohexane, methylcyclohexane and the like; aromatic hydrocarbons such as benzene, xylene, toluene and the like; halogenated hydrocarbons such as dichloromethane, chloroform, carbon tetrachloride, 1,2-dichloroethane and the like; ethers such as diethyl ether, dioxane, tetrahydrofuran, 1,2-dimethoxyethane and the like; amides such as dimethylformamide, dimethylacetamide and the like; nitriles such as acetonitrile, propionitrile and the like; ketones such as acetone, methyl isobutyl ketone, cyclohexanone, methylethyl ketone and the like; esters such as ethyl acetate, butyl acetate and the like; alcohols such as 1,3-dimethyl-2-imidazolidinone, sulfolane, dimethylsulfoxide, methanol, ethanol and the like; water and the like. These solvents can be used singly or in combination of 2 or more kinds.

Examples of the formylating agent include anhydrous formic acids such as formaldehyde, formic acid, fluoroformic acid, formyl (2,2-dimethylpropionic acid) and the like; formic acid esters such as phenyl formate and the like; pentafluorobenzaldehyde, oxazole and the like.

Examples of the additive include inorganic acids such as sulfuric acid and the like; organic acids such as formic acid and the like; borohydrides such as sodium borohydride, sodium cyanoborohydride and the like; boronic acid, lithium aluminum hydride and the like.

The reaction temperature may be suitably selected in the range of -20°C to the reflux temperature of a solvent in use, while the reaction time may be properly selected in the range of several minutes to 96 hours.

Preparation Method 5

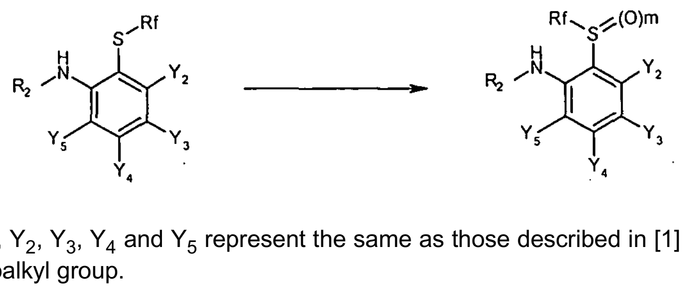

wherein, in the formula, $R_2$, $Y_2$, $Y_3$, $Y_4$ and $Y_5$ represent the same as those described in [1]; m represents 1 or 2; and Rf represents a C1-C3 haloalkyl group.

By using a suitable oxidant, a compound represented by the general formula can be prepared from a compound represented by the general formula. For example, a method described in Tetrahedron Lett. p. 4955 (1994) can be cited.

Examples of the oxidant include organic peroxides such as m-chloroperbenzoic acid and the like, sodium metaperiodate, hydrogen peroxide, ozone, selenium dioxide, chromic acid, dinitrogen tetraoxide, acyl nitrate, iodine,

bromine, N-bromosuccinimide, iodosylbenzyl, t-butyl hypochlorite and the like. The oxidant may be suitably selected in the range of 1 to 5 mole equivalents, based on the compound represented by the general formula.

A suitable additive can be added and examples thereof include ruthenium (III) chloride and the like.

The solvent used in the process is not restricted to the solvents described in the above literatures and may not remarkably hinder the progress of the reaction. These solvents can be used singly or in combination of 2 or more kinds. The reaction temperature may be suitably selected in the range of -20°C to the reflux temperature of a solvent in use, while the reaction time may be properly selected in the range of several minutes to 96 hours.

Preparation Example 6

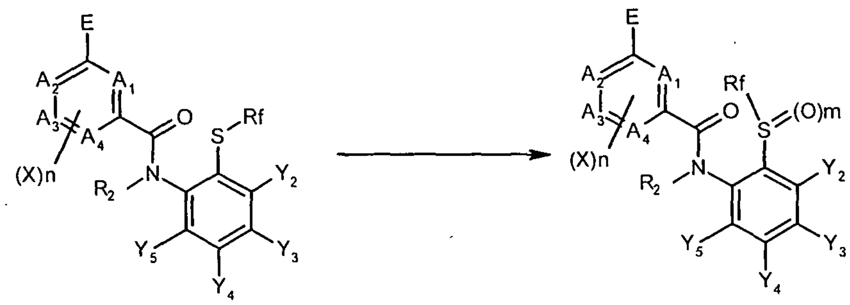

wherein, in the formula, E represents a nitro group, an amino group, a -NH-$R_1$ group or a -N ($R_1$) -C (=$G_1$) $Q_1$ group; $A_1$, $A_2$, $A_3$, $A_4$, X, n, $Y_2$, $Y_3$, $Y_4$, $Y_5$, $R_1$, $G_1$ and $Q_1$ represent the same as those described in [1]; m represents 1 or 2; and Rf represents a C1-C3 haloalkyl group.

By using a suitable oxidant, a compound represented by the general formula can be prepared from a compound represented by the general formula. The compound represented by the general formula can be prepared according to the conditions described in Preparation Method 5 using a compound represented by the general formula as a starting raw material.

Preparation Method 7

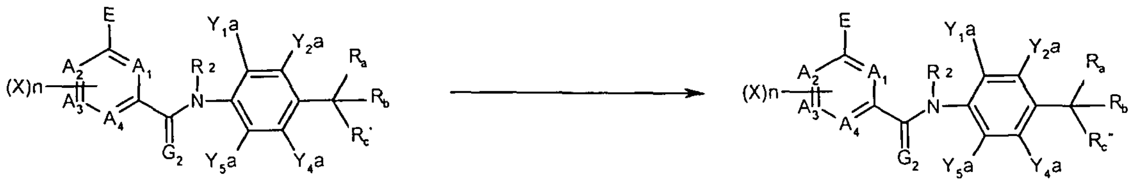

(13) (14)

wherein, in the formula, E represents a nitro group, an amino group, a -NH-$R_1$ group or a -N($R_1$)-C(=$G_1$) $Q_1$ group; $A_1$, $A_2$, $A_3$, $A_4$, X, n, $R_1$, $G_1$ and $Q_1$ represent the same as those described in [1]; and $Y_1$a, $Y_2$a, $Y_4$a, $Y_5$a, $R_a$, $R_b$, $R_{c'}$ and $R_{c''}$ represent the same as those described in [23].

By reacting a compound represented by the general formula (51) with a suitable halogenating agent in a suitable solvent or without a solvent, a chlorine compound (a bromine compound or an iodine compound) represented by the general formula (52) can be prepared. In the process, a suitable additive can also be used.

Solvents may not remarkably hinder the progress of the reaction and examples thereof include aliphatic hydrocarbons such as hexane, cyclohexane, methylcyclohexane and the like; aromatic hydrocarbons such as benzene, xylene, toluene and the like; halogenated hydrocarbons such as dichloromethane, chloroform, carbon tetrachloride, 1, 2-dichloroethane and the like; ethers such as diethyl ether, dioxane, tetrahydrofuran, 1,2-dimethoxyethane and the like; amides such as dimethylformamide, dimethylacetamide and the like; nitriles such as acetonitrile, propionitrile and the like; ketones such as acetone, methyl isobutyl ketone, cyclohexanone and the like; esters such as ethyl acetate, butyl acetate and the like; alcohols such as 1,3-dimethyl-2-imidazolidinone, sulfolane, dimethylsulfoxide, methanol, ethanol

and the like; and solvents such as water and the like. These solvents can be used singly or in combination of 2 or more kinds.

Examples of the halogenating agent include thionyl chloride, thionyl bromide, phosphorus oxychloride, oxalyl chloride, phosphorus trichloride, phosphorus tribromide, phosphorus pentachloride, Rydon reagents, sulfonyl halides such as methanesulfonyl chloride, p-toluenesulfonyl chloride, benzenesulfonyl chloride and the like, sulfonium halides, sulfonate esters, chlorine, bromine, iodine, hypohalite esters, N-halogenoamines, hydrogen chloride, hydrogen bromide, sodium bromide, potassium bromide, cyanuric chloride, 1,3-dichloro-1,2,4-triazole, titanium (IV) chloride, vanadium (IV) chloride, arsenic (III) chloride, N,N-diethyl-1,2,2-trichlorovinylamine, trichloroacetonitrile, sodium chloride, ammonium bromide, N,N-dimethylchloroforminium chloride, N,N-dimethylchloroforminium bromide, phosphorus trichloride, phosphorus tribromide, N,N-dimethyl phosphoamidine dichloride and the like.

Examples of the additive include metal salts such as zinc chloride, lithium bromide and the like; organic bases such as a phase transfer catalyst, hexamethylphosphoric triamide and the like; inorganic acids such as sulfuric acid and the like; N,N-dimethylformamide and the like.

The halogenating agent may be suitably selected in the range of 0.01 to 10 mole equivalents, based on the compound represented by the general formula (51) or may be used as a solvent.

The reaction temperature may be suitably selected in the range of -80°C to the reflux temperature of a solvent in use, while the reaction time may be properly selected in the range of several minutes to 96 hours.

Preparation Method 8

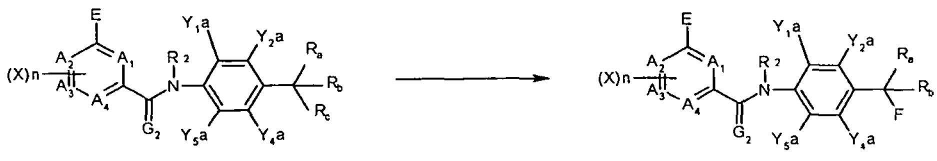

(53) (54)

wherein, in the formula, E represents a nitro group, an amino group, a -NH-$R_1$ group, a -N($R_1$)-C(=$G_1$) $Q_1$ group; $A_1$, $A_2$, $A_3$, $A_4$, X, n, $R_1$, $G_1$ and $Q_1$ represent the same as those described in [1] ; and $Y_1$a, $Y_2$a, $Y_4$a, $Y_5$a, $R_a$, $R_b$ and $R_c$ represent the same as those described in [15].

By reacting a compound represented by the general formula (53) with a suitable fluorinating agent in a suitable solvent or without a solvent, a compound represented by the general formula (54) can be prepared.

Solvents may not remarkably hinder the progress of the reaction and examples thereof include aliphatic hydrocarbons such as hexane, cyclohexane, methylcyclohexane and the like; aromatic hydrocarbons such as benzene, xylene, toluene and the like; halogenated hydrocarbons such as dichloromethane, chloroform, carbon tetrachloride, 1,2-dichloroethane and the like; ethers such as diethyl ether, dioxane, tetrahydrofuran, 1,2-dimethoxyethane and the like; amides such as dimethylformamide, dimethylacetamide and the like; nitriles such as acetonitrile, propionitrile and the like; ketones such as acetone, methyl isobutyl ketone, cyclohexanone, methylethyl ketone and the like; esters such as ethyl acetate, butyl acetate and the like; alcohols such as 1,3-dimethyl-2-imidazolidinone, sulfolane, dimethylsulfoxide, methanol, ethanol and the like; and solvents such as water and the like. These solvents can be used singly or in combination of 2 or more kinds.

Examples of the fluorinating agent include 1,1,2,2-tetrafluoroethyldiethylamine, 2-chloro-1,1,2-trifluoroethyldiethylamine, trifluorodiphenylphosphorane, difluorotriphenylphosphorane, fluoroformate esters, sulfur tetrafluoride, potassium fluoride, potassium hydrogen fluoride, cesium fluoride, rubidium fluoride, sodium fluoride, lithium fluoride, antimony (III) fluoride, antimony (V) fluoride, zinc fluoride, cobalt fluoride, lead fluoride, copper fluoride, mercury (II) fluoride, silver fluoride, silver fluoronitrate, thallium (I) fluoride, molybdenum (VI) fluoride, arsenic (III) fluoride, bromine fluoride, selenium tetrafluoride, tris(dimethylamino)sulfonium difluorotrimethylsilicate, sodium hexafluorosilicate, quaternary ammonium fluoride, (2-chloroethyl)diethylamine, diethylaminosulfur trifluoride, morpholinosulfur trifluoride, silicon tetrafluoride, hydrogen fluoride, hydrofluoric acid, hydrogen fluoride pyridine complex, hydrogen fluoride triethylamine complex, hydrogen fluoride salt, bis(2-methoxyethyl)aminosulfur trifluoride, 2,2-difluoro-1,3-dimethyl-2-imidazolidinone, iodine pentafluoride, tris(diethylamino)phosphonium-2,2,3,3,4,4-hexafluoro-cy clobutane ylide, triethylammonium hexafluorocyclobutane ylides, hexafluoropropene and the like. These fluorinating agents can be used singly or in combination of 2 or more kinds. These fluorinating agents may be suitably selected in the range of 1 to 10 mole equivalents, based on the compound represented by the general formula (53) or may be suitably used as a solvent.

An additive may also be used and examples thereof include crown ethers such as 18-crown-6 and the like;

phase transfer catalysts such as tetraphenylphosphonium salts and the like; inorganic salts such as calcium fluoride, calcium chloride and the like; metal oxides such as mercury oxide and the like; ion exchange resins and the like. These additive can be not added during the reaction and only be used as pretreatment agents for fluorinating agents.

The reaction temperature may be suitably selected in the range of -80°C to the reflux temperature of a solvent in use, while the reaction time may be properly selected in the range of several minutes to 96 hours.

Typical compounds of the compound that is an active ingredient of an insecticide of the present invention are illustrated in Tables 1 to 6 below, but the present invention is not restricted thereto.

Incidentally, in the tables, "n-" refers to normal, "Me" refers to a methyl group, "Et" refers to an ethyl group, "n-Pr" refers to a normal propyl group, "i-Pr" refers to an isopropyl group, "n-Bu" refers to a normal butyl group, "i-Bu" refers to an isobutyl group, "s-Bu" refers to a secondary butyl group, "t-Bu" refers to a tertiary butyl group, "H" refers to a hydrogen atom, "O" refers to an oxygen atom, "S" refers to a sulfur atom, "C" refers to a carbon atom, "N" refers to a nitrogen atom, "F" refers to a fluorine atom, "Cl" refers to a chlorine atom, "Br" refers to a bromine atom, "I" refers to an iodine atom, "$CF_3$" refers to a trifluoromethyl group, "$C_2F_5$" refers to a pentafluoroethyl group, "n-$C_3F_7$" refers to a heptafluoro-n-propyl group, "i-$C_3F_7$" refers to a heptafluoro-i-propyl group, "2-$C_4F_9$" refers to a nonofluoro-2-butyl group, "$SCF_3$" refers to a trifluoromethylthio group, "$SC_2F_5$" refers to a pentafluoroethylthio group, "S-n-$C_3F_7$" refers to a heptafluoro-n-propylthio group, "S(O)$CF_3$" refers to a trifluoromethylsulfinyl group, "S(O)$C_2F_5$" refers to a pentafluoroethylsulfinyl group, "S(O)-n-$C_3F_7$" refers to a heptafluoro-n-propylsulfinyl group, "$SO_2CF_3$" refers to a trifluoromethylsulfonyl group, "$SO_2C_2F_5$" refers to a pentafluoroethylsulfonyl group, "$SO_2$-n-$C_3F_7$" refers to a heptafluoro-n-propylsulfonyl group, "OMe" refers to a methoxy group, "OEt" refers to an ethoxy group, "$OCF_3$" refers to a trifluoromethoxy group, "$OCH_2CF_3$" refers to a 2,2,2-trifluoroethoxy group, "$OCH(CF_3)_2$" refers to a 1,1,1,3,3,3-hexafluoro-2-propyloxy group, and "Ac" refers to an acetyl group.

General Formula (A)

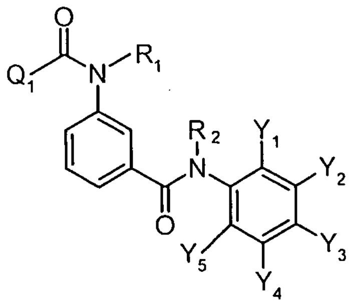

(A)

Table 1-1

| Compound No. | $R_1$ | $R_2$ | $Q_1$ | $Y_1$ | $Y_2$ | $Y_3$ | $Y_4$ | $Y_5$ |
|---|---|---|---|---|---|---|---|---|
| 1-1 | H | H | phenyl | $SCF_3$ | H | i-$C_3F_7$ | H | Cl |
| 1-2 | H | H | 2-methylphenyl | $SCF_3$ | H | i-$C_3F_7$ | H | Cl |
| 1-3 | H | H | 3-methylphenyl | $SCF_3$ | H | i-$C_3F_7$ | H | Cl |
| 1-4 | H | H | 4-methylphenyl | $SCF_3$ | H | i-$C_3F_7$ | H | Cl |
| 1-5 | H | H | 2-ethylphenyl | $SCF_3$ | H | i-$C_3F_7$ | H | Cl |
| 1-6 | H | H | 3-ethylphenyl | $SCF_3$ | H | i-$C_3F_7$ | H | Cl |
| 1-7 | H | H | 4-ethylphenyl | $SCF_3$ | H | i-$C_3F_7$ | H | Cl |
| 1-8 | H | H | 2-fluorophenyl | $SCF_3$ | H | i-$C_3F_7$ | H | Cl |
| 1-9 | H | H | 3-fluorophenyl | $SCF_3$ | H | i-$C_3F_7$ | H | Cl |
| 1-10 | H | H | 4-fluorophenyl | $SCF_3$ | H | i-$C_3F_7$ | H | Cl |
| 1-11 | H | H | 2-chlorophenyl | $SCF_3$ | H | i-$C_3F_7$ | H | Cl |
| 1-12 | H | H | 3-chlorophenyl | $SCF_3$ | H | i-$C_3F_7$ | H | Cl |

(continued)

| Compound No. | $R_1$ | $R_2$ | $Q_1$ | $Y_1$ | $Y_2$ | $Y_3$ | $Y_4$ | $Y_5$ |
|---|---|---|---|---|---|---|---|---|
| 1-13 | H | H | 4-chlorophenyl | $SCF_3$ | H | i-$C_3F_7$ | H | Cl |
| 1-14 | H | H | 2-bromophenyl | $SCF_3$ | H | i-$C_3F_7$ | H | Cl |
| 1-15 | H | H | 3-bromophenyl | $SCF_3$ | H | i-$C_3F_7$ | H | Cl |
| 1-16 | H | H | 4-bromophenyl | $SCF_3$ | H | i-$C_3F_7$ | H | Cl |
| 1-17 | H | H | 2-iodophenyl | $SCF_3$ | H | i-$C_3F_7$ | H | Cl |
| 1-18 | H | H | 3-iodophenyl | $SCF_3$ | H | i-$C_3F_7$ | H | Cl |
| 1-19 | H | H | 4-iodophenyl | $SCF_3$ | H | i-$C_3F_7$ | H | Cl |
| 1-20 | H | H | 3-cyanophenyl | $SCF_3$ | H | i-$C_3F_7$ | H | Cl |
| 1-21 | H | H | 4-cyanophenyl | $SCF_3$ | H | i-$C_3F_7$ | H | Cl |
| 1-22 | H | H | 2-nitrophenyl | $SCF_3$ | H | i-$C_3F_7$ | H | Cl |
| 1-23 | H | H | 3-nitrophenyl | $SCF_3$ | H | i-$C_3F_7$ | H | Cl |
| 1-24 | H | H | 4-nitrophenyl | $SCF_3$ | H | i-$C_3F_7$ | H | Cl |
| 1-25 | H | H | 2-aminophenyl | $SCF_3$ | H | i-$C_3F_7$ | H | Cl |
| 1-26 | H | H | 3-aminophenyl | $SCF_3$ | H | i-$C_3F_7$ | H | Cl |
| 1-27 | H | H | 4-aminophenyl | $SCF_3$ | H | i-$C_3F_7$ | H | Cl |
| 1-28 | H | H | 2-trifluoro methylphenyl | $SCF_3$ | H | i-$C_3F_7$ | H | Cl |
| 1-29 | H | H | 3-trifluoro methylphenyl | $SCF_3$ | H | i-$C_3F_7$ | H | Cl |
| 1-30 | H | H | 4-trifluoro methylphenyl | $SCF_3$ | H | i-$C_3F_7$ | H | Cl |
| 1-31 | H | H | 2-hydroxyphenyl | $SCF_3$ | H | i-$C_3F_7$ | H | Cl |
| 1-32 | H | H | 2-methoxyphenyl | $SCF_3$ | H | i-$C_3F_7$ | H | Cl |
| 1-33 | H | H | 3-methoxyphenyl | $SCF_3$ | H | i-$C_3F_7$ | H | Cl |
| 1-34 | H | H | 4-methoxyphenyl | $SCF_3$ | H | i-$C_3F_7$ | H | Cl |
| 1-35 | H | H | 2-phenoxyphenyl | $SCF_3$ | H | i-$C_3F_7$ | H | Cl |
| 1-36 | H | H | 3-(dimethyl amino)phenyl | $SCF_3$ | H | i-$C_3F_7$ | H | Cl |
| 1-37 | H | H | 4-(dimethyl amino)phenyl | $SCF_3$ | H | i-$C_3F_7$ | H | Cl |
| 1-38 | H | H | 4-trifluoro methoxyphenyl | $SCF_3$ | H | i-$C_3F_7$ | H | Cl |
| 1-39 | H | H | 2-(acetylamino)ph enyl | $SCF_3$ | H | i-$C_3F_7$ | H | Cl |
| 1-40 | H | H | 3-(acetylamino)ph enyl | $SCF_3$ | H | i-$C_3F_7$ | H | Cl |

Table 1-2

| Compound No. | $R_1$ | $R_2$ | $Q_1$ | $Y_1$ | $Y_2$ | $Y_3$ | $Y_4$ | $Y_5$ |
|---|---|---|---|---|---|---|---|---|
| 1-41 | H | H | 4-(acetylamino)phenyl | $SCF_3$ | H | i-$C_3F_7$ | H | Cl |
| 1-42 | H | H | 2-acetoxyphenyl | $SCF_3$ | H | i-$C_3F_7$ | H | Cl |
| 1-43 | H | H | 2-(methoxycarbonyl)phenyl | $SCF_3$ | H | i-$C_3F_7$ | H | Cl |
| 1-44 | H | H | 4-(methoxycarbonyl)phenyl | $SCF_3$ | H | i-$C_3F_7$ | H | Cl |
| 1-45 | H | H | 2,3-dimethylphenyl | $SCF_3$ | H | i-$C_3F_7$ | H | Cl |
| 1-46 | H | H | 2,4-dimethylphenyl | $SCF_3$ | H | i-$C_3F_7$ | H | Cl |
| 1-47 | H | H | 2,6-dimethylphenyl | $SCF_3$ | H | i-$C_3F_1$ | H | Cl |

(continued)

| Compound No. | $R_1$ | $R_2$ | $Q_1$ | $Y_1$ | $Y_2$ | $Y_3$ | $Y_4$ | $Y_5$ |
|---|---|---|---|---|---|---|---|---|
| 1-48 | H | H | 2,3-difluorophenyl | $SCF_3$ | H | i-$C_3F_1$ | H | Cl |
| 1-49 | H | H | 2,9-difluorophenyl | $SCF_3$ | H | i-$C_3F_7$ | H | Cl |
| 1-50 | H | H | 2,5-difluorophenyl | $SCF_3$ | H | i-$C_3F_7$ | H | Cl |
| 1-51 | H | H | 2,6-difluorophenyl | $SCF_3$ | H | i-$C_3F_7$ | H | Cl |
| 1-52 | H | H | 3,4-difluorophenyl | $SCF_3$ | H | i-$C_3F_7$ | H | Cl |
| 1-53 | H | H | 3,5-difluorophenyl | $SCF_3$ | H | i-$C_3F_7$ | H | Cl |
| 1-54 | H | H | 2,3-dichlorophenyl | $SCF_3$ | H | i-$C_3F_7$ | H | Cl |
| 1-55 | H | H | 2,9-dichlorophenyl | $SCF_3$ | H | i-$C_3F_7$ | H | Cl |
| 1-56 | H | H | 2,5-dichlorophenyl | $SCF_3$ | H | i-$C_3F_7$ | H | Cl |
| 1-57 | H | H | 2,6-dichlorophenyl | $SCF_3$ | H | i-$C_3F_7$ | H | Cl |
| 1-58 | H | H | 3,4-dichlorophenyl | $SCF_3$ | H | i-$C_3F_7$ | H | Cl |
| 1-59 | H | H | 3,9-dinitrophenyl | $SCF_3$ | H | i-$C_3F_7$ | H | Cl |
| 1-60 | H | H | 2,6-dimethoxyphenyl | $SCF_3$ | H | i-$C_3F_4$ | H | Cl |
| 1-61 | H | H | 3,5-dimethoxyphenyl | $SCF_3$ | H | i-$C_3F_7$ | H | Cl |
| 1-62 | H | H | 3-methyl-4-nitrophenyl | $SCF_3$ | H | i-$C_3F_7$ | H | Cl |
| 1-63 | H | H | 5-amino-2-fluorophenyl | $SCF_3$ | H | i-$C_3F_7$ | H | Cl |
| 1-64 | H | H | 3-fluoro-2-methylphenyl | $SCF_3$ | H | i-$C_3F_7$ | H | Cl |
| 1-65 | H | H | 2-fluoro-5-nitrophenyl | $SCF_3$ | H | i-$C_3F_7$ | H | Cl |
| 1-66 | H | H | 4-fluoro-3-nitrophenyl | $SCF_3$ | H | i-$C_3F_7$ | H | Cl |
| 1-67 | H | H | 5-fluoro-2-nitrophenyl | $SCF_3$ | H | i-$C_3F_7$ | H | Cl |
| 1-68 | H | H | 2-fluoro-6-iodophenyl | $SCF_3$ | H | i-$C_3F_7$ | H | Cl |
| 1-69 | H | H | 2-fluoro-5-trifluoromethylphenyl | $SCF_3$ | H | i-$C_3F_7$ | H | Cl |
| 1-70 | H | H | 2-chloro-4-nitrophenyl | $SCF_3$ | H | i-$C_3F_7$ | H | Cl |
| 1-71 | H | H | 2-chloro-4-fluorophenyl | $SCF_3$ | H | i-$C_3F_1$ | H | Cl |
| 1-72 | H | H | 2-chloro-6-fluorophenyl | $SCF_3$ | H | i-$C_3F_7$ | H | Cl |
| 1-73 | H | H | 3-chloro-4-fluorophenyl | $SCF_3$ | H | i-$C_3F_7$ | H | Cl |
| 1-74 | H | H | 4-chloro-2-fluorophenyl | $SCF_3$ | H | i-$C_3F_7$ | H | Cl |
| 1-75 | H | H | 4-chloro-2-nitrophenyl | $SCF_3$ | H | i-$C_3F_7$ | H | Cl |
| 1-76 | H | H | 3-methoxy-4-nitrophenyl | $SCF_3$ | H | i-$C_3F_7$ | H | Cl |
| 1-77 | H | H | 2-methoxy-4-nitrophenyl | $SCF_3$ | H | i-$C_3F_7$ | H | Cl |
| 1-78 | H | H | 2,3,4-trifluorophenyl | $SCF_3$ | H | i-$C_3F_7$ | H | Cl |
| 1-79 | H | H | 2,4,6-trimethylphenyl | $SCF_3$ | H | i-$C_3F_7$ | H | Cl |
| 1-80 | H | H | 2,3,6-trifluorophenyl | $SCF_3$ | H | i-$C_3F_7$ | H | Cl |

Table 1-3

| Compound No. | $R_1$ | $R_2$ | $Q_1$ | $Y_1$ | $Y_2$ | $Y_3$ | $Y_4$ | $Y_5$ |
|---|---|---|---|---|---|---|---|---|
| 1-81 | H | H | 2,4,5-trimethoxyphenyl | $SCF_3$ | H | i-$C_3F_7$ | H | Cl |
| 1-82 | H | H | 3,4,5-trimethoxyphenyl | $SCF_3$ | H | i-$C_3F_7$ | H | Cl |

(continued)

| Compound No. | $R_1$ | $R_2$ | $Q_1$ | $Y_1$ | $Y_2$ | $Y_3$ | $Y_4$ | $Y_5$ |
|---|---|---|---|---|---|---|---|---|
| 1-83 | H | H | 2,3,4,5,6-pentafluorophenyl | $SCF_3$ | H | i-$C_3F_7$ | H | Cl |
| 1-84 | H | H | 2-biphenyl | $SCF_3$ | H | i-$C_3F_7$ | H | Cl |
| 1-85 | H | H | 3-biphenyl | $SCF_3$ | H | i-$C_3F_7$ | H | Cl |
| 1-86 | H | H | 1-naphthyl | $SCF_3$ | H | i-$C_3F_7$ | H | Cl |
| 1-87 | H | H | 2-naphthyl | $SCF_3$ | H | i-$C_3F_7$ | H | Cl |
| 1-88 | H | H | pyridin-2-yl | $SCF_3$ | H | i-$C_3F_7$ | H | Cl |
| 1-89 | H | H | pyridin-3-yl | $SCF_3$ | H | i-$C_3F_7$ | H | Cl |
| 1-90 | H | H | pyridin-4-yl | $SCF_3$ | H | i-$C_3F_7$ | H | Cl |
| 1-91 | H | H | 2-methylpyridin-5-yl | $SCF_3$ | H | i-$C_3F_7$ | H | Cl |
| 1-92 | H | H | 3-methylpyridin-2-yl | $SCF_3$ | H | i-$C_3F_7$ | H | Cl |
| 1-93 | H | H | 2-fluoropyridin-3-yl | $SCF_3$ | H | i-$C_3F_7$ | H | Cl |
| 1-94 | H | H | 2-chloropyridin-3-yl | $SCF_3$ | H | i-$C_3F_7$ | H | Cl |
| 1-95 | H | H | 2-chloropyridin-4-yl | $SCF_3$ | H | i-$C_3F_7$ | H | Cl |
| 1-96 | H | H | 2-chloropyridin-6-yl | $SCF_3$ | H | i-$C_3F_7$ | H | Cl |
| 1-97 | H | H | 2-chloropyridin-5-yl | $SCF_3$ | H | i-$C_3F_7$ | H | Cl |
| 1-98 | H | H | 5-chloropyridin-2-yl | $SCF_3$ | H | i-$C_3F_7$ | H | Cl |
| 1-99 | H | H | 4-trifluoromethylpyridin-3-yl | $SCF_3$ | H | i-$C_3F_7$ | H | Cl |
| 1-100 | H | H | 3-hydroxypyridin-2-yl | $SCF_3$ | H | i-$C_3F_7$ | H | Cl |
| 1-101 | H | H | 2-phenoxypyridin-3-yl | $SCF_3$ | H | i-$C_3F_7$ | H | Cl |
| 1-102 | H | H | 2-methylthiopyridin-3-yl | $SCF_3$ | H | i-$C_3F_7$ | H | Cl |
| 1-103 | H | H | 2,6-dimethoxypyridin-3-yl | $SCF_3$ | H | i-$C_3F_7$ | H | Cl |
| 1-104 | H | H | 2,3-dichloropyridin-5-yl | $SCF_3$ | H | i-$C_3F_7$ | H | Cl |
| 1-105 | H | H | 2,5-dichloropyridin-3-yl | $SCF_3$ | H | i-$C_3F_7$ | H | Cl |
| 1-106 | H | H | 2,6-dichloropyridin-3-yl | $SCF_3$ | H | i-$C_3F_7$ | H | Cl |
| 1-107 | H | H | 3,5-dichloropyridin-9-yl | $SCF_3$ | H | i-$C_3F_7$ | H | Cl |
| 1-108 | H | H | pyridine-N-oxide-2-yl | $SCF_3$ | H | i-$C_3F_7$ | H | Cl |
| 1-109 | H | H | N-methylpyrrol-2-yl | $SCF_3$ | H | i-$C_3F_7$ | H | Cl |
| 1-110 | H | H | pyrazin-2-yl | $SCF_3$ | H | i-$C_3F_7$ | H | Cl |
| 1-111 | H | H | 2-methylpyrazin-5-yl | $SCF_3$ | H | i-$C_3F_7$ | H | Cl |
| 1-112 | H | H | 4-trifluoromethyl pyrimidin-5-yl | $SCF_3$ | H | i-$C_3F_7$ | H | Cl |
| 1-113 | H | H | furan-2-yl | $SCF_3$ | H | i-$C_3F_7$ | H | Cl |
| 1-114 | H | H | furan-3-yl | $SCF_3$ | H | i-$C_3F_7$ | H | Cl |
| 1-115 | H | H | thiophen-2-yl | $SCF_3$ | H | i-$C_3F_7$ | H | Cl |
| 1-116 | H | H | thiophen-3-yl | $SCF_3$ | H | i-$C_3F_7$ | H | Cl |
| 1-117 | H | H | 3-methylthiophen-2-yl | $SCF_3$ | H | i-$C_3F_7$ | H | Cl |
| 1-118 | H | H | 2-nitrothiophen-4-yl | $SCF_3$ | H | i-$C_3F_7$ | H | Cl |
| 1-119 | H | H | 2-methylthiophen-5-yl | $SCF_3$ | H | i-$C_3F_7$ | H | Cl |
| 1-120 | H | H | 3-chlorothiophen-2-yl | $SCF_3$ | H | i-$C_3F_7$ | H | Cl |

Table 1-4

| Compound No. | $R_1$ | $R_2$ | $Q_1$ | $Y_1$ | $Y_2$ | $Y_3$ | $Y_4$ | $Y_5$ |
|---|---|---|---|---|---|---|---|---|
| 1-121 | H | H | 2-chlorothiophen-5-yl | $SCF_3$ | H | i-$C_3F_7$ | H | Cl |
| 1-122 | H | H | 3-bromothiophen-2-yl | $SCF_3$ | H | i-$C_3F_7$ | H | Cl |
| 1-123 | H | H | 2-bromothiophen-5-yl | $SCF_3$ | H | i-$C_3F_7$ | H | Cl |
| 1-124 | H | H | 3-iodothiophen-2-yl | $SCF_3$ | H | i-$C_3F_7$ | H | Cl |
| 1-125 | H | H | 3-phenylthiophen-2-yl | $SCF_3$ | H | i-$C_3F_7$ | H | Cl |
| 1-126 | H | H | 2,4-dimethylthiophen-5-yl | $SCF_3$ | H | i-$C_3F_7$ | H | Cl |
| 1-127 | H | H | 4-nitro-1H-pyrrol-2-yl | $SCF_3$ | H | i-$C_3F_7$ | H | Cl |
| 1-128 | H | H | 3-ethyl-3H-pyrazol-4-yl | $SCF_3$ | H | i-$C_3F_7$ | H | Cl |
| 1-129 | H | H | 1-methyl-3-nitro-1H-pyrazol -4-yl | $SCF_3$ | H | i-$C_3F_7$ | H | Cl |
| 1-130 | H | H | 3-chloro-1-methyl-1H-pyrazol-4-yl | $SCF_3$ | H | i-$C_3F_7$ | H | Cl |
| 1-131 | H | H | 3-bromo-1-methyl-1H-pyrazol-4-yl | $SCF_3$ | H | i-$C_3F_7$ | H | Cl |
| 1-132 | H | H | 1-methyl-3-trifluoromethyl-1H-pyrazol-4-yl | $SCF_3$ | H | i-$C_3F_7$ | H | Cl |
| 1-133 | H | H | 1-methyl-5-trifluoromethyl-1H-pyrazol-4-yl | $SCF_3$ | H | i-$C_3F_7$ | H | Cl |
| 1-134 | H | H | isoxazol-5-yl | $SCF_3$ | H | i-$C_3F_7$ | H | Cl |
| 1-135 | H | H | 4-trifluoromethythiazol-5-yl | $SCF_3$ | H | i-$C_3F_7$ | H | C1 |
| 1-136 | H | H | 2,4-dimethylthiazol-5-yl | $SCF_3$ | H | i-$C_3F_7$ | H | Cl |
| 1-137 | H | H | 2-ethyl-4-methyl thiazol-5-yl | $SCF_3$ | H | i-$C_3F_7$ | H | Cl |
| 1-138 | H | H | 2-chloro-4-methyl thiazol-5-yl | $SCF_3$ | H | i-$C_3F_7$ | H | Cl |
| 1-139 | H | H | 3-methylisothiazol- | $SCF_3$ | H | i-$C_3F_7$ | H | Cl |
| 1-140 | H | H | 3,4-dichloro-isothiazol-5-yl | $SCF_3$ | H | i-$C_3F_7$ | H | Cl |
| 1-141 | H | H | 2-methylphenyl | $SCF_3$ | H | i-$C_3F_7$ | H | Br |
| 1-142 | H | H | 4-methylphenyl | $SCF_3$ | H | i-$C_3F_7$ | H | Br |
| 1-143 | H | H | 2-fluorophenyl | $SCF_3$ | H | i-$C_3F_7$ | H | Br |
| 1-144 | H | H | 3-fluorophenyl | $SCF_3$ | H | i-$C_3F_7$ | H | Br |
| 1-145 | H | H | 4-fluorophenyl | $SCF_3$ | H | i-$C_3F_7$ | H | Br |
| 1-146 | H | H | 4-chlorophenyl | $SCF_3$ | H | i-$C_3F_7$ | H | Br |
| 1-147 | H | H | 4-cyanophenyl | $SCF_3$ | H | i-$C_3F_7$ | H | Br |
| 1-148 | H | H | 4-nitrophenyl | $SCF_3$ | H | i-$C_3F_7$ | H | Br |
| 1-149 | H | H | 2-(trifluoromethyl)phenyl | $SCF_3$ | H | i-$C_3F_7$ | H | Br |
| 1-150 | H | H | 4-(trifluoromethyl)phenyl | $SCF_3$ | H | i-$C_3F_7$ | H | Br |
| 1-151 | H | H | 2,6-difluorophenyl | $SCF_3$ | H | i-$C_3F_7$ | H | Br |
| 1-152 | H | H | 2,4-difluorophenyl | $SCF_3$ | H | i-$C_3F_7$ | H | Br |
| 1-153 | H | H | 3-chloro-4-fluorophenyl | $SCF_3$ | H | i=$C_3F_7$ | H | Br |
| 1-154 | H | H | pyridin-2-yl | $SCF_3$ | H | i-$C_3F_7$ | H | Br |
| 1-155 | H | H | pyridin-3-yl | $SCF_3$ | H | i-$C_3F_7$ | H | Br |
| 1-156 | H | H | pyridin-4-yl | $SCF_3$ | H | i-$C_3F_7$ | H | Br |

(continued)

| Compound No. | $R_1$ | $R_2$ | $Q_1$ | $Y_1$ | $Y_2$ | $Y_3$ | $Y_4$ | $Y_5$ |
|---|---|---|---|---|---|---|---|---|
| 1-157 | H | H | 2-fluoropyridin-3-yl | $SCF_3$ | H | i-$C_3F_7$ | H | Br |
| 1-158 | H | H | 2-chloropyridin-3-yl | $SCF_3$ | H | i-$C_3F_7$ | H | Br |
| 1-159 | H | H | 2-chloropyridin-5-yl | $SCF_3$ | H | i-$C_3F_7$ | H | Br |
| 1-160 | H | H | thiophen-2-yl | $SCF_3$ | H | i-$C_3F_7$ | H | Br |

Table 1-5

| Compound No. | $R_1$ | $R_2$ | $Q_1$ | $Y_1$ | $Y_2$ | $Y_3$ | $Y_4$ | $Y_5$ |
|---|---|---|---|---|---|---|---|---|
| 1-161 | H | H | phenyl | $SCF_3$ | H | i-$C_3F_7$ | H | I |
| 1-162 | H | H | 2-fluorophenyl | $SCF_3$ | H | i-$C_3F_7$ | H | I |
| 1-163 | H | H | 4-cyanophenyl | $SCF_3$ | H | i-$C_3F_7$ | H | I |
| 1-164 | H | H | 2-fluoropyridin-3-yl | $SCF_3$ | H | i-$C_3F_7$ | H | I |
| 1-165 | H | H | 2-chloropyridin-3-yl | $SCF_3$ | H | i-$C_3F_7$ | H | I |
| 1-166 | H | H | phenyl | $SCF_3$ | H | i-$C_3F_7$ | H | Me |
| 1-167 | H | H | 2-fluorophenyl | $SCF_3$ | H | i-$C_3F_7$ | H | Me |
| 1-168 | H | H | 4-cyanophenyl | $SCF_3$ | H | i-$C_3F_7$ | H | Me |
| 1-169 | H | H | 2-fluoropyridin-3-yl | $SCF_3$ | H | i-$C_3F_7$ | H | Me |
| 1-170 | H | H | 2-chloropyridin-3-yl | $SCF_3$ | H | i-$C_3F_7$ | H | Me |
| 1-171 | H | H | phenyl | $S(O)CF_3$ | H | i-$C_3F_7$ | H | Cl |
| 1-172 | H | H | 2-fluorophenyl | $S(O)CF_3$ | H | i-$C_3F_7$ | H | Cl |
| 1-173 | H | H | 4-cyanophenyl | $S(O)CF_3$ | H | i-$C_3F_7$ | H | Cl |
| 1-174 | H | H | 2-fluoropyridin-3-yl | $S(O)CF_3$ | H | i-$C_3F_7$ | H | Cl |
| 1-175 | H | H | 2-chloropyridin-3-yl | $S(O)CF_3$ | H | i-$C_3F_7$ | H | Cl |
| 1-176 | H | H | phenyl | S (O) $CF_3$ | H | i-$C_3F_7$ | H | Br |
| 1-177 | H | H | 2-fluorophenyl | $S(O)CF_3$ | H | i-$C_3F_7$ | H | Br |
| 1-178 | H | H | 4-cyanophenyl | $S(O)CF_3$ | H | i-$C_3F_7$ | H | Br |
| 1-179 | H | H | 2-fluoropyridin-3-yl | $S(O)CF_3$ | H | i-$C_3F_7$ | H | Br |
| 1-180 | H | H | 2-chloropyridin-3-yl | $S(O)CF_3$ | H | i-$C_3F_7$ | H | Br |
| 1-181 | H | H | phenyl | S(O) $CF_3$ | H | i-$C_3F_7$ | H | Me |
| 1-182 | H | H | 2-fluorophenyl | $S(O)CF_3$ | H | i-$C_3F_7$ | H | Me |
| 1-183 | H | H | 4-cyanophenyl | $S(O)CF_3$ | H | i-$C_3F_7$ | H | Me |
| 1-184 | H | H | 2-fluoropyridin-3-yl | $S(O)CF_3$ | H | i-$C_3F_7$ | H | Me |
| 1-185 | H | H | 2-chloropyridin-3-yl | $S(O)CF_3$ | H | i-$C_3F_7$ | H | Me |
| 1-186 | H | H | phenyl | $SO_2CF_3$ | H | i-$C_3F_7$ | H | Cl |
| 1-187 | H | H | 2-fluorophenyl | $SO_2CF_3$ | H | i-$C_3F_7$ | H | Cl |
| 1-188 | H | H | 4-cyanophenyl | $SO_2CF_3$ | H | i-$C_3F_7$ | H | Cl |
| 1-189 | H | H | 2-fluoropyridin-3-yl | $SO_2CF_3$ | H | i-$C_3F_7$ | H | Cl |
| 1-190 | H | H | 2-chloropyridin-3-yl | $SO_2CF_3$ | H | i-$C_3F_7$ | H | Cl |
| 1-191 | H | H | phenyl | $SO_2CF_3$ | H | i-$C_3F_7$ | H | Br |

(continued)

| Compound No. | $R_1$ | $R_2$ | $Q_1$ | $Y_1$ | $Y_2$ | $Y_3$ | $Y_4$ | $Y_5$ |
|---|---|---|---|---|---|---|---|---|
| 1-192 | H | H | 2-fluorophenyl | $SO_2CF_3$ | H | i-$C_3F_7$ | H | Br |
| 1-193 | H | H | 4-cyanophenyl | $SO_2CF_3$ | H | i-$C_3F_7$ | H | Br |
| 1-194 | H | H | 2-fluoro pyridin-3-yl | $SO_2CF_3$ | H | i-$C_3F_7$ | H | Br |
| 1-195 | H | H | 2-chloro pyridin-3-yl | $SO_2CF_3$ | H | i-$C_3F_7$ | H | Br |
| 1-196 | H | H | phenyl | $SO_2CF_3$ | H | i-$C_3F_7$ | H | Me |
| 1-197 | H | H | 2-fluorophenyl | $SO_2CF_3$ | H | i-$C_3F_7$ | H | Me |
| 1-198 | H | H | 4-cyanophenyl | $SO_2CF_3$ | H | i-$C_3F_7$ | H | Me |
| 1-199 | H | H | 2-fluoro pyridin-3-yl | $SO_2CF_3$ | H | i-$C_3F_7$ | H | Me |
| 1-200 | H | H | 2-chloro pyridin-3-yl | $SO_2CF_3$ | H | i-$C_3F_7$ | H | Me |

Table 1-6

| Compound No. | $R_1$ | $R_2$ | $Q_1$ | $Y_1$ | $Y_2$ | $Y_3$ | $Y_4$ | $Y_5$ |
|---|---|---|---|---|---|---|---|---|
| 1-201 | H | H | phenyl | $SC_2F_5$ | H | i-$C_3F_7$ | H | Br |
| 1-202 | H | H | 2-fluorophenyl | $SC_2F_5$ | H | i-$C_3F_7$ | H | Br |
| 1-203 | H | H | 2-chloro pyridin-3-yl | $SC_2F_5$ | H | i-$C_3F_7$ | H | Br |
| 1-204 | H | H | phenyl | S-n-$C_3F_7$ | H | i-$C_3F_7$ | H | Br |
| 1-205 | H | H | 2-fluorophenyl | S-n-$C_3F_7$ | H | i-$C_3F_7$ | H | Br |
| 1-206 | H | H | 2-chloro pyridin-3-yl | S-n-$C_3F_7$ | H | i-$C_3F_7$ | H | Br |
| 1-207 | H | H | phenyl | $SCF_3$ | H | n-$C_3F_7$ | H | Br |
| 1-208 | H | H | 2-fluorophenyl | $SCF_3$ | H | n-$C_3F_7$ | H | Br |
| 1-209 | H | H | 2-chloro pyridin-3-yl | $SCF_3$ | H | n-$C_3F_7$ | H | Br |
| 1-210 | H | H | phenyl | $SCF_3$ | H | $C_2F_5$ | H | Cl |
| 1-211 | H | H | phenyl | $S(O)CF_3$ | H | $C_2F_5$ | H | Cl |
| 1-212 | H | H | phenyl | $SO_2CF_3$ | H | $C_2F_5$ | H | Cl |
| 1-213 | H | H | phenyl | $SCF_3$ | H | $C_2F_5$ | H | Br |
| 1-214 | H | H | phenyl | $S(O)CF_3$ | H | $C_2F_5$ | H | Br |
| 1-215 | H | H | phenyl | $SO_2CF_3$ | H | $C_2F_5$ | H | Br |
| 1-216 | H | H | phenyl | $SCF_3$ | H | $C_2F_5$ | H | Me |
| 1-217 | H | H | phenyl | $S(O)CF_3$ | H | $C_2F_5$ | H | Me |
| 1-218 | H | H | phenyl | $SO_2CF_3$ | H | $C_2F_5$ | H | Me |
| 1-219 | H | H | phenyl | $SCF_3$ | H | 2-$C_4F_9$ | H | Cl |
| 1-220 | H | H | phenyl | $S(O)CF_3$ | H | 2-$C_4F_9$ | H | Cl |
| 1-221 | H | H | phenyl | $SO_2CF_3$ | H | 2-$C_4F_9$ | H | Cl |
| 1-222 | H | H | phenyl | $SCF_3$ | H | 2-$C_4F_9$ | H | Br |
| 1-223 | H | H | phenyl | $S(O)CF_3$ | H | 2-$C_4F_9$ | H | Br |
| 1-224 | H | H | phenyl | $SO_2CF_3$ | H | 2-$C_4F_9$ | H | Br |
| 1-225 | H | H | phenyl | $SCF_3$ | H | 2-$C_4F_9$ | H | Me |
| 1-226 | H | H | phenyl | $S(O)CF_3$ | H | 2-$C_4F_9$ | H | Me |

(continued)

| Compound No. | $R_1$ | $R_2$ | $Q_1$ | $Y_1$ | $Y_2$ | $Y_3$ | $Y_4$ | $Y_5$ |
|---|---|---|---|---|---|---|---|---|
| 1-227 | H | H | phenyl | $SO_2CF_3$ | H | 2-$C_4F_9$ | H | Me |
| 1-228 | H | H | 2-chloro pyridin-3-yl | $SCF_3$ | H | 2-$C_4F_9$ | H | Cl |
| 1-229 | H | H | 2-chloro pyridin-3-yl | S(O)$CF_3$ | H | 2-$C_4F_9$ | H | Cl |
| 1-230 | H | H | 2-chloro pyridin-3-yl | $SO_2CF_3$ | H | 2-$C_4F_9$ | H | Cl |
| 1-231 | H | H | 2-chloro yridin-3-yl | $SCF_3$ | H | 2-$C_4F_9$ | H | Br |
| 1-232 | H | H | 2-chloro pyridin-3-yl | S(O) $CF_3$ | H | 2-$C_4F_9$ | H | Br |
| 1-233 | H | H | 2-chloro pyridin-3-yl | $SO_2CF_3$ | H | 2-$C_4F_9$ | H | Br |
| 1-234 | H | H | 2-chloro pyridin-3-yl | $SCF_3$ | H | 2-$C_4F_9$ | H | Me |
| 1-235 | H | H | 2-chloro pyridin-3-yl | $SCF_3$ | Me | i-$C_3F_7$ | H | Br |
| 1-236 | H | H | 2-chloro pyridin-3-yl | $SCF_3$ | H | i-$C_3F_7$ | Me | Br |
| 1-237 | H | H | 2-chloro pyridin-3-yl | $SCF_3$ | Cl | i-$C_3F_7$ | H | Br |
| 1-238 | H | H | 2-chloro pyridin-3-yl | $SCF_3$ | Me | i-$C_3F_7$ | H | Br |
| 1-239 | H | H | 2-chloro pyridin-3-yl | $SCF_3$ | H | i-$C_3F_7$ | Me | Br |
| 1-240 | H | H | 2-chloro pyridin-3-yl | $SCF_3$ | Br | i-$C_3F_7$ | H | Br |

Table 1-7

| Compound No. | $R_1$ | $R_2$ | $Q_1$ | $Y_1$ | $Y_2$ | $Y_3$ | $Y_4$ | $Y_5$ |
|---|---|---|---|---|---|---|---|---|
| 1-241 | H | H | phenyl | $OCF_3$ | H | i-$C_3F_7$ | H | Cl |
| 1-242 | H | H | 2-fluorophenyl | $OCF_3$ | H | i-$C_3F_7$ | H | Cl |
| 1-243 | H | H | phenyl | $OCF_3$ | H | i-$C_3F_7$ | H | Br |
| 1-244 | H | H | 2-fluorophenyl | $OCF_3$ | H | i-$C_3F_7$ | H | Br |
| 1-245 | H | H | 4-nitrophenyl | $OCF_3$ | H | i-$C_3F_7$ | H | Br |
| 1-246 | H | H | 4-cyanophenyl | $OCF_3$ | H | i-$C_3F_7$ | H | Br |
| 1-247 | H | H | 4-fluorophenyl | $OCF_3$ | H | i-$C_3F_7$ | H | Br |
| 1-248 | H | H | 4-(trifluoro methyl)phenyl | $OCF_3$ | H | i-$C_3F_7$ | H | Br |
| 1-249 | H | H | 4-chlorophenyl | $OCF_3$ | H | i-$C_3F_7$ | H | Br |
| 1-250 | H | H | phenyl | $OCF_3$ | H | 2-$C_4F_9$ | H | Br |
| 1-251 | H | H | 2-fluorophenyl | $OCF_3$ | H | 2-$C_4F_9$ | H | Br |
| 1-252 | H | H | 2-chloro pyridin-3-yl | $OCF_3$ | H | 2-$C_4F_9$ | H | Br |
| 1-253 | H | H | phenyl | OMe | H | i-$C_3F_7$ | H | Br |
| 1-254 | H | H | 2-fluorophenyl | OMe | H | i-$C_3F_7$ | H | Br |
| 1-255 | H | H | 4-fluorophenyl | OMe | H | i-$C_3F_7$ | H | Br |
| 1-256 | H | H | 2-chloro pyridin-3-yl | OMe | H | i-$C_3F_7$ | H | Br |
| 1-257 | H | H | phenyl | OMe | H | i-$C_3F_7$ | H | I |
| 1-258 | H | H | 2-fluorophenyl | OMe | H | i-$C_3F_7$ | H | I |
| 1-259 | H | H | 2-chloro pyridin-3-yl | OMe | H | i-$C_3F_7$ | H | I |
| 1-260 | H | H | phenyl | OMe | H | i-$C_3F_7$ | H | Me |
| 1-261 | H | H | 2-chloro pyridin-3-yl | OMe | H | i-$C_3F_7$ | H | Me |

(continued)

| Compound No. | $R_1$ | $R_2$ | $Q_1$ | $Y_1$ | $Y_2$ | $Y_3$ | $Y_4$ | $Y_5$ |
|---|---|---|---|---|---|---|---|---|
| 1-262 | H | H | 2-fluorophenyl | OMe | H | i-$C_3F_7$ | H | Me |
| 1-263 | H | H | phenyl | OMe | H | i-$C_3F_7$ | H | Cl |
| 1-264 | H | H | 2-fluorophenyl | OMe | H | i-$C_3F_7$ | H | Cl |
| 1-265 | H | H | 2-chloro pyridin-3-yl | OMe | H | i-$C_3F_7$ | H | Cl |
| 1-266 | H | H | 4-cyanophenyl | OMe | H | i-$C_3F_7$ | H | Br |
| 1-267 | H | H | pyridin-2-yl | OMe | H | i-$C_3F_7$ | H | Br |
| 1-268 | H | H | pyridin-3-yl | OMe | H | i-$C_3F_7$ | H | Br |
| 1-269 | H | H | phenyl | $OCH_2CF_3$ | H | i-$C_3F_7$ | H | Br |
| 1-270 | H | H | 2-chloro pyridin-3-yl | $OCH_2CF_3$ | H | i-$C_3F_7$ | H | Br |
| 1-271 | H | H | phenyl | OMe | H | $C_2F_5$ | H | Br |
| 1-272 | H | H | 2-chloro pyridin-3-yl | OMe | H | $C_2F_5$ | H | Br |
| 1-273 | H | H | phenyl | OEt | H | i-$C_3F_7$ | H | Br |
| 1-274 | H | H | 2-chloro pyridin-3-yl | OEt | H | i-$C_3F_7$ | H | Br |
| 1-275 | H | H | phenyl | OMe | Me | i-$C_3F_7$ | H | Br |
| 1-276 | H | H | phenyl | OMe | H | i-$C_3F_7$ | Me | Br |
| 1-277 | H | H | phenyl | OMe | Cl | i-$C_3F_7$ | H | Br |
| 1-278 | H | H | phenyl | OMe | Me | i-$C_3F_7$ | H | Br |
| 1-279 | H | H | phenyl | OMe | H | i-$C_3F_7$ | Me | Br |
| 1-280 | H | H | phenyl | OMe | Br | i-$C_3F_7$ | H | Br |

General Formula (A)

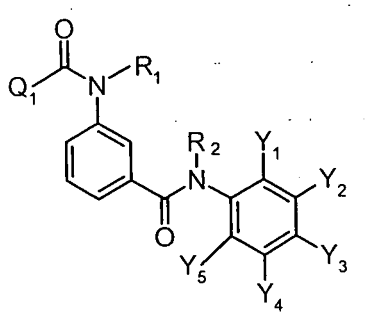

(A)

Table 2-1

| Compound No. | $R_1$ | $R_2$ | $Q_1$ | $Y_1$ | $Y_2$ | $Y_3$ | $Y_4$ | $Y_5$ |
|---|---|---|---|---|---|---|---|---|
| 2-1 | Me | H | phenyl | $SCF_3$ | H | i-$C_3F_7$ | H | Cl |
| 2-2 | Me | H | 2-methylphenyl | $SCF_3$ | H | i-$C_3F_7$ | H | Cl |
| 2-3 | Me | H | 3-methylphenyl | $SCF_3$ | H | i-$C_3F_7$ | H | Cl |
| 2-4 | Me | H | 4-methylphenyl | $SCF_3$ | H | i-$C_3F_7$ | H | Cl |
| 2-5 | Me | H | 2-ethylphenyl | $SCF_3$ | H | i-$C_3F_7$ | H | Cl |

(continued)

| Compound No. | $R_1$ | $R_2$ | $Q_1$ | $Y_1$ | $Y_2$ | $Y_3$ | $Y_4$ | $Y_5$ |
|---|---|---|---|---|---|---|---|---|
| 2-6 | Me | H | 3-ethylphenyl | $SCF_3$ | H | i-$C_3F_7$ | H | Cl |
| 2-7 | Me | H | 4-ethylphenyl | $SCF_3$ | H | i-$C_3F_7$ | H | Cl |
| 2-8 | Me | H | 2-fluorophenyl | $SCF_3$ | H | i-$C_3F_7$ | H | Cl |
| 2-9 | Me | H | 3-fluorophenyl | $SCF_3$ | H | i-$C_3F_7$ | H | Cl |
| 2-10 | Me | H | 4-fluorophenyl | $SCF_3$ | H | i-$C_3F_7$ | H | Cl |
| 2-11 | Me | H | 2-chlorophenyl | $SCF_3$ | H | i-$C_3F_7$ | H | Cl |
| 2-12 | Me | H | 3-chlorophenyl | $SCF_3$ | H | i-$C_3F_7$ | H | Cl |
| 2-13 | Me | H | 4-chlorophenyl | $SCF_3$ | H | i-$C_3F_7$ | H | Cl |
| 2-14 | Me | H | 2-bromophenyl | $SCF_3$ | H | i-$C_3F_7$ | H | Cl |
| 2-15 | Me | H | 3-bromophenyl | $SCF_3$ | H | i-$C_3F_7$ | H | Cl |
| 2-16 | Me | H | 4-bromophenyl | $SCF_3$ | H | i-$C_3F_7$ | H | Cl |
| 2-17 | Me | H | 2-iodophenyl | $SCF_3$ | H | i-$C_3F_7$ | H | Cl |
| 2-18 | Me | H | 3-iodophenyl | $SCF_3$ | H | i-$C_3F_7$ | H | Cl |
| 2-19 | Me | H | 4-iodophenyl | $SCF_3$ | H | i-$C_3F_7$ | H | Cl |
| 2-20 | Me | H | 3-cyanophenyl | $SCF_3$ | H | i-$C_3F_7$ | H | Cl |
| 2-21 | Me | H | 4-cyanophenyl | $SCF_3$ | H | i-$C_3F_7$ | H | Cl |
| 2-22 | Me | H | 2-nitrophenyl | $SCF_3$ | H | i-$C_3F_7$ | H | Cl |
| 2-23 | Me | H | 3-nitrophenyl | $SCF_3$ | H | i-$C_3F_7$ | H | Cl |
| 2-24 | Me | H | 4-nitrophenyl | $SCF_3$ | H | i-$C_3F_7$ | H | Cl |
| 2-25 | Me | H | 2-aminophenyl | $SCF_3$ | H | i-$C_3F_7$ | H | Cl |
| 2-26 | Me | H | 3-aminophenyl | $SCF_3$ | H | i-$C_3F_7$ | H | Cl |
| 2-27 | Me | H | 4-aminophenyl | $SCF_3$ | H | i-$C_3F_7$ | H | Cl |
| 2-28 | Me | H | 2-trifluoro methylphenyl | $SCF_3$ | H | i-$C_3F_7$ | H | Cl |
| 2-29 | Me | H | 3-trifluoro methylphenyl | $SCF_3$ | H | i-$C_3F_7$ | H | Cl |
| 2-30 | Me | H | 4-trifluoro methylphenyl | $SCF_3$ | H | i-$C_3F_7$ | H | Cl |
| 2-31 | Me | H | 2-hydroxyphenyl | $SCF_3$ | H | i-$C_3F_7$ | H | Cl |
| 2-32 | Me | H | 2-methoxyphenyl | $SCF_3$ | H | i-$C_3F_7$ | H | Cl |
| 2-33 | Me | H | 3-methoxyphenyl | $SCF_3$ | H | i-$C_3F_7$ | H | Cl |
| 2-34 | Me | H | 4-methoxyphenyl | $SCF_3$ | H | i-$C_3F_7$ | H | Cl |
| 2-35 | Me | H | 2-phenoxyphenyl | $SCF_3$ | H | i-$C_3F_7$ | H | Cl |
| 2-36 | Me | H | 3-(dimethyl amino)phenyl | $SCF_3$ | H | i-$C_3F_7$ | H | C1 |
| 2-37 | Me | H | 4-(dimethyl amino)phenyl | $SCF_3$ | H | i-$C_3F_7$ | H | Cl |
| 2-38 | Me | H | 4-trifluoro methoxyphenyl | $SCF_3$ | H | i-$C_3F_7$ | H | Cl |
| 2-39 | Me | H | 2-(acetylamino)phenyl | $SCF_3$ | H | i-$C_3F_7$ | H | Cl |
| 2-40 | Me | H | 3-(acetylamino)phen yl | $SCF_3$ | H | i-$C_3F_7$ | H | Cl |

Table 2-2

| Compound No. | $R_1$ | $R_2$ | $Q_1$ | $Y_1$ | $Y_2$ | $Y_3$ | $Y_4$ | $Y_5$ |
|---|---|---|---|---|---|---|---|---|
| 2-41 | Me | H | 4-(acetylamino)phenyl | $SCF_3$ | H | i-$C_3F_7$ | H | Cl |
| 2-42 | Me | H | 2-acetoxyphenyl | $SCF_3$ | H | i-$C_3F_7$ | H | Cl |
| 2-43 | Me | H | 2-(methoxycarbonyl)phenyl | $SCF_3$ | H | i-$C_3F_7$ | H | Cl |
| 2-44 | Me | H | 4-(methoxycarbonyl)phenyl | $SCF_3$ | H | i-$C_3F_7$ | H | Cl |
| 2-45 | Me | H | 2,3-dimethylphenyl | $SCF_3$ | H | i-$C_3F_7$ | H | Cl |
| 2-46 | Me | H | 2,4-dimethylphenyl | $SCF_3$ | H | i-$C_3F_7$ | H | Cl |
| 2-47 | Me | H | 2,6-dimethylphenyl | $SCF_3$ | H | i-$C_3F_7$ | H | Cl |
| 2-48 | Me | H | 2,3-difluorophenyl | $SCF_3$ | H | i-$C_3F_7$ | H | Cl |
| 2-49 | Me | H | 2,4-difluorophenyl | $SCF_3$ | H | i-$C_3F_7$ | H | Cl |
| 2-50 | Me | H | 2,5-difluorophenyl | $SCF_3$ | H | i-$C_3F_7$ | H | Cl |
| 2-51 | Me | H | 2,6-difluorophenyl | $SCF_3$ | H | i-$C_3F_7$ | H | Cl |
| 2-52 | Me | H | 3,4-difluorophenyl | $SCF_3$ | H | i-$C_3F_7$ | H | Cl |
| 2-53 | Me | H | 3,5-difluorophenyl | $SCF_3$ | H | i-$C_3F_7$ | H | Cl |
| 2-54 | Me | H | 2,3-dichlorophenyl | $SCF_3$ | H | i-$C_3F_7$ | H | Cl |
| 2-55 | Me | H | 2,4-dichlorophenyl | $SCF_3$ | H | i-$C_3F_7$ | H | Cl |
| 2-56 | Me | H | 2,5-dichlorophenyl | $SCF_3$ | H | i-$C_3F_7$ | H | Cl |
| 2-57 | Me | H | 2,6-dichlorophenyl | $SCF_3$ | H | i-$C_3F_7$ | H | Cl |
| 2-58 | Me | H | 3,4-dichlorophenyl | $SCF_3$ | H | i-$C_3F_7$ | H | Cl |
| 2-59 | Me | H | 3,4-dinitrophenyl | $SCF_3$ | H | i-$C_3F_7$ | H | Cl |
| 2-60 | Me | H | 2,6-dimethoxyphenyl | $SCF_3$ | H | i-$C_3F_7$ | H | Cl |
| 2-61 | Me | H | 3,5-dimethoxyphenyl | $SCF_3$ | H | i-$C_3F_7$ | H | Cl |
| 2-62 | Me | H | 3-methyl-4-nitrophenyl | $SCF_3$ | H | i-$C_3F_7$ | H | Cl |
| 2-63 | Me | H | 5-amino-2-fluorophenyl | $SCF_3$ | H | i-$C_3F_7$ | H | Cl |
| 2-64 | Me | H | 3-fluoro-2-methylphenyl | $SCF_3$ | H | i-$C_3F_7$ | H | Cl |
| 2-65 | Me | H | 2-fluoro-5-nitrophenyl | $SCF_3$ | H | i-$C_3F_7$ | H | Cl |
| 2-66 | Me | H | 4-fluoro-3-nitrophenyl | $SCF_3$ | H | i-$C_3F_7$ | H | Cl |
| 2-67 | Me | H | 5-fluoro-2-nitrophenyl | $SCF_3$ | H | i-$C_3F_7$ | H | Cl |
| 2-68 | Me | H | 2-fluoro-6-iodophenyl | $SCF_3$ | H | i-$C_3F_7$ | H | Cl |
| 2-69 | Me | H | 2-fluoro-5-trifluoromethylp henyl | $SCF_3$ | H | i-$C_3F_7$ | H | Cl |
| 2-70 | Me | H | 2-chloro-4-nitrophenyl | $SCF_3$ | H | i-$C_3F_7$ | H | Cl |
| 2-71 | Me | H | 2-chloro-4-fluorophenyl | $SCF_3$ | H | i-$C_3F_7$ | H | Cl |
| 2-72 | Me | H | 2-chloro-6-fluorophenyl | $SCF_3$ | H | i-$C_3F_7$ | H | Cl |
| 2-73 | Me | H | 3-chloro-4-fluorophenyl | $SCF_3$ | H | i-$C_3F_7$ | H | Cl |
| 2-74 | Me | H | 4-chloro-2-fluorophenyl | $SCF_3$ | H | i-$C_3F_7$ | H | Cl |
| 2-75 | Me | H | 4-chloro-2-nitrophenyl | $SCF_3$ | H | i-$C_3F_7$ | H | Cl |
| 2-76 | Me | H | 3-methoxy-4-nitrophenyl | $SCF_3$ | H | i-$C_3F_7$ | H | Cl |
| 2-77 | Me | H | 2-methoxy- 1 4-nitrophenyl | $SCF_3$ | H | i-$C_3F_7$ | H | Cl |
| 2-78 | Me | H | 2,3,4-trifluorophenyl | $SCF_3$ | H | i-$C_3F_7$ | H | Cl |

(continued)

| Compound No. | $R_1$ | $R_2$ | $Q_1$ | $Y_1$ | $Y_2$ | $Y_3$ | $Y_4$ | $Y_5$ |
|---|---|---|---|---|---|---|---|---|
| 2-79 | Me | H | 2,9,6-trimethylphenyl | $SCF_3$ | H | i-$C_3F_7$ | H | Cl |
| 2-80 | Me | H | 2,3,6-trifluorophenyl | $SCF_3$ | H | i-$C_3F_7$ | H | Cl |

Table 2-3

| Compound No. | $R_1$ | $R_2$ | $Q_1$ | $Y_1$ | $Y_2$ | $Y_3$ | $Y_4$ | $Y_5$ |
|---|---|---|---|---|---|---|---|---|
| 2-81 | Me | H | 2,4,5-trimethoxyphenyl | $SCF_3$ | H | i-$C_3F_7$ | H | Cl |
| 2-82 | Me | H | 3,4,5-trimethoxyphenyl | $SCF_3$ | H | i-$C_3F_7$ | H | Cl |
| 2-83 | Me | H | 2,3,4,5,6-pentafluoroph enyl | $SCF_3$ | H | i-$C_3F_7$ | H | Cl |
| 2-84 | Me | H | 2-biphenyl | $SCF_3$ | H | i-$C_3F_7$ | H | Cl |
| 2-85 | Me | H | 3-biphenyl | $SCF_3$ | H | i-$C_3F_7$ | H | Cl |
| 2-86 | Me | H | 1-naphthyl | $SCF_3$ | H | i-$C_3F_7$ | H | Cl |
| 2-87 | Me | H | 2-naphthyl | $SCF_3$ | H | i-$C_3F_7$ | H | Cl |
| 2-88 | Me | H | pyridin-2-yl | $SCF_3$ | H | i-$C_3F_7$ | H | Cl |
| 2-89 | Me | H | pyridin-3-yl | $SCF_3$ | H | i-$C_3F_7$ | H | Cl |
| 2-90 | Me | H | pyridin-4-yl | $SCF_3$ | H | i-$C_3F_7$ | H | Cl |
| 2-91 | Me | H | 2-methylpyridin-5-yl | $SCF_3$ | H | i-$C_3F_7$ | H | Cl |
| 2-92 | Me | H | 3-methylpyridin-2-yl | $SCF_3$ | H | i-$C_3F_7$ | H | Cl |
| 2-93 | Me | H | 2-fluoropyridin-3-yl | $SCF_3$ | H | i-$C_3F_7$ | H | Cl |
| 2-94 | Me | H | 2-chloropyridin-3-yl | $SCF_3$ | H | i-$C_3F_7$ | H | Cl |
| 2-95 | Me | H | 2-chloropyridin-4-yl | $SCF_3$ | H | i-$C_3F_7$ | H | Cl |
| 2-96 | Me | H | 2-chloropyridin-6-yl | $SCF_3$ | H | i-$C_3F_7$ | H | Cl |
| 2-97 | Me | H | 2-chloropyridin-5-yl | $SCF_3$ | H | i-$C_3F_7$ | H | Cl |
| 2-98 | Me | H | 5-chloropyridin-2-yl | $SCF_3$ | H | i-$C_3F_7$ | H | Cl |
| 2-99 | Me | H | 4-trifluoro methylpyridin-3-yl | $SCF_3$ | H | i-$C_3F_7$ | H | Cl |
| 2-100 | Me | H | 3-hydroxy pyridin-2-yl | $SCF_3$ | H | i-$C_3F_7$ | H | Cl |
| 2-101 | Me | H | 2-phenoxy pyridin-3-yl | $SCF_3$ | H | i-$C_3F_7$ | H | Cl |
| 2-102 | Me | H | 2-methylthio pyridin-3-yl | $SCF_3$ | H | i-$C_3F_7$ | H | Cl |
| 2-103 | Me | H | 2,6-dimethoxy pyridin-3-yl | $SCF_3$ | H | i-$C_3F_7$ | H | Cl |
| 2-104 | Me | H | 2,3-dichloro pyridin-5-yl | $SCF_3$ | H | i-$C_3F_7$ | H | Cl |
| 2-105 | Me | H | 2,5-dichloro pyridin-3-yl | $SCF_3$ | H | i-$C_3F_7$ | H | Cl |
| 2-106 | Me | H | 2,6-dichloro pyridin-3-yl | $SCF_3$ | H | i-$C_3F_7$ | H | Cl |
| 2-107 | Me | H | 3,5-dichloro pyridin-4-yl | $SCF_3$ | H | i-$C_3F_7$ | H | Cl |
| 2-108 | Me | H | pyridine-N-oxid-2-yl | $SCF_3$ | H | i-$C_3F_7$ | H | Cl |
| 2-109 | Me | H | N-methylpyrrol-2-yl | $SCF_3$ | H | i-$C_3F_7$ | H | Cl |
| 2-110 | Me | H | pyrazin-2-yl | $SCF_3$ | H | i-$C_3F_7$ | H | Cl |
| 2-111 | Me | H | 2-methylpyrazin-5-yl | $SCF_3$ | H | i-$C_3F_7$ | H | Cl |
| 2-112 | Me | H | 4-trifluoromethyl pyrimidin-5-yl | $SCF_3$ | H | i-$C_3F_7$ | H | Cl |
| 2-113 | Me | H | furan-2-yl | $SCF_3$ | H | i-$C_3F_7$ | H | Cl |

(continued)

| Compound No. | $R_1$ | $R_2$ | $Q_1$ | $Y_1$ | $Y_2$ | $Y_3$ | $Y_4$ | $Y_5$ |
|---|---|---|---|---|---|---|---|---|
| 2-114 | Me | H | furan-3-yl | $SCF_3$ | H | i-$C_3F_7$ | H | Cl |
| 2-115 | Me | H | thiophen-2-yl | $SCF_3$ | H | i-$C_3F_7$ | H | Cl |
| 2-116 | Me | H | thiophen-3-yl | $SCF_3$ | H | i-$C_3F_7$ | H | Cl |
| 2-117 | Me | H | 3-methylthiophen-2-yl | $SCF_3$ | H | i-$C_3F_7$ | H | Cl |
| 2-118 | Me | H | 2-nitro thiophen-4-yl | $SCF_3$ | H | i-$C_3F_7$ | H | Cl |
| 2-119 | Me | H | 2-methyl thiophen-5-yl | $SCF_3$ | H | i-$C_3F_7$ | H | Cl |
| 2-120 | Me | H | thiopheno-2-yl thiophen-2-yl | $SCF_3$ | H | i-$C_3F_7$ | H | Cl |

Table 2-4

| Compound No. | $R_1$ | $R_2$ | $Q_1$ | $Y_1$ | $Y_2$ | $Y_3$ | $Y_4$ | $Y_5$ |
|---|---|---|---|---|---|---|---|---|
| 2-121 | Me | H | 2-chlorothiophen-5-yl | $SCF_3$ | H | i-$C_3F_7$ | H | Cl |
| 2-122 | Me | H | 3-bromothiophen-2-yl | $SCF_3$ | H | i-$C_3F_7$ | H | Cl |
| 2-123 | Me | H | 2-bromothiophen-5-yl | $SCF_3$ | H | i-$C_3F_7$ | H | Cl |
| 2-124 | Me | H | 3-iodothiophen-2-yl | $SCF_3$ | H | i-$C_3F_7$ | H | Cl |
| 2-125 | Me | H | 3-phenylthiophen-2-yl | $SCF_3$ | H | i-$C_3F_7$ | H | Cl |
| 2-126 | Me | H | 2,4-dimethylthiophen-5-yl | $SCF_3$ | H | i-$C_3F_7$ | H | Cl |
| 2-127 | Me | H | 4-nitro-1H-pyrrol-2-yl | $SCF_3$ | H | i-$C_3F_7$ | H | Cl |
| 2-128 | Me | H | 3-ethyl-3H-pyrazol-4-yl | $SCF_3$ | H | i-$C_3F_7$ | H | Cl |
| 2-129 | Me | H | 1-methyl-3-nitro-1H-pyrazol-4-yl | $SCF_3$ | H | i-$C_3F_7$ | H | Cl |
| 2-130 | Me | H | 3-chloro-1-methyl-1H-pyrazol-4-yl | $SCF_3$ | H | i-$C_3F_7$ | H | Cl |
| 2-131 | Me | H | 3-bromo-1-methyl-1H-pyrazol -4-yl | $SCF_3$ | H | i-$C_3F_7$ | H | Cl |
| 2-132 | Me | H | 1-methyl-3-trifluoromethyl-1H-pyrazol-4-yl | $SCF_3$ | H | i-$C_3F_7$ | H | Cl |
| 2-133 | Me | H | 1-methyl-5-trifluoromethyl-1H-pyrazol-4-yl | $SCF_3$ | H | i-$C_3F_7$ | H | Cl |
| 2-134 | Me | H | isoxazol-5-yl | $SCF_3$ | H | i-$C_3F_7$ | H | Cl |
| 2-135 | Me | H | 4-trifluoromethylthiazol-5-yl | $SCF_3$ | H | i-$C_3F_7$ | H | Cl |
| 2-136 | Me | H | 2,4-dimethylthiazol-5-yl | $SCF_3$ | H | i-$C_3F_7$ | H | Cl |
| 2-137 | Me | H | 2-ethyl-4-methylthiazol-5-yl | $SCF_3$ | H | i-$C_3F_7$ | H | Cl |
| 2-138 | Me | H | 2-chloro-4-methylthiazol-5-yl | $SCF_3$ | H | i-$C_3F_7$ | H | Cl |
| 2-139 | Me | H | 3-methylisothiazol-5-yl | $SCF_3$ | H | i-$C_3F_7$ | H | Cl |
| 2-140 | Me | H | 3,4-dichloro-isothiazol-5-yl | $SCF_3$ | H | i-$C_3F_7$ | H | Cl |
| 2-141 | Me | H | phenyl | $SCF_3$ | H | i-$C_3F_7$ | H | Br |
| 2-142 | Me | H | 4-methylphenyl | $SCF_3$ | H | i-$C_3F_7$ | H | Br |
| 2-143 | Me | H | 2-fluorophenyl | $SCF_3$ | H | -$C_3F_7$ | H | Br |
| 2-144 | Me | H | 3-fluorophenyl | $SCF_3$ | H | i-$C_3F_7$ | H | Br |
| 2-145 | Me | H | 4-fluorophenyl | $SCF_3$ | H | i-$C_3F_7$ | H | Br |
| 2-146 | Me | H | 4-chlorophenyl | $SCF_3$ | H | i-$C_3F_7$ | H | Br |
| 2-147 | Me | H | 4-cyanophenyl | $SCF_3$ | H | i-$C_3F_7$ | H | Br |
| 2-148 | Me | H | 4-nitrophenyl | $SCF_3$ | H | i-$C_3F_7$ | H | Br |

(continued)

| Compound No. | $R_1$ | $R_2$ | $Q_1$ | $Y_1$ | $Y_2$ | $Y_3$ | $Y_4$ | $Y_5$ |
|---|---|---|---|---|---|---|---|---|
| 2-149 | Me | H | 2-(trifluoromethyl)phenyl | $SCF_3$ | H | i-$C_3F_7$ | H | Br |
| 2-150 | Me | H | 4-(trifluoromethyl)phenyl | $SCF_3$ | H | i-$C_3F_7$ | H | Br |
| 2-151 | Me | H | 2,6-difluorophenyl | $SCF_3$ | H | i-$C_3F_7$ | H | Br |
| 2-152 | Me | H | 2,4-difluorophenyl | $SCF_3$ | H | i-$C_3F_7$ | H | Br |
| 2-153 | Me | H | 3-chloro-4-fluorophenyl | $SCF_3$ | H | i-$C_3F_7$ | H | Br |
| 2-154 | Me | H | pyridin-2-yl | $SCF_3$ | H | i-$C_3F_7$ | H | Br |
| 2-155 | Me | H | pyridin-3-yl | $SCF_3$ | H | i-$C_3F_7$ | H | Br |
| 2-156 | Me | H | pyridin-4-yl | $SCF_3$ | H | i-$C_3F_7$ | H | Br |
| 2-157 | Me | H | 2-fluoropyridin-3-yl | $SCF_3$ | H | i-$C_3F_7$ | H | Br |
| 2-158 | Me | H | 2-chloropyridin-3-yl | $SCF_3$ | H | i-$C_3F_7$ | H | Br |
| 2-159 | Me | H | 2-chloropyridin-5-yl | $SCF_3$ | H | i-$C_3F_7$ | H | Br |
| 2-160 | Me | H | thiophen-2-yl | $SCF_3$ | H | i-$C_3F_7$ | H | Br |

Table 2-5

| Compound No. | $R_1$ | $R_2$ | $Q_1$ | $Y_1$ | $Y_2$ | $Y_3$ | $Y_4$ | $Y_5$ |
|---|---|---|---|---|---|---|---|---|
| 2-161 | Me | H | phenyl | $SCF_3$ | H | i-$C_3F_7$ | H | I |
| 2-162 | Me | H | 2-fluorophenyl | $SCF_3$ | H | i-$C_3F_7$ | H | I |
| 2-163 | Me | H | 4-cyanophenyl | $SCF_3$ | H | i-$C_3F_7$ | H | I |
| 2-164 | Me | H | 2-fluoro pyridin-3-yl | $SCF_3$ | H | i-$C_3F_7$ | H | I |
| 2-165 | Me | H | 2-chloro pyridin-3-yl | $SCF_3$ | H | i-$C_3F_7$ | H | I |
| 2-166 | Me | H | phenyl | $SCF_3$ | H | i-$C_3F_7$ | H | Me |
| 2-167 | Me | H | 2-fluorophenyl | $SCF_3$ | H | i-$C_3F_7$ | H | Me |
| 2-168 | Me | H | 4-cyanophenyl | $SCF_3$ | H | i-$C_3F_7$ | H | Me |
| 2-169 | Me | H | 2-fluoro pyridin-3-yl | $SCF_3$ | H | i-$C_3F_7$ | H | Me |
| 2-170 | Me | H | 2-chloro pyridin-3-yl | $SCF_3$ | H | i-$C_3F_7$ | H | Me |
| 2-171 | Me | H | phenyl | $S(O)CF_3$ | H | i-$C_3F_7$ | H | Cl |
| 2-172 | Me | H | 4-fluorophenyl | $S(O)CF_3$ | H | i-$C_3F_7$ | H | Br |
| 2-173 | Me | H | 2,4-difluorophenyl | $S(O)CF_3$ | H | i-$C_3F_7$ | H | Br |
| 2-174 | Me | H | 2,6-difluorophenyl | $S(O)CF_3$ | H | i-$C_3F_7$ | H | Br |
| 2-175 | Me | H | pyridin-4-yl | $S(O)CF_3$ | H | i-$C_3$-$F_7$ | H | Br |
| 2-176 | Me | H | phenyl | $S(O)CF_3$ | H | i-$C_3F_7$ | H | Br |
| 2-177 | Me | H | 2-fluorophenyl | $S(O)CF_3$ | H | i-$C_3F_7$ | H | Br |
| 2-178 | Me | H | 4-cyanophenyl | $S(O)CF_3$ | H | i-$C_3F_7$ | H | Br |
| 2-179 | Me | H | pyridin-3-yl | $S(O)CF_3$ | H | i-$C_3F_7$ | H | Br |
| 2-180 | Me | H | 2-chloro pyridin-3-yl | $S(O)CF_3$ | H | i-$C_3F_7$ | H | Br |
| 2-181 | Me | H | pyridin-3-yl | $S(O)CF_3$ | H | i-$C_3F_7$ | H | Br |
| 2-182 | Me | H | phenyl | $S(O)CF_3$ | H | i-$C_3F_7$ | H | Me |
| 2-183 | Me | H | 4-cyanophenyl | $S(O)CF_3$ | H | i-$C_3F_7$ | H | Me |

(continued)

| Compound No. | $R_1$ | $R_2$ | $Q_1$ | $Y_1$ | $Y_2$ | $Y_3$ | $Y_4$ | $Y_5$ |
|---|---|---|---|---|---|---|---|---|
| 2-184 | Me | H | 2-fluoropyridin-3-yl | $S(O)CF_3$ | H | $i-C_3F_7$ | H | Me |
| 2-185 | Me | H | 2-chloropyridin-3-yl | $S(O)CF_3$ | H | $i-C_3F_7$ | H | Me |
| 2-186 | Me | H | phenyl | $SO_2CF_3$ | H | $i-C_3F_7$ | H | Cl |
| 2-187 | Me | H | 2-fluorophenyl | $SO_2CF_3$ | H | $i-C_3F_7$ | H | Cl |
| 2-188 | Me | H | 4-cyanophenyl | $SO_2CF_3$ | H | $i-C_3F_7$ | H | Cl |
| 2-189 | Me | H | 2-fluoropyridin-3-yl | $SO_2CF_3$ | H | $i-C_3F_7$ | H | Cl |
| 2-190 | Me | H | 2-chloropyridin-3-yl | $SO_2CF_3$ | H | $i-C_3F_7$ | H | Cl |
| 2-191 | Me | H | phenyl | $SO_2CF_3$ | H | $i-C_3F_7$ | H | Br |
| 2-192 | Me | H | 2-fluorophenyl | $SO_2CF_3$ | H | $i-C_3F_7$ | H | Br |
| 2-193 | Me | H | 4-cyanophenyl | $SO_2CF_3$ | H | $i-C_3F_7$ | H | Br |
| 2-194 | Me | H | 2-fluoropyridin-3-yl | $SO_2CF_3$ | H | $i-C_3F_7$ | H | Br |
| 2-195 | Me | H | 2-chloropyridin-3-yl | $SO_2CF_3$ | H | $i-C_3F_7$ | H | Br |
| 2-196 | Me | H | phenyl | $SO_2CF_3$ | H | $i-C_3F_7$ | H | Me |
| 2-197 | Me | H | 2-fluorophenyl | $SO_2CF_3$ | H | $i-C_3F_7$ | H | Me |
| 2-198 | Me | H | 4-cyanophenyl | $SO_2CF_3$ | H | $i-C_3F_7$ | H | Me |
| 2-199 | Me | H | 2-fluoropyridin-3-yl | $SO_2CF_3$ | H | $i-C_3F_7$ | H | Me |
| 2-200 | Me | H | 2-chloropyridin-3-yl | $SO_2CF_3$ | H | $i-C_3F_7$ | H | Me |

Table 2-6

| Compound No. | $R_1$ | $R_2$ | $Q_1$ | $Y_1$ | $Y_2$ | $Y_3$ | $Y_4$ | $Y_5$ |
|---|---|---|---|---|---|---|---|---|
| 2-201 | Me | H | phenyl | $SC_2F_5$ | H | $i-C_3F_7$ | H | Br |
| 2-202 | Me | H | 2-fluorophenyl | $SC_2F_5$ | H | $i-C_3F_7$ | H | Br |
| 2-203 | Me | H | 2-chloropyridin-3-yl | $SC_2F_5$ | H | $i-C_3F_7$ | H | Br |
| 2-204 | Me | H | phenyl | $S-n-C_3F_7$ | H | $i-C_3F_7$ | H | Br |
| 2-205 | Me | H | 2-fluorophenyl | $S-n-C_3F_7$ | H | $i-C_3F_7$ | H | Br |
| 2-206 | Me | H | 2-chloropyridin-3-yl | $S-n-C_3F_7$ | H | $i-C_3F_7$ | H | Br |
| 2-207 | Me | H | phenyl | $SCF_3$ | H | $n-C_3F_7$ | H | Br |
| 2-208 | Me | H | 2-fluorophenyl | $SCF_3$ | H | $n-C_3F_7$ | H | Br |
| 2-209 | Me | H | 2-chloropyridin-3-yl | $SCF_3$ | H | $n-C_3F_7$ | H | Br |
| 2-210 | Me | H | phenyl | $SCF_3$ | H | $C_2F_5$ | H | Cl |
| 2-211 | Me | H | phenyl | $S(O)CF_3$ | H | $C_2F_5$ | H | Cl |
| 2-212 | Me | H | phenyl | $SO_2CF_3$ | H | $C_2F_5$ | H | Cl |
| 2-213 | Me | H | phenyl | $SCF_3$ | H | $C_2F_5$ | H | Br |
| 2-214 | Me | H | phenyl | $S(O)CF_3$ | H | $C_2F_5$ | H | Br |
| 2-215 | Me | H | phenyl | $SO_2CF_3$ | H | $C_2F_5$ | H | Br |
| 2-216 | Me | H | phenyl | $SCF_3$ | H | $C_2F_5$ | H | Me |
| 2-217 | Me | H | phenyl | $S(O)CF_3$ | H | $C_2F_5$ | H | Me |
| 2-218 | Me | H | phenyl | $SO_2CF_3$ | H | $C_2F_5$ | H | Me |

(continued)

| Compound No. | $R_1$ | $R_2$ | $Q_1$ | $Y_1$ | $Y_2$ | $Y_3$ | $Y_4$ | $Y_5$ |
|---|---|---|---|---|---|---|---|---|
| 2-219 | Me | H | phenyl | $SCF_3$ | H | $2\text{-}C_4F_9$ | H | Cl |
| 2-220 | Me | H | phenyl | $S(O)CF_3$ | H | $2\text{-}C_4F_9$ | H | Cl |
| 2-221 | Me | H | phenyl | $SO_2CF_3$ | H | $2\text{-}C_4F_9$ | H | Cl |
| 2-222 | Me | H | phenyl | $SCF_3$ | H | $2\text{-}C_4F_9$ | H | Br |
| 2-223 | Me | H | phenyl | $S(O)CF_3$ | H | $2\text{-}C_4F_9$ | H | Br |
| 2-224 | Me | H | phenyl | $SO_2CF_3$ | H | $2\text{-}C_4F_9$ | H | Br |
| 2-225 | Me | H | phenyl | $SCF_3$ | H | $2\text{-}C_4F_9$ | H | Me |
| 2-226 | Me | H | phenyl | $S(O)CF_3$ | H | $2\text{-}C_4F_9$ | H | Me |
| 2-227 | Me | H | phenyl | $SO_2CF_3$ | H | $2\text{-}C_4F_9$ | H | Me |
| 2-228 | Me | H | 2-chloro pyridin-3-yl | $SCF_3$ | H | $2\text{-}C_4F_9$ | H | Cl |
| 2-229 | Me | H | 2-chloro pyridin-3-yl | $S(O)CF_3$ | H | $2\text{-}C_4F_9$ | H | Cl |
| 2-230 | Me | H | 2-chloro pyridin-3-yl | $SO_2CF_3$ | H | $2\text{-}C_4F_9$ | H | Cl |
| 2-231 | Me | H | 2-chloro pyridin-3-yl | $SCF_3$ | H | $2\text{-}C_4F_9$ | H | Br |
| 2-232 | Me | H | 2-chloro pyridin-3-yl | $S(O)CF_3$ | H | $2\text{-}C_4F_9$ | H | Br |
| 2-233 | Me | H | 2-chloro pyridin-3-yl | $SO_2CF_3$ | H | $2\text{-}C_4F_9$ | H | Br |
| 2-234 | Me | H | 2-chloro pyridin-3-yl | $SCF_3$ | H | $2\text{-}C_4F_9$ | H | Me |
| 2-235 | Me | H | 2-chloro pyridin-3-yl | $SCF_3$ | M e | $i\text{-}C_3F_7$ | H | Br |
| 2-236 | Me | H | 2-chloro pyndin-3-yl | $SCF_3$ | H | $i\text{-}C_3F_7$ | Me | Br |
| 2-237 | Me | H | 2-chloro pyridin-3-yl | $SCF_3$ | C 1 | $i\text{-}C_3F_7$, | H | Br |
| 2-238 | Me | H | 2-chloro pyridin-3-yl | $SCF_3$ | M e | $i\text{-}C_3F_7$ | H | Br |
| 2-239 | Me | H | 2-chloro pyridin-3-yl | $SCF_3$ | H | $i\text{-}C_3F_7$ | Me | Br |
| 2-240 | Me | H | 2-chloro pyridin-3-yl | $SCF_3$ | B r | $i\text{-}C_3F_7$ | H | Br |

Table 2-7

| Compound No. | $R_1$ | $R_2$ | $Q_1$ | $Y_1$ | $Y_2$ | $Y_3$ | $Y_4$ | $Y_5$ |
|---|---|---|---|---|---|---|---|---|
| 2-241 | Me | H | phenyl | $OCF_3$ | H | $i\text{-}C_3F_7$ | H | Cl |
| 2-242 | Me | H | 2-fluorophenyl | $OCF_3$ | H | $i\text{-}C_3F_7$ | H | Cl |
| 2-243 | Me | H | phenyl | $OCF_3$ | H | $i\text{-}C_3F_7$ | H | Br |
| 2-244 | Me | H | 2-fluorophenyl | $OCF_3$ | H | $i\text{-}C_3F_7$ | H | Br |
| 2-245 | Me | H | 4-nitrophenyl | $OCF_3$ | H | $i\text{-}C_3F_7$ | H | Br |
| 2-246 | Me | H | 4-cyanophenyl | $OCF_3$ | H | $i\text{-}C_3F_7$ | H | Br |
| 2-247 | Me | H | 4-fluorophenyl | $OCF_3$ | H | $i\text{-}C_3F_7$ | H | Br |
| 2-248 | Me | H | 4-(trifluoro methyl)phenyl | $OCF_3$ | H | $i\text{-}C_3F_7$ | H | Br |
| 2-249 | Me | H | 4-chlorophenyl | $OCF_3$ | H | $i\text{-}C_3F_7$ | H | Br |
| 2-250 | Me | H | phenyl | $OCF_3$ | H | $2\text{-}C_4F_9$ | H | Br |
| 2-251 | Me | H | 2-fluorophenyl | $OCF_3$ | H | $2\text{-}C_4F_9$ | H | Br |
| 2-252 | Me | H | 2-chloro pyridin-3-yl | $OCF_3$ | H | $2\text{-}C_4F_9$ | H | Br |
| 2-253 | Me | H | phenyl | OMe | H | $i\text{-}C_3F_7$ | H | Br |

(continued)

| Compound No. | $R_1$ | $R_2$ | $Q_1$ | $Y_1$ | $Y_2$ | $Y_3$ | $Y_4$ | $Y_5$ |
|---|---|---|---|---|---|---|---|---|
| 2-254 | Me | H | 2-fluorophenyl | OMe | H | $i-C_3F_7$ | H | Br |
| 2-255 | Me | H | 4-fluorophenyl | OMe | H | $i-C_3F_7$ | H | Br |
| 2-256 | Me | H | 2-chloro pyridin-3-yl | OMe | H | $i-C_3F_7$ | H | Br |
| 2-257 | Me | H | phenyl | OMe | H | $i-C_3F_7$ | H | I |
| 2-258 | Me | H | 2-fluorophenyl | OMe | H | $i-C_3F_7$ | H | I |
| 2-259 | Me | H | 2-chloro pyridin-3-yl | OMe | H | $i-C_3F_7$ | H | I |
| 2-260 | Me | H | phenyl | OMe | H | $i-C_3F_7$ | H | Me |
| 2-261 | Me | H | 2-chloro pyridin-3-yl | OMe | H | $i-C_3F_7$ | H | Me |
| 2-262 | Me | H | 2-fluorophenyl | OMe | H | $i-C_3F_7$ | H | Me |
| 2-263 | Me | H | phenyl | OMe | H | $i-C_3F_7$ | H | Cl |
| 2-264 | Me | H | 2-fluorophenyl | OMe | H | $i-C_3F_7$ | H | Cl |
| 2-265 | Me | H | 2-chloro pyridin-3-yl | OMe | H | $i-C_3F_7$ | H | Cl |
| 2-266 | Me | H | 4-cyanophenyl | OMe | H | $i-C_3F_7$ | H | Br |
| 2-267 | Me | H | pyridin-2-yl | OMe | H | $i-C_3F_7$ | H | Br |
| 2-268 | Me | H | pyridin-3-yl | OMe | H | $i-C_3F_7$ | H | Br |
| 2-269 | Me | H | phenyl | $OCH_2CF_3$ | H | $i-C_3F_7$ | H | Br |
| 2-270 | Me | H | 2-chloro pyridin-3-yl | $OCH_2CF_3$ | H | $i-C_3F_7$ | H | Br |
| 2-271 | Me | H | phenyl | OMe | H | $C_2F_5$ | H | Br |
| 2-272 | Me | H | 2-chloro pyridin-3-yl | OMe | H | $C_2F_5$ | H | Br |
| 2-273 | Me | H | phenyl | OEt | H | $i-C_3F_7$ | H | Br |
| 2-274 | Me | H | 2-chloro pyridin-3-yl | OEt | H | $i-C_3F_7$ | H | Br |
| 2-275 | Me | H | phenyl | OMe | Me | $i-C_3F_7$ | H | Br |
| 2-276 | Me | H | phenyl | OMe | H | $i-C_3F_7$ | Me | Br |
| 2-277 | Me | H | phenyl | OMe | Cl | $i-C_3F_7$ | H | Br |
| 2-278 | Me | H | phenyl | OMe | Me | $i-C_3F_7$ | H | Br |
| 2-279 | Me | H | phenyl | OMe | H | $i-C_3F_7$ | Me | Br |
| 2-280 | Me | H | phenyl | OMe | Br | $i-C_3F_7$ | H | Br |
| 2-281 | Me | H | pyrimidin-5-yl | $S(O)CF_3$ | H | $i-C_3F_7$ | H | Br |

General Formula (A)

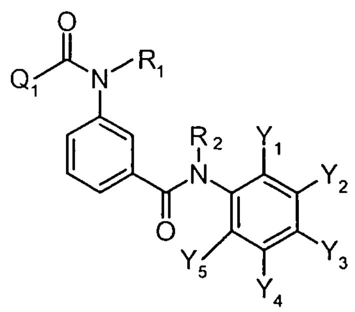

(A)

Table 3-1

| Compound No. | $R_1$ | $R_2$ | $Q_1$ | $Y_1$ | $Y_2$ | $Y_3$ | $Y_4$ | $Y_5$ |
|---|---|---|---|---|---|---|---|---|
| 3-1 | Me | Me | phenyl | $SCF_3$ | H | i-$C_3F_7$ | H | Cl |
| 3-2 | Me | Me | 2-fluorophenyl | $SCF_3$ | H | i-$C_3F_7$ | H | Cl |
| 3-3 | Me | Me | 2-chloropyridin-3-yl | $SCF_3$ | H | i-$C_3F_7$ | H | Cl |
| 3-4 | Me | Me | phenyl | $SCF_3$ | H | i-$C_3F_7$ | H | Br |
| 3-5 | Me | Me | 2-fluorophenyl | $SCF_3$ | H | i-$C_3F_7$ | H | Br |
| 3-6 | Me | Me | 2-chloropyridin-3-yl | $SCF_3$ | H | i-$C_3F_7$ | H | Br |
| 3-7 | Me | Me | phenyl | $S(O)CF_3$ | H | i-$C_3F_7$ | H | Br |
| 3-8 | Me | Me | 2-fluorophenyl | $S(O)CF_3$ | H | i-$C_3F_7$ | H | Br |
| 3-9 | Me | Me | 2-chloropyridin-3-yl | $S(O)CF_3$ | H | i-$C_3F_7$ | H | Br |
| 3-10 | Me | Me | phenyl | $SO_2CF_3$ | H | i-$C_3F_7$ | H | Br |
| 3-11 | Me | Me | 2-fluorophenyl | $SO_2CF_3$ | H | i-$C_3F_7$ | H | Br |
| 3-12 | Me | Me | 2-chloropyridin-3-yl | $SO_2CF_3$ | H | i-$C_3F_7$ | H | Br |
| 3-13 | H | Me | phenyl | $SCF_3$ | H | i-$C_3F_7$ | H | Cl |
| 3-14 | H | Me | 2-fluorophenyl | $SCF_3$ | H | i-$C_3F_7$ | H | Cl |
| 3-15 | H | Me | 2-chloropyridin-3-yl | $SCF_3$ | H | i-$C_3F_7$ | H | Cl |
| 3-16 | H | Me | phenyl | $SCF_3$ | H | i-$C_3F_7$ | H | Br |
| 3-17 | H | Me | 2-fluorophenyl | $SCF_3$ | H | i -$C_3F_7$ | H | Br |
| 3-18 | H | Me | 2-chloropyridin-3-yl | $SCF_3$ | H | i-$C_3F_7$ | H | Br |
| 3-19 | H | Me | phenyl | $S(O)CF_3$ | H | i-$C_3F_7$ | H | Br |
| 3-20 | H | Me | 2-fluorophenyl | $S(O)CF_3$ | H | i-$C_3F_7$ | H | Br |
| 3-21 | H | Me | 2-chloropyridin-3-yl | $S(O)CF_3$ | H | i-$C_3F_7$ | H | Br |
| 3-22 | H | Me | phenyl | $SO_2CF_3$ | H | i-$C_3F_7$ | H | Br |
| 3-23 | H | Me | 2-fluorophenyl | $SO_2CF_3$ | H | i-$C_3F_7$ | H | Br |
| 3-24 | H | Me | 2-chloropyridin-3-yl | $SO_2CF_3$ | H | i-$C_3F_7$ | H | Br |
| 3-25 | H | Me | phenyl | $SCF_3$ | H | 2-$C_4F_9$ | H | Br |
| 3-26 | H | Me | phenyl | S (O) $CF_3$ | H | 2-$C_4F_9$ | H | Br |
| 3-27 | H | Me | phenyl | $SO_2CF_3$ | H | 2-$C_4F_9$ | H | Br |
| 3-28 | H | Me | 2-chloropyridin-3-yl | $SCF_3$ | H | 2-$C_4F_9$ | H | Br |
| 3-29 | H | Me | 2-chloropyridin-3-yl | $S(O)CF_3$ | H | 2-$C_4F_9$ | H | Br |
| 3-30 | H | Me | 2-chloropyridin-3-yl | $SO_2CF_3$ | H | 2-$C_4F_9$ | H | Br |

Table 3-2

| Compound No. | $R_1$ | $R_2$ | $Q_1$ | $Y_1$ | $Y_2$ | $Y_3$ | $Y_4$ | $Y_5$ |
|---|---|---|---|---|---|---|---|---|
| 3-31 | Me | Me | phenyl | $SCF_3$ | H | 2-$C_4F_9$ | H | Br |
| 3-32 | Me | Me | phenyl | S(O) $CF_3$ | H | 2-$C_4F_9$ | H | Br |
| 3-33 | Me | Me | phenyl | $SO_2CF_3$ | H | 2-$C_4F_9$ | H | Br |
| 3-34 | Me | Me | 2-chloro pyridin-3-yl | $SCF_3$ | H | 2-$C_4F_9$ | H | Br |

(continued)

| Compound No. | $R_1$ | $R_2$ | $Q_1$ | $Y_1$ | $Y_2$ | $Y_3$ | $Y_4$ | $Y_5$ |
|---|---|---|---|---|---|---|---|---|
| 3-35 | Me | Me | 2-chloro pyridin-3-yl | S(O) $CF_3$ | H | 2-$C_4F_9$ | H | Br |
| 3-36 | Me | Me | 2-chloro pyridin-3-yl | $SO_2CF_3$ | H | 2-$C_4F_9$ | H | Br |
| 3-37 | Et | H | phenyl | $SCF_3$ | H | i-$C_3F_7$ | H | Br |
| 3-38 | H | Et | phenyl | $SCF_3$ | H | i-$C_3F_7$ | H | Br |
| 3-39 | Et | Et | phenyl | $SCF_3$ | H | i-$C_3F_7$ | H | Br |
| 3-40 | Ac | H | phenyl | $SCF_3$ | H | i-$C_3F_7$ | H | Br |
| 2-41 | H | Ac | phenyl | $SCF_3$ | H | i-$C_3F_7$ | H | Br |
| 3-42 | Ac | Ac | phenyl | $SCF_3$ | H | i-$C_3F_7$ | H | Br |
| 3-43 | H | Me | phenyl | $OCF_3$ | H | i-$C_3F_7$ | H | Br |
| 3-44 | H | Me | 2-chloro pyridin-3-yl | $OCF_3$ | H | i-$C_3F_7$ | H | Br |
| 3-45 | Me | Me | phenyl | $OCF_3$ | H | i-$C_3F_7$ | H | Br |
| 3-46 | Me | Me | 2-chloro pyridin-3-yl | $OCF_3$ | H | i-$C_3F_7$ | H | Br |
| 3-47 | H | Me | phenyl | OMe | H | i-$C_3F_7$ | H | Br |
| 3-48 | H | Me | 2-chloro pyridin-3-yl | OMe | H | i-$C_3F_7$ | H | Br |
| 3-49 | Me | Me | phenyl | OMe | H | i-$C_3F_7$ | H | Br |
| 3-50 | Me | Me | 2-chloro pyridin-3-yl | OMe | H | i-$C_3F_7$ | H | Br |
| 3-51 | Me | Me | phenyl | OMe | H | i-$C_3E_7$ | H | Cl |
| 3-52 | Me | Me | 2-chloro pyridin-3-yl | OMe | H | i-$C_3F_7$ | H | Cl |

General Formula (B)

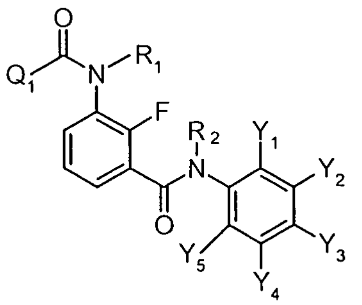

(B)

Table 4-1

| Compound No. | $R_1$ | $R_2$ | $Q_1$ | $Y_1$ | $Y_2$ | $Y_3$ | $Y_4$ | $Y_5$ |
|---|---|---|---|---|---|---|---|---|
| 4-1 | H | H | phenyl | $SCF_3$ | H | i-$C_3F_7$ | H | Cl |
| 4-2 | H | H | 2-fluorophenyl | $SCF_3$ | H | i-$C_3F_7$ | H | Cl |
| 4-3 | H | H | 2-chloro pyridin-3-yl | $SCF_3$ | H | i-$C_3F_7$ | H | Cl |
| 4-4 | H | H | phenyl | $SCF_3$ | H | i-$C_3F_7$ | H | Br |
| 4-5 | H | H | 2-fluorophenyl | $SCF_3$ | H | i-$C_3F_7$ | H | Br |
| 4-6 | H | H | 2-chloro pyridin-3-yl | $SCF_3$ | H | i-$C_3F_7$ | H | Br |
| 4-7 | H | H | phenyl | $S(O)CF_3$ | H | i-$C_3F_7$ | H | Br |

(continued)

| Compound No. | $R_1$ | $R_2$ | $Q_1$ | $Y_1$ | $Y_2$ | $Y_3$ | $Y_4$ | $Y_5$ |
|---|---|---|---|---|---|---|---|---|
| 4-8 | H | H | phenyl | $SO_2CF_3$ | H | i-$C_3F_7$ | H | Br |
| 4-9 | H | H | phenyl | $SCF_3$ | H | 2-$C_4F_9$ | H | Br |
| 4-10 | H | H | phenyl | $S(O)CF_3$ | H | 2-$C_4F_9$ | H | Br |
| 4-11 | H | H | phenyl | $SO_2CF_3$ | H | 2-$C_4F_9$ | H | Br |
| 4-12 | Me | H | phenyl | $SCF_3$ | H | i-$C_3F_7$ | H | Cl |
| 4-13 | Me | H | 2-fluorophenyl | $SCF_3$ | H | i-$C_3F_7$ | H | Cl |
| 4-14 | Me | H | 2-chloro pyridin-3-yl | $SCF_3$ | H | i-$C_3F_7$ | H | Cl |
| 4-15 | Me | H | phenyl | $SCF_3$ | H | i-$C_3F_7$ | H | Br |
| 4-16 | Me | H | 2-fluorophenyl | $SCF_3$ | H | i-$C_3F_7$ | H | Br |
| 4-17 | Me | H | 2-chloro pyridin-3-yl | $SCF_3$ | H | i-$C_3F_7$ | H | Br |
| 4-18 | Me | H | phenyl | $S(O)CF_3$ | H | i-$C_3F_7$ | H | Br |
| 4-19 | Me | H | phenyl | $SO_2CF_3$ | H | i-$C_3F_7$ | H | Br |
| 4-20 | Me | H | phenyl | $SCF_3$ | H | 2-$C_4F_9$ | H | Br |
| 4-21 | Me | H | phenyl | $S(O)CF_3$ | H | 2-$C_4F_9$ | H | Br |
| 4-22 | Me | H | phenyl | $SO_2CF_3$ | H | 2-$C_4F_9$ | H | Br |
| 4-23 | Me | Me | phenyl | $SCF_3$ | H | i-$C_3F_7$ | H | Cl |
| 4-24 | Me | Me | 2-fluorophenyl | $SCF_3$ | H | i-$C_3F_7$ | H | Cl |
| 4-25 | Me | Me | 2-chloro pyridin-3-yl | $SCF_3$ | H | i-$C_3F_7$ | H | Cl |
| 4-26 | Me | Me | phenyl | $SCF_3$ | H | i-$C_3F_7$ | H | Me |
| 4-27 | Me | Me | 2-fluorophenyl | $SCF_3$ | H | i-$C_3F_7$ | H | Me |
| 4-28 | Me | Me | 2-chloro pyridin-3-yl | $SCF_3$ | H | i-$C_3F_7$ | H | Br |
| 4-29 | Me | Me | phenyl | $S(O)CF_3$ | H | i-$C_3F_7$ | H | Br |
| 4-30 | Me | Me | phenyl | $SO_2CF_3$ | H | i-$C_3F_7$ | H | Br |
| 4-31 | Me | Me | phenyl | $SCF_3$ | H | 2-$C_4F_9$ | H | Br |
| 4-32 | Me | Me | phenyl | $S(O)CF_3$ | H | 2-$C_4F_9$ | H | Br |
| 4-33 | Me | Me | phenyl | $SO_2CF_3$ | H | 2-$C_4F_9$ | H | Br |
| 4-34 | H | Me | phenyl | $OCF_3$ | H | i-$C_3F_7$ | H | Br |
| 4-35 | H | Me | 2-chloro pyridin-3-yl | $OCF_3$ | H | i-$C_3F_7$ | H | Br |
| 4-36 | Me | H | phenyl | $OCF_3$ | H | i-$C_3F_7$ | H | Br |
| 4-37 | Me | H | 2-chloro pyridin-3-yl | $OCF_3$ | H | i-$C_3F_7$ | H | Br |
| 4-38 | H | Me | phenyl | OMe | H | i-$C_3F_7$ | H | Br |
| 4-39 | H | Me | 2-chloro pyridin-3-yl | OMe | H | i-$C_3F_7$ | H | Br |
| 4-40 | Me | H | phenyl | OMe | H | i-$C_3F_7$ | H | Br |
| 4-41 | Me | H | 2-chloro pyridin-3-yl | OMe | H | i-$C_3F_7$ | H | Br |
| 4-42 | Me | Me | phenyl | OMe | H | i-$C_3F_7$ | H | Cl |
| 4-93 | Me | Me | 2-chloro pyridin-3-yl | OMe | H | i-$C_3F_7$ | H | Cl |

General Formula (C)

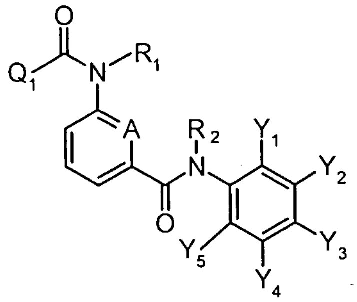

(C)

Table 5-1

| Compound No. | $R_1$ | $R_2$ | A | $Q_1$ | $Y_1$ | $Y_2$ | $Y_3$ | $Y_4$ | $Y_5$ |
|---|---|---|---|---|---|---|---|---|---|
| 5-1 | H | H | N | phenyl | $SCF_3$ | H | i-$C_3F_7$ | H | Cl |
| 5-2 | H | H | N | 2-fluorophenyl | $SCF_3$ | H | i-$C_3F_7$ | H | Cl |
| 5-3 | H | H | N | 2-chloro pyridin-3-yl | $SCF_3$ | H | i-$C_3F_7$ | H | Cl |
| 5-4 | H | H | N | phenyl | $SCF_3$ | H | i-$C_3F_7$ | H | Br |
| 5-5 | H | H | N | 2-fluorophenyl | $SCF_3$ | H | i-$C_3F_7$ | H | Br |
| 5-6 | H | H | N | 2-chloro pyridin-3-yl | $SCF_3$ | H | i-$C_3F_7$ | H | Br |
| 5-7 | H | H | N | phenyl | $S(O)CF_3$ | H | i-$C_3F_7$ | H | Br |
| 5-8 | H | H | N | phenyl | $SO_2CF_3$ | H | i-$C_3F_7$ | H | Br |
| 5-9 | H | H | N | phenyl | $SCF_3$ | H | 2-$C_4F_9$ | H | Br |
| 5-10 | H | H | N | phenyl | $S(O)CF_3$ | H | 2-$C_4F_9$ | H | Br |
| 5-11 | H | H | N-oxide | phenyl | $SO_2CF_3$ | H | 2-$C_4F_9$ | H | Br |
| 5-12 | H | H | N-oxide | phenyl | $SCF_3$ | H | i-$C_3F_7$ | H | Br |
| 5-13 | H | H | N-oxide | 2-fluorophenyl | $SCF_3$ | H | i-$C_3F_7$ | H | Br |
| 5-14 | H | H | N-oxide | 2-chloro pyridin73-yl | $SCF_3$ | H | i-$C_3F_7$ | H | Br |
| 5-15 | H | H | N-oxide | phenyl | $S(O)CF_3$ | H | i-$C_3F_7$ | H | Br |
| 5-16 | H | H | N-oxide | phenyl | $SO_2CF_3$ | H | i-$C_3F_7$ | H | Br |
| 5-17 | H | H | N-oxide | phenyl | $SCF_3$ | H | 2-$C_4F_9$ | H | Br |
| 5-18 | H | H | N-oxide | phenyl | $S(O)CF_3$ | H | 2-$C_4F_9$ | H | Br |
| 5-19 | H | H | N-oxide | phenyl | $SO_2CF_3$ | H | 2-$C_4F_9$ | H | Br |
| 5-20 | Me | H | N | phenyl | $SCF_3$ | H | i-$C_3F_7$ | H | Cl |
| 5-21 | Me | H | N | 2-fluorophenyl | $SCF_3$ | H | i-$C_3F_7$ | H | Cl |
| 5-22 | Me | H | N | 2-chloro pyridin-3-yl | $SCF_3$ | H | i-$C_3F_7$ | H | Cl |
| 5-23 | Me | H | N | phenyl | $SCF_3$ | H | i-$C_3F_7$ | H | Br |
| 5-24 | Me | H | N | 2-fluoro phenyl | $SCF_3$ | H | i-$C_3F_7$ | H | Br |
| 5-25 | Me | H | N | 2-chloro pyridin-3-yl | $SCF_3$ | H | $C_3F_7$ | H | Br |
| 5-26 | Me | H | N | phenyl | $S(O)CF_3$ | H | i-$C_3F_7$ | H | Br |
| 5-27 | Me | H | N | phenyl | $SO_2CF_3$ | H | i-$C_3F_7$ | H | Br |

(continued)

| Compound No. | $R_1$ | $R_2$ | A | $Q_1$ | $Y_1$ | $Y_2$ | $Y_3$ | $Y_4$ | $Y_5$ |
|---|---|---|---|---|---|---|---|---|---|
| 5-28 | Me | H | N | phenyl | $SCF_3$ | H | 2-$C_4F_9$ | H | Br |
| 5-29 | Me | H | N | phenyl | $S(O)CF_3$ | H | 2-$C_4F_9$ | H | Br |
| 5-30 | Me | H | N | phenyl | $SO_2CF_3$ | H | 2-$C_4F_9$ | H | Br |

Table 5-2

| Compound No. | $R_1$ | $R_2$ | A | $Q_1$ | $Y_1$ | $Y_2$ | $Y_3$ | $Y_4$ | $Y_5$ |
|---|---|---|---|---|---|---|---|---|---|
| 5-31 | Me | H | N-oxide | phenyl | $SO_2CF_3$ | H | 2-$C_4F_9$ | H | Br |
| 5-32 | Me | H | N-oxide | phenyl | $SCF_3$ | H | i-$C_3F_7$ | H | Br |
| 5-33 | Me | H | N-oxide | 2-fluoro phenyl | $SCF_3$ | H | i-$C_3F_7$ | H | Br |
| 5-34 | Me | H | N-oxide | 2-chloro pyridin-3-yl | $SCF_3$ | H | i-$C_3F_7$ | H | Br |
| 5-35 | Me | H | N-oxide | phenyl | $S(O)CF_3$ | H | i-$C_3F_7$ | H | Br |
| 5-36 | Me | H | N-oxide | phenyl | $SO_2CF_3$ | H | i-$C_3F_7$ | H | Br |
| 5-37 | Me | H | N-oxide | phenyl | $SCF_3$ | H | 2-$C_4F_9$ | H | Br |
| 5-38 | Me | H | N-oxide | phenyl | $S(O)CF_3$ | H | 2-$C_4F_9$ | H | Br |
| 5-39 | Me | H | N-oxide | phenyl | $SO_2CF_3$ | H | 2-$C_4F_9$ | H | Br |
| 5-40 | Me | Me | N | 2-fluorophenyl | $SCF_3$ | H | i-$C_3F_7$ | H | Cl |
| 5-41 | Me | Me | N | 2-chloro pyridin-3-yl | $SCF_3$ | H | i-$C_3F_7$ | H | Cl |
| 5-42 | Me | Me | N | phenyl | $SCF_3$ | H | i-$C_3F_7$ | H | Br |
| 5-43 | Me | Me | N | 2-fluoro phenyl | $SCF_3$ | H | i-$C_3F_7$ | H | Br |
| 5-44 | Me | Me | N | 2-chloro pyridin-3-yl | $SCF_3$ | H | i-$C_3F_7$ | H | Br |
| 5-45 | Me | Me | N | phenyl | $S(O)CF_3$ | H | i-$C_3F_7$ | H | Br |
| 5-46 | Me | Me | N | phenyl | $SO_2CF_3$ | H | i-$C_3F_7$ | H | Br |
| 5-47 | Me | Me | N | phenyl | $SCF_3$ | H | 2-$C_4F_9$ | H | Br |
| 5-48 | Me | Me | N | phenyl | $S(O)CF_3$ | H | 2-$C_4F_9$ | H | Br |
| 5-49 | Me | Me | N | phenyl | $SO_2CF_3$ | H | 2-$C_4F_9$ | H | Br |
| 5-50 | H | Me | N | phenyl | $OCF_3$ | H | i-$C_3F_7$ | H | Br |
| 5-51 | H | Me | N | 2-chloro pyridin-3-yl | $OCF_3$ | H | i-$C_3F_7$ | H | Br |
| 5-52 | Me | H | N | phenyl | $OCF_3$ | H | i-$C_3F_7$ | H | Br |
| 5-53 | Me | H | N | 2-chloropyridin -3-yl | $OCF_3$ | H | i-$C_3F_7$ | H | Br |
| 5-54 | H | Me | N | phenyl | OMe | H | i-$C_3F_7$ | H | Br |
| 5-55 | H | Me | N | 2-chloro 1 pyridin-3-yl | OMe | H | i-$C_3F_7$ | H | Br |
| 5-56 | Me | H | N | phenyl | OMe | H | i-$C_3F_7$ | H | Br |
| 5-57 | Me | H | N | 2-chloro pyridin-3-yl | OMe | H | i-$C_3F_7$ | H | Br |
| 5-58 | Me | Me | N | phenyl | OMe | H | i-$C_3F_7$ | H | C1 |
| 5-59 | Me | Me | N | 2-chloro pyridin-3-yl | OMe | H | i-$C_3F_7$ | H | Cl |

General Formula (D)

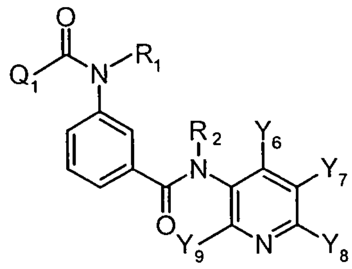

(D)

Table 6-1

| Compound No. | $R_1$ | $R_2$ | $Q_1$ | $Y_6$ | $Y_7$ | $Y_8$ | $Y_9$ |
|---|---|---|---|---|---|---|---|
| 6-1 | H | H | phenyl | $SCF_3$ | H | i-$C_3F_7$ | Cl |
| 6-2 | H | H | phenyl | $SCF_3$ | H | i-$C_3F_7$ | Br |
| 6-3 | H | H | 2-chloro pyridin-3-yl | $SCF_3$ | H | i-$C_3F_7$ | Br |
| 6-4 | H | H | phenyl | $S(O)CF_3$ | H | i-$C_3F_7$ | Br |
| 6-5 | H | H | phenyl | $SO_2CF_3$ | H | i-$C_3F_7$ | Br |
| 6-6 | H | H | phenyl | $SCF_3$ | H | i-$C_3F_7$ | Me |
| 6-7 | H | H | phenyl | $OCF_3$ | H | i-$C_3F_7$ | Br |
| 6-8 | H | H | phenyl | OMe | H | i-$C_3F_7$ | Br |
| 6-9 | Me | H | phenyl | $SCF_3$ | H | i-$C_3F_7$ | Cl |
| 6-10 | Me | H | phenyl | $SCF_3$ | H | i-$C_3F_7$ | Br |
| 6-11 | Me | H | 2-chloro pyridin-3-yl | $SCF_3$ | H | i-$C_3F_7$ | Br |
| 6-12 | Me | H | phenyl | $S(O)CF_3$ | H | i-$C_3F_7$ | Br |
| 6-13 | Me | H | phenyl | $SO_2CF_3$ | H | i-$C_3F_7$ | Br |
| 6-14 | Me | H | phenyl | $SCF_3$ | H | i-$C_3F_7$ | Me |
| 6-15 | Me | H | phenyl | $OCF_3$ | H | i-$C_3F_7$ | Br |
| 6-16 | Me | H | phenyl | OMe | H | i-$C_3F_7$ | Br |
| 6-17 | H | H | phenyl | Cl | H | i-$C_3F_7$ | $SCF_3$ |
| 6-18 | H | H | phenyl | Br | H | i-$C_3F_7$ | $SCF_3$ |
| 6-19 | H | H | 2-chloro pyridin-3-yl | Br | H | i-$C_3F_7$ | $SCF_3$ |
| 6-20 | H | H | phenyl | Br | H | i-$C_3F_7$ | $S(O)\ CF_3$ |
| 6-21 | H | H | phenyl | Br | H | i-$C_3F_7$ | $SO_2CF_3$ |
| 6-22 | H | H | phenyl | Me | H | i-$C_3F_7$ | $SCF_3$ |
| 6-23 | H | H | phenyl | Br | H | i-$C_3F_7$ | $OCF_3$ |
| 6-24 | H | H | phenyl | Br | H | i-$C_3F_7$ | OMe |
| 6-25 | Me | H | phenyl | Cl | H | i-$C_3F_7$ | $SCF_3$ |
| 6-26 | Me | H | phenyl | Br | H | i-$C_3F_7$ | $SCF_3$ |
| 6-27 | Me | H | 2-chloro pyridin-3-yl | Br | H | i-$C_3F_7$ | $SCF_3$ |
| 6-28 | Me | H | phenyl | Br | H | i-$C_3F_7$ | $S(O)CF_3$ |

(continued)

| Compound No. | $R_1$ | $R_2$ | $Q_1$ | $Y_6$ | $Y_7$ | $Y_8$ | $Y_9$ |
|---|---|---|---|---|---|---|---|
| 6-29 | Me | H | phenyl | Br | H | $i-C_3F_7$ | $SO_2CF_3$ |
| 6-30 | Me | H | phenyl | Me | H | $i-C_3F_7$ | $SCF_3$ |
| 6-31 | Me | H | phenyl | Br | H | $i-C_3F_7$ | $OCF_3$ |
| 6-32 | Me | H | phenyl | Br | H | $i-C_3F_7$ | OMe |
| 6-33 | H | H | phenyl | $SCF_3$ | H | $OCH (CF_3)_2$ | Cl |

Table 6-2

| Compound No. | $R_1$ | $R_2$ | $Q_1$ | $Y_6$ | $Y_7$ | $Y_8$ | $Y_9$ |
|---|---|---|---|---|---|---|---|
| 6-34 | H | H | phenyl | $SCF_3$ | H | $OCH(CF_3)_2$ | Br |
| 6-35 | H | H | 2-chloro pyridin-3-yl | $SCF_3$ | H | $OCH(CF_3)_2$ | Br |
| 6-36 | H | H | phenyl | $S (O) CF_3$ | H | $OCH(CF_3)_2$ | Br |
| 6-37 | H | H | phenyl | $SO_2CF_3$ | H | $OCH(CF_3)_2$ | Br |
| 6-38 | H | H | phenyl | $SCF_3$ | H | $OCH(CF_3)_2$ | Me |
| 6-39 | H | H | phenyl | $OCF_3$ | H | $OCH(CF_3)_2$ | Br |
| 6-40 | H | H | phenyl | OMe | H | $OCH(CF_3)_2$ | Br |
| 6-41 | Me | H | phenyl | $SCF_3$ | H | $OCH(CF_3)_2$ | Cl |
| 6-42 | Me | H | phenyl | $SCF_3$ | H | $OCH(CF_3)_2$ | Br |
| 6-43 | Me | H | 2-chloro pyridin-3-yl | $SCF_3$ $SCF_3$ | H | $OCH(CF_3)_2$ | Br |
| 6-44 | Me | H | phenyl | $S(O)CF_3$ | H | $OCH(CF_3)_2$ | Br |
| 6-45 | Me | H | phenyl | $SO_2CF_3$ | H | $OCH(CF_3)_2$ | Br |
| 6-46 | Me | H | phenyl | $SCF_3$ | H | $OCH(CF_3)_2$ | Me |
| 6-47 | Me | H | phenyl | $OCF_3$ | H | $OCH(CF_3)_2$ | Br |
| 6-48 | Me | H | phenyl | OMe | H | $OCH(CF_3)_2$ | Bt |
| 6-49 | H | H | phenyl | C1 | H | $OCH(CF3)_2$ | $SCF_3$ |
| 6-50 | H | H | phenyl | Br | H | $OCH(CF_3)_2$ | $SCF_3$ |
| 6-51 | H | H | 2-chloro pyridin-3-yl | Br | H | $OCH(CF_3)_2$ | $SCF_3$ |
| 6-52 | H | H | phenyl | Br | H | $OCH(CF_3)_2$ | $S(O)CF_3$ |
| 6-53 | H | H | phenyl | Br | H | $OCH(CF_3)_2$ | $SO_2CF_3$ |
| 6-54 | H | H | phenyl | Me | H | $OCH(CF_3)_2$ | $SCF_3$ |
| 6-55 | H | H | phenyl | Br | H | $OCH(CF_3)_2$ | $OCF_3$ |
| 6-56 | H | H | phenyl | Br | H | $OCH(CF_3)_2$ | OMe |
| 6-57 | Me | H | phenyl | C1 | H | $OCH(CF_3)_2$ | $SCF_3$ |
| 6-58 | Me | H | phenyl | Br | H | $OCH (CF_3)$ | $SCF_3$ |
| 6-59 | Me | H | 2-chloro pyridin-3-yl | Br | H | $OCH(CF3)_2$ | $SCF_3$ |
| 6-60 | Me | H | phenyl | Br | H | $OCH(CF3)_2$ | $S(O)CF_3$ |
| 6-61 | Me | H | phenyl | Br | H | $OCH(CF_3)_2$ | $SO_2CF_3$ |
| 6-62 | Me | H | phenyl | Me | H | $OCH(CF_3)_2$ | $SCF_3$ |
| 6-63 | Me | H | phenyl | Br | H | $OCH(CF_3)_2$ | $OCF_3$ |

(continued)

| Compound No. | $R_1$ | $R_2$ | $Q_1$ | $Y_6$ | $Y_7$ | $Y_8$ | $Y_9$ |
|---|---|---|---|---|---|---|---|
| 6-64 | Me | H | phenyl | Br | H | $OCH(CF3)_2$ | OMe |

The physical properties of the compound of the present invention are shown in Table 7 below. Tetramethylsilane is used as an internal standard substance to record shift values of $^1$H-NMR as shown herein, unless otherwise particularly mentioned.

Table 7-1

| Compound No. | $^1$H-NMR (ppm) |
|---|---|
| 1-1 | ($CDCl_3$) δ 7.45-7.58(4H, m), 7.72(1H, d, J = 7.8Hz), 7.83-7.88(3H, m), 7.92(1H, d, J = 7.8Hz), 7.97 (1H, s), 8.20(1H, s), 8.27(1H, s), 8.52(1H, s). |
| 1-8 | ($CDCl_3$) δ 7.20-7.25(1H, m), 7.33-7.37(1H, m), 7.54-7.64(2H, m), 7.76(1H, dd, J = 1.5Hz and 7.8Hz), 7.86(1H, d, J = 1.5Hz), 7.95(1, dd, J = 1.5Hz and 7.8Hz), 7.99(1H, s), 8.17-8.22(1H, m), 8.34(1 H, d, J = 1,5Hz), 8.35(1H, ,d, J = 1.5 Hz), 8.65(1 H, d, J = 16.1 Hz). |
| 1-94 | ($CDCl_3$) δ 7.44(1H, dd, J = 4.9Hz and 7.3Hz), 7.60(1H, t, J = 7.8Hz), 7.79 (1H, d, J = 7.BHz), 7.86 (1H, d, J = 2.0Hz), 7.94(1H, d, J = 7.8Hz), 7.99(1H, s), 8.24(1H, dd, J = 2.0H and 7.3Hz), 8.30-8.32 (2H, m), 8.41 (1H, s), 8.55(1H, dd, J = 2.0Hz and 4.9Hz). |
| 1-143 | (DMSO-$d_6$) δ 7.33-7.40(2H, m), 7.57-7.62(2H, m), 7.68-7.73(1H, m), 7.81 (1H, d, J = 7.BHz), 8.01 (1H, d, J = 7.8Hz), 8,04(1H, s), 8,30(1H, d, J = 2.0Hz) 8.37(1H, s), 10.70(1H, s), 10.95(1H, s). |
| 1-145 | (DMSO-$d_6$) δ 7.37-7.42(2H, m), 7.6(1H, t, J = 7.BHz), 7.81(1H, d, J = 7.8Hz), 8.04-8.11(4H, m), 8.31(1H, d, J = 2.0Hz), 8.39(1H, s), 10.53(1H, s), 10.93 (1H, s). |
| 1-146 | (DMSO-$d_6$) δ 7.56-7.65(3H, m), 7.81 (1H, d, J =7.8Hz), 8.02-8.10(4H, m), 8.30(1H, d, J = 2.0Hz), 8.38(1H, s), 10.56(1H, s), 10.91 (1H, s). |
| 1-148 | (DMSO-$d_6$) δ 7.63(1H, t,J = 7.BHz), 7.85(1H, d, J = 7.8Hz), 8.05(1H, s), 8.11(1H, dd, J = 1.5Hz and 7.8Hz), 8.22-8.25(2H, m), 8.31(1H, d, J = 2.0Hz) 8.38-8.41(3H, m), 10.83(1H, s), 10.95(1H, s). |
| 1-149 | (DMSO-$d_6$) δ 7.59(1H, t, J = 7.8Hz), 7.71-7.88(5H, m), 7.97(1H, d, J = 8.3Hz), 8.04(1H, s), 8.30 (1H,s), 8.31 (1H, s), 10.84(1H, s), 10.93(1H, s). |
| 1-150 | (DMSO-$d_6$) δ 7.62(1H, t, J = 7.8Hz), 7.83(1H, d, J = 7.8Hz), 7.94(2H, d, J = 8.3Hz), 8.05(1H, s), 8.11(1H, d,J = 7.8Hz), 8.20(2H, d, J = 8.3Hz), 8.31 (1H, d,J = 1.5Hz), 8.40(1H, s), 10.73(1H, s). 10.94(1H,s). |
| 1-151 | (DMSO-$d_6$) δ 7.25-7.30(2H, m), 7.57-7.65(2H, m), 7.82(1H, d, J = 7.8Hz), 7.96(1H, d, J = 1.5Hz and 7.8Hz), 8.04(1H, s), 8.29-8.33(2H, m), 10.96 (1H, s), 11.07(1H, s). |
| 1-152 | (DMSO-$d_6$) δ 7.23-7.28(1H, m), 7.42-7.48(1H, m), 7.59(1H, t, J = 7.8Hz), 7.76-7.83(2H, m), 7.99 (1H, d, J = 8.8Hz), 8.04(1H, s), 8.30(1 H, d, J = 1.5Hz), 8.34(1H, s), 10.69(1H, s), 10.94(1H, s). |
| 1-158 | ($CDCl_3$) δ 7.42-7.46(1H, m), 7.60(1H, t, J = 7.8Hz), 7.80(1H, d, J = 7.8Hz), 7.94(1H, d, J = 7.8Hz), 8.02(1H, s), 8.03(1H, s), 8.22-8.28(2H, m), 8.33 (1H, s), 8.41(1H, s), 8.55(1H, dd, J = 2.0Hz and 2.9Hz). |
| 1-166 | (DMSO-$d_6$) δ 2.44(3H, s), 7.45-7.57(4H, m), 7.64(1H, s), 7.73(1H, dd, J = 1.5,7.8Hz), 7.88(1H, s), 7.94-7.97(2H, m), 8.10-8.13(1H, m), 8.30(1H, t, J = 1.5Hz), 8.94(1H, s), 9.27(1H, s). |
| 1-176 | ($CDCl_3$) δ 7.51-7,55 (2H, m), 7.57-7.62 (2H, m), 7.75 (1H, dd, J =1.5,8.3Hz), 7.89-7.91 (3H, m), 8.03 (1H, s), 8.13 (1H, d, J =2.OHz), 8.30 (1H, s), 8.38 (1H, t, J =2.0Hz), 8.52 (1H, s). |
| 1-180 | (DMSO-$d_6$) δ 7.41 (1H, dd, J =4.9,7.3Hz), 7.52 (1H, t, J =7.8Hz), 7.80 (1H, d, J =7.8Hz), 7.94 (1H, dd, J =2.0,7.8Hz), 8.12 (1H, d, J =1.5Hz), 8.14 (1H, d, J =1.SHz), 8.24 (1H, s), 8.33-8.34 (1H, m), 8.50 (1H, dd, J =2.0,4.9Hz), 10.38 (1H, s), 10.54 (1H, s). |

Table 7-2

| Compound No. | $^1$H-NMR (ppm) |
|---|---|
| 1-191 | (DMSO-$d_6$) δ 7.53-7.63(4H, m), 7.74(1H, d, J = 7.8Hz), 7.98-8.01(2H, m), 8.08(1H, d, J = 8.3Hz), 8.24(1H, s), 8.38(1H, t, J = 1.5Hz). 8.81(1H, s), 10.51(1H,s), 1 0.82(1H, s). |
| 1-195 | ($CDCl_3$) δ 7.44 (1H, dd, J =4.9,7.8Hz), 7.60 (1H, t, J =7.8Hz), 7.76 (1H, d, J =7.8Hz), 8.02 (1H, d, J =7.8Hz), 8.24-8.25 (1H, m), 8.26 (1H, t, J =2.0Hz), 8.30 (1H, d, J =2.0Hz), 8.34 (1H, d, J =2.0Hz) 8.39 (1H, s), 8.55 (1H, dd, J =2.0.4.9Hz), 9.06 (1H, s). |
| 1-201 | (DMSO-$d_6$) δ 7.53-7.64(4H, m), 7.80(1H, d, J = 7.8Hz), 7.99-8.04(3H, m), 8.08-8.11(1H, m), 8.33 (1H, d, J =2.0Hz), 8.40(1H, s), 10.51(1H, s), 10.96 (1H, s). |
| 1-202 | (DMSO-$d_6$) δ 7.33-7.40(2H, m), 7.56-7.63(2H, m), 7.67-7.72(1H, m), 7.79 (1H, d, J = 8.3Hz), 7.99 (1H, d, J = 7.8Hz), 8.04(1H, s), 8.32(1H, d, J = 2.0Hz), 8.3 6(1H, s), 10.68(1H, s), 10. 97(1H, s). |
| 1-203 | (DMSO-$d_6$) δ 7.57-7.63(2H, m), 7.82(1H, d, J = 7.8Hz), 7.97(1H, d, J = 7.8Hz), 8.04(1H, s), 8.12 (1H, dd, J = 2.0Hz and 7.8Hz), 8.33(2H, s), 8.56(1H, dd. J = 2.0Hz and 4.9Hz), 10.91(1H, s), 10.99 (1H, s). |
| 1-204 | (DMSO-$d_6$) δ 7.53-7.63(4H, m), 7.80(1H, d, J = 7.8Hz), 7.98-8.11(4H, m), 8.33(1H, d, J = 2.0Hz), 8.40(1H, s), 10.50(1H, s), 10.93(1H, s). |
| 1-205 | (DMSO-$d_6$) δ 7.28-7.40(2H, m), 7.57-7.63(2H, m), 7.68-7.72(1H, m), 7.79(1H, d, J = 7.8Hz), 8.00 (1H, d, J = 7.8Hz), 8.04(1H, s), 8.32-8.36 (2H, m), 10.67(1H, s). 10.95(1H, s). |
| 1-207 | ($CDCl_3$) δ 7.50-7.61(4H, m), 7.75(1H, dd, J = 1.5,6.3Hz), 7.88-7.90(2H, m), 7.95-7.99(3H, m), 8.04 (1H, s), 8.30-8.33(2H, m). |
| 1-241 | ($CDCl_3$) δ 7.36-7.43(3H, m), 7.47-7.53(2H, m), 7.63-7.65(2H, m), 7.81-7.87(3H, m), 8.20(1H, s), 8.28(1H, s), 8.39(1H, s). |
| 1-242 | ($CDCl_3$) δ 7.14-7.20(1H, m), 7.26-7.31(1H, m), 7.42-7.51(3H, m). 7.68-7.71 (2H, m), 7.89(1H, d, J = 7.8Hz), 8.10(1H, t, J = 7.8Hz), 8.21 (1H, s), 8.25(1H, s), 8.63(1H, d, J = 15.1Hz). |
| 1-243 | (DMSO-$d_6$) δ 7.48-7.67(4H, m), 7.73-7.81(2H, m), 7.94-8.14(4H, m), 8.38 (1H, s). 10.51(1H, s). 10.63(1H, s). |
| 1-244 | (CDC13) δ 7.18-7.36(3H, m), 7.50-7.59(3H, m), 7.70-7.96(3H, m), 8.16-8.20(1H, m). 8.291(1H, s), 8.66(1H, s). |
| 1-245 | (DMSO-$d_6$) δ 7.47-7,55(2H, m), 7.76-7.81 (1H, m), 7.86(1H, d, J = 1.5Hz), 8.22-8.43(6H, m), 9.35 (1H, s), 10.28(1H, s). |
| 1-246 | (DMSO-$d_6$) δ 7.55-7.62(1H, m), 7.76-7.86(2H, m), 8.04-8.16(6H, m), 8.36(1H, s), 10.64(1H, s), 10.73(1H, s). |
| 1-247 | (DMSO-$d_6$) δ 7.36-7.41 (2H, m), 7.52-7.60(1H, m), 7.73-7.85(2H, m), 8.04-8.14(4H, m), 8.35(1H, s), 10.49(1H, s), 10.62(1H, s). |

Table 7-3

| Compound No. | $^1$H-NMR (ppm) |
|---|---|
| 1-248 | (DMSO-$d_6$) δ 7.55-7.62(1H, m), 7.75-7.86(2H, m), 7.94(2H, d, J = 8.3Hz), 8.05-8.14(2H, m), 8.19 (2H, d, J = 8.3Hz), 8.36(1H, d, J = 2.0Hz), 10.64(1H, s), 10.72(1H, s). |
| 1-249 | (DMSO-$d_6$) δ 7.52-7.64(3H, m), 7.70-7.85(2H, m), 8.01-8.17(4H, m), 8.34 (1H, s), 10.53(1H, s), 10.61(1H, s). |
| 1-253 | (DMSO-$d_6$) δ 3.88(3H, s), 7.26(1H, s), 7.50-7,62(5H, m), 7.74(1H, d, J = 7.3Hz), 7.97-8.05(3H, m), 8.32(1H, t, J = 1.5Hz), 10.08(1H, s), 10.46(1H,s). |
| 1-254 | (DMSO-$d_6$) δ 3.87(3H, s), 7.26(1H, s), 7.32-7.38(2H, m), 7.50-7.61(3H, m), 7.66-7.75(2H, m), 7.96 (1H, d, J = 7.8Hz), 8.28(1H, s), 10.11(1H, s), 10.63(1H, s). |

(continued)

| Compound No. | $^1$H-NMR (ppm) |
|---|---|
| 1-255 | ($CDCl_3$) δ 3.88(3H, s), 7.10(1H, s), 7.17-7.21(2H, m), 7.50(1H, s), 7.53 (1H, t, J = 7.8Hz), 7.66(1H, s), 7.73(1H, d, J = 7.8Hz), 7.89-7.98(4H, m), 8.20(1H, t, J = 2.0Hz). |
| 1-256 | ($CDCl_3$)δ 3.90(3H, s), 7.11 (1H, s), 7.41-7.45(1 H, m), 7.51 (1H, s), 7.55 (1H, t, J = 7.8Hz), 7.64(1 H, s), 7.76-7.78(1 H, m), 7.92(1H, dd, J = 1.5,7.8Hz), 8.20-8.24(2H, m), 8.39(1H, s), 8.54(1H, dd, J = 2.0,4.9Hz). |
| 1-257 | ($CDCl_3$)δ 3.87(3H, s), 7.12(1H, s), 7.50-7.59(4H, m), 7.67(1H, s), 7.71 (1H, s), 7.74(1H, dd, J = 1.0,7.8Hz), 7.88-7.91 (2H, m), 7.94-8.00(2H, m), 8.24(1H, t, J = 2.0Hz). |
| 1-258 | ($CDCl_3$)δ 3.87(3H, s), 7.13(1H, s), 7.19-7.25(1H, m), 7.32-7.37(1H, m), 7.51-7.59(2H, m), 7.66(1H, s), 7.72(1H, s), 7.76(1H, dd, J = 1.5,7.8Hz), 7.96(1H, dd, J = 1.5,7.8Hz), 8.17-8.21(1H, m), 8.27 (1 H, t, J = 1.5Hz), 8.62(1H, d.J=16.1Hz). |
| 1-259 | ($CDCl_3$) δ 3,88(3H, s), 7.13(1H, s), 7.43(1H, dd, J = 4.4,7.8Hz), 7.56(1H, t, J = 7.8Hz), 7.63(1H, s), 7.71(1H, s), 7.78(1H, d, J = 7.8Hz), 7.92-7.95(1H, m), 8.21-8.24(2H. m), 8.38(1H, s), 8.55(1H, dd, J = 2.0,4.4Hz). |
| 1-260 | ($CDCl_3$) δ 2.36 (3H, s), 3.85 (3H, s), 6.96 (1H, s), 7.13 (1H, s), 7.50-7.54 (3H, m), 7.58 (1H, t, J =7.3Hz), 7.70-7.74 (2H, m), 7.88-7.90 (2H, m), 7.94 (1H, d, J =8.3Hz), 8.00 (1H, s), 8.22 (1H, s). |
| 1-261 | ($CDCl_3$) δ 3.70 (3H, s), 3.86 (3H, s), 6.97 (1H, s), 7.14 (1H, s), 7.42-7.45 (1H, m), 7.55 (1H, t, J =7.8Hz), 7.7 2(1H, s), 7.7 5(1H, d, J =7.8Hz), 7.92 (1H, d, J =7,8Hz). 8.21-8.24 (2H. m), 8.35 (1H, s), 8.55 (1H, d, J =6.8Hz). |
| 1-262 | ($CDCl_3$) δ 2.37 (3H, s), 3.86 (3H, s), 6.97 (1H, s), 7.14 (1H, s), 7.32 (1H, d, J =8.3Hz), 7.34 (1H, t, J =7.8Hz), 7.52-7.58 (2H, m), 7.73 (2H, d, J =6.8Hz), 7.95 (1H, d, J =7,8Hz), 8.19 (1H, t, J =7.8Hz), 8.25 (1H, s), 8.60 (1H, d, J =15.6Hz). |
| 1-263 | ($CDCl_3$) δ 3.88 (3H, s), 7.05 (1H, s), 7.34 (1H, s), 7.51 (3H, t, J =8.3Hz), 7.58 (1H, t, J =7.8Hz), 7.64 (1H, s), 7.72 (1H, d, J =6.8Hz), 7.88 (2H, d, J =6.8Hz), 7.94 (1H, d. J =6.3Hz). 8.02 (1H, s), 8.22-8.23 (1H, m). |
| 1-264 | ($CDCl_3$) δ 3.90 (3H, s), 7.06 (1H, s), 7.19-7.24 (1H, m), 7.32-7.36 (2H, m), 7.51 (1H, s), 7.56 (1H, t, J =7.8Hz), 7.63 (1H, s), 7.75 (1H, d, J =7.8Hz), 7.95 (1H, d, J =7.8Hz), 8.18 (1H, t, J =7.8Hz), 8.26 (1H,s). 8.62 (1H, d,J=16.1Hz). |
| 1-265 | ($CDCl_3$) δ 3.90 (3H, s), 7.07 (1H, s), 7,35 (1H, s), 7.43 (1H, dd, J =4.8,7.8Hz), 7.55 (1H, t, J =7.8Hz), 7.61 (1H, s), 7.76-7.78 (1H, m), 7.92-7.94 (1H, m), 8.21-8.23 (2H, m), 8.37 (1H, s), 8.55 (1H, dd, J =2,0,4.8Hz). |

Table 7-4

| Compound No. | $^1$H-NMR (ppm) |
|---|---|
| 1-266 | ($CDCl_3$) δ 3.88 (3H, s), 7.10 (1H, s), 7.49 (1 H, s), 7.55 (1H, t, J =7.8Hz), 7.60 (1H, s), 7.75 (1H, d, J =7.8Hz), 7.81 (2H, d, J =7.8Hz), 7.97-8.01 (3H. m), 8.06 (1H, s), 8.19-8.20 (1H, m). |
| 1-267 | ($CDCl_3$) δ 3.89 (3H, s), 7.10 (1H, m), 7.50-7.56 (3H, m), 7.65 (1H, s), 7.74 (1H, d, J =7.8Hz), 7.91-7.96 (1H, m), 8.04 (1H, d, J =7.8Hz), 8.30 (1H, d, J =7.8Hz), 8.39 (1H, s). 8.64 (1H, d, J =4.4Hz), 10.22 (1H, s). |
| 1-268 | DMSO-$d_6$) δ 3.90 (3H, s), 7.13 (1 H, s), 7.45-7.52 (3H, m), 7.80 (1H, d, J =7.8Hz), 8.13(1H, d, J =7.8Hz), 8.28 (1H, s), 8.35-8.37 (1H, m), 8.74 (1H, d, J =6.8Hz). 9.22-9.23 (1H, m). 9.70 (1H, s). 10.55 (1H, s). |
| 1-269 | ($CDCl_3$) δ 4.37(2H, q, J = 7.8Hz), 7.12(1H, s), 7.35-7.42(3H, m), 7.50(1H, t, J = 7.3Hz), 7.57(1H, s), 7.62(1H, d, J = 7.8Hz), 7.81-7.84(2H, m), 7.91-7.93 (2H, m), 8.16(1H, s), 8.43(1H, s). |
| 1-270 | ($CDCl_3$) δ 4.45(2H, q, J = 7.8Hz), 7.16(1H, d, J = 1.5Hz), 7.34(1H, dd, J = 4.9,7.8Hz), 7.48(1H, t, J = 7.8Hz), 7.61(1H, s), 7.68(1H, d, J = 7.8Hz), 7.88 (1H, s), 7.90(1H, d, J = 7,8Hz), 8.07(1H, dd, J = 2.0,7.8Hz), 8.17(1H, d, J = 1.5Hz), 8.44(1H, dd, J = 2.0,4.9Hz), 8.71(1 H, s). |

(continued)

| Compound No. | $^1$H-NMR (ppm) |
|---|---|
| 2-141 | ($CDCl_3$)δ 3.55(3H, s), 7.18-7.22(2H, m), 7.27-7.35(4H, m), 7.42(1 H, t, J = 7.8Hz), 7.62(1H, s), 7.73(1H, d, J = 7.8Hz), 7.97(1H, s), 7.99(1H, s), 8.10 (1H. s). |
| 2-143 | ($CDCl_3$) δ 3.55(3H, s), 6.84(1H, broad), 7.09(1H, broad), 7.19-7.41(4H, m), 7.62-7.68(1H, m), 7.74-7.83(2H, m). 8.00(1 H, s). 8.01 (1 H. s). |
| 2-145 | ($CDCl_3$) δ 3.57(3H, s), 6.89-6.93(2H, m), 7.32-7.36(3H, m), 7.46(1H, t, J = 7.8Hz), 7.52(1H, s), 7.62(1H, t, J = 2.0Hz), 7.87(1H, s), 7.99-8.01(2H, m), |
| 2-148 | ($CDCl_3$) δ 3.58(3H, s), 7.30(1H, d, J = 7.8Hz), 7.43-7.51(3H, m), 7.71 (1H, s), 7.75(1H, d, J = 7.8Hz), 7.98-8.01(3H, m), 8.09(2H, d, J = 8.3Hz). |
| 2-151 | ($CDCl_3$) δ 3.56(3H, s), 6.69-6.74(2H, m), 7.13-7.18(1H, m), 7.43-7.47 (2H, m), 7.72(1H, s), 7.77-7.81(1H, m), 7.91(1H, s), 8.01(2H, s). |
| 2-152 | ($CDCl_3$) δ 3.54(3H, s), 6.61(1H, broad), 6.85(1H, broad), 7.40-7.49(3H, m), 7.65(1H, broad), 7.76 (1H, d, J = 7.3Hz), 7.93(1H, d, J = 7.3Hz), 8.00(1H, s), 8.01(1H, s). |
| 2-158 | ($CDCl_3$) δ 3.59(3H, s), 7.14(1H, dd, J = 4.9,7.3Hz), 7.41-7.44(2H, m), 7.60(1H, d, J = 4.9Hz), 7.72-7.73(2H, m), 7.91-7.94(1 H, m), 8.01(2H, s), 8.26(1H, d, J = 2.9Hz). |
| 2-172 | ($CDCl_3$) δ 3.55 (3H, s), 6.88-6.94 (2H, m), 7.31-7.36 (3H, m), 7.46 (1H, t, J =7.8Hz), 7.62 (1H, t, J =2.0Hz), 7.72 (1H, d, J =7.8Hz), 8.12 (1H, d, J =2.0Hz), 8.16 (1H, s), 8.24 (1 H, s). |
| 2-173 | ($CDCl_3$) δ 3.53 (3H, s), 6.60-6.70 (1H, m), 6.85-6.91 (1H, m), 7.41-7.47 (3H, m), 7.60 (1H, s), 7.74 (1H, d, J =7.3Hz), 8.13 (1H, s), 8.17 (1H, s), 8.25 (1H,s). |
| 2-174 | ($CDCl_6$) δ 3.56 (3H, s), 6.70-6.74 (2H, m), 7.13-7.23 (1H, m), 7.44-7.49 (2H, m), 7.71 (1H, s), 7.77-7.79 (1H, m), 8.12 (1H, s), 8.13 (1H, s), 8.25 (1H, s). |

Table 7-5

| Compound No. | $^1$H-NMR (ppm) |
|---|---|
| 2-175 | ($CDCl_3$) δ 3.55 (3H, s), 7.32-7.46 (5H, m), 7.75-7.77 (2H, m), 8.13 (1H, s), 8.23 (1H, s), 8.59-8.62 (2H, m). |
| 2-176 | ($CDCl_3$) δ 3.57(3H, s), 7.20-7.33(5H, m), 7.40(1H, d, J = 7.8Hz), 7.47(1H, t, J = 7.8Hz), 7.57(1H, s), 7.71(1H, d, J = 7.8Hz), 8.03-8.05(1H. m), 8.11(1H, s), 8.26(1H, s). |
| 2-177 | ($CDCl_3$) δ 3.54 (3H, s), 6.80-6.90 (1H, m), 7.11-7.15 (2H, m), 7.40-7.42 (5H, m), 7.71-7.73 (1H, m), 8.12 (1H, d, J =2.0Hz), 8.20 (1H, d, J =2.0Hz). |
| 2-178 | ($CDCl_3$) δ 3.56 (3H, s), 7.31 (1H, d, J =7.8Hz), 7.42 (2H, d, J =8.3Hz), 7.46 (1H, t, J =7.8Hz), 7.53 (2H, d, J =8.3Hz), 7.69 (1H, s), 7.72 (1H, dd, J = 1.0,7.8Hz), 8.14 (1H, d, J =2.0Hz), 8.22 (1 H, s), 8.27 (1H, s). |
| 2-179 | ($CDCl_3$) δ 3.55 (3H, s), 7.18-7.19 (1 H, m), 7.42-7.52 (2H, m), 7.65-7.66 (1H, m), 7.73-7.75 (1H, m), 7.90-7.91 (1H, m), 8.12-8.13 (2H, m), 8.22-8.25 (2H, m). |
| 2-180 | ($CDCl_3$) 6 3.58 (3H, s), 7.15 (1H, dd, J =4.9,7.3Hz), 7.42-7.45 (2H, m), 7.60 (1H, d, J =7.3Hz), 7.71-7.72 (2H, m), 8.13 (1H, s), 8.19-8.29 (3H, m). |
| 2-181 | ($CDCl_3$) δ 3.58 (3H, s), 7.22 (1H, dd, J =4.9,7.8Hz), 7.35 (1H, d, J =7.8Hz), 7.47 (1H, t, J =7.8Hz), 7.68 (1H, s), 7.73 (2H, d, J =7.8Hz), 8.12 (1H, d, J =1.9Hz), 8.23 (1H, s), 8.24 (1H, s), 8.47 (1H, s), 8.50 (1H, d, J =3.4Hz). |
| 2-191 | ($CDCl_3$) δ 3.57(3H, s), 7.18-7.36(6H, m), 7.43(1H, t, J = 7.8Hz), 7.61(1H, d, J = 2.0Hz), 7.68(1H, d, J = 7.8Hz), 8.28(1H, d, J = 2.0Hz), 8.32(1H, d, J = 2.0Hz), 8.76(1H, s). |
| 2-195 | ($CDCl_3$) δ 3.45 (3H, s), 7.00-7.11 (4H, m), 7.43-7.46 (1H, m), 7.82-7.83 (1H, m), 8.26-8.28 (2H, m), 8.40-8.45 (2H, m). |

(continued)

| Compound No. | $^1$H-NMR (ppm) |
|---|---|
| 2-201 | ($CDCl_3$) δ 3.56(3H, s), 7.19-7.37(6H, m), 7.44(1H, t, J = 7.3Hz), 7.59 (1H, s), 7.71 (1H, d, J = 7.3Hz), 7.87(1H, s), 7.99(1H, s), 8.00(1H, s). |
| 2-203 | ($CDCl_3$) δ 3.58(3H, s), 7.13(1H, dd, J = 4.9,7.8Hz), 7.42-7.44(2H, m), 7.59-7.61 (1H, m), 7.71-7.73 (2H, m), 7.91 (1H, s), 8.00(1H, s), 8.02(1H, s), 8.25(1H, d, J = 2.4Hz). |
| 2-207 | ($CDCl_3$) δ 3.56(3H, s), 7.19-7.37(7H, m), 7.44(1 H, t, J = 7.8Hz), 7.61(1H, s), 7.71-7.74(1H, m), 7.95-7.97(2H, m). |
| 2-243 | ($CDCl_3$) δ 3.53(3H, s), 7.16-7.20(2H, m), 7.24-7.32(4H, m), 7.39(1H, t, J = 7.8Hz), 7.53(1H, s), 7.61 (1H, s), 7.70(1H, d, J = 7.8Hz), 7.78(1H, s), 7.82(1H, s). |
| 2-260 | ($CDCl_3$) δ 2.30 (3H, s), 3.55 (3H, s), 3.85 (3H, s), 6.95 (1 H, s), 7.1 (1H, s), 7.18-7.24 (3H. m). 7.27-7.40 (5H. m). 7.61 (1H, s), 7.67 (1H, d, J =7.3Hz). |
| 2-261 | ($CDCl_3$) δ 2.29 (3H, s), 3.54 (3H, s), 3.85 (3H, s), 6.96 (1H, s), 7.12 (2H, m), 7.34 (2H, m), 7.56 (1H, d, J =6.8Hz), 7.67 (1H, s), 7.70-7.71 (1H, m), 7.75 (1H, s), 8.23-8.24 (1H, m). |

Table 7-6

| Compound No. | $^1$H-NMR (ppm) |
|---|---|
| 2-262 | ($CDCl_3$) δ 2.30 (3H, s), 3.53 (3H, s), 3.85 (3H, s), 6.85 (1H, m), 6.95 (1H, m), 7.07 (1H, m), 7.12 (1H, m), 7.29-7.37 (5H, m), 7.61 (1H, m), 7.69 (1H, m). |
| 2-269 | ($CDCl_3$) δ 3.54(3H, s), 4.42(2H, q, J = 7.8Hz), 7.14-7.21(3H, m), 7.24-7.31 (4H, m), 7.37(1H, t, J = 7.8Hz), 7.46(1H, s), 7.60-7.62(2H, m), 7.66-7.69 (1H, m). |
| 2-270 | ($CDCl_3$) δ 3.53(3H, s), 4.45(2H, q, J = 7.8Hz), 7.09(1H, dd, J = 4.9,7.BHz), 7.16(1H, s), 7.33-7.37 (2H, m), 7.54(1H, dd, J = 1.5,7.8Hz), 7.61 (1H, s), 7.69-7.73(3H, m), 8.22(1H, dd J = 1.5,4.9Hz). |
| 2-281 | ($CDCl_3$) δ 3.59 (3H, s), 7.34 (1H, d, J =8.3Hz), 7.51 (1H, t, J =7.8Hz), 7.78 (2H, dd, J =3.9, 1.5Hz), 8.14 (1H, d, J =2.0Hz), 8.24 (1H, s), 8.35 (1H, s), 8.67 (2H, s), 9.11 (1H, s). |
| 3-49 | ($CDCl_3$) δ 3.21 (3H, s), 3.27 (3H, s), 3.76 (3H, s), 6.73 (1H, d, J =7.8Hz), 6.91-6.92 (1H, m), 6.94 (1H, d, J =7.8Hz), 7.09-7.13 (2H, m), 7.16-7.17 (3H, m), 7.19-7.24 (2H, m), 7.37 (1H, s). |
| 3-51 | ($CDCl_3$) δ 3.21 (3H, s), 3.28 (3H, s), 3.78 (3H, s), 6.88 (1H, s), 6.94 (1H, t, J =7.8Hz), 7.09-7.24 (9H, m). |

An insecticide comprising the compound represented by the general formula (1) of the present invention as an active ingredient is suitable for preventing insect pests such as various agricultural, forest insect pests and horticultural insect pests, stored grain insect pests, hygienic insect pests, nematodes or the like which are injurious to paddy rice, fruit trees, vegetables, other crops, flowers and the like, and has a strong insecticidal effect, for example, on LEPIDOPTERA such as cucumber moth (Diaphania indica), oriental tea tortrix moth (Homona magnanima), cabbage webworm (Hellulla undalis), summer fruit tortrix (Adoxophyes orana fasciata), smaller tea tortrix (Adoxophyes sp.), apple tortrix (Archips fuscocupreanus), peach fruit moth (Carposina niponensis), Manchurian apple moth (Grapholita inopinata), oriental fruit moth (Grapholita molesta), soybean pod borer (Leguminivora glycinivorella), mulberry leafroller (Olethreutes mori), citrus leafminer (Phyllocnistis citrella), persimmon fruit moth (Stathmopoda masinissa), tea leafroller (Caloptilia thevivora), azalea leafminer (Caloptilia zachrysa), apple leafminer (Phyllonorycter ringoniella), pear barkminer (Spulerrina astaurota), swallowtail butterfly (Papilio xuthus), common white (Piers rapae crucivora), tobacco budworm (Heliothis armigera), codling moth (Cydia pomonella), diamondback moth (Plutella xylostella), apple fruit moth (Argyresthia conjugella), peach fruit moth (Carposina niponensis), rice stem borer (Chilo suppressalis), rice leafroller (Cnaphalocrocis medinalis), tabacco moth (Ephestia elutella), mulberry pyralid (Glyphodes pyloalis), yellow rice borer (Scirpophaga incertulas), rice skipper (Parnara guttata), rice armyworm (Pseudaletia separata), pink borer (Sesamia inferens), cabbage moth (Mamestra brassicae), common cutworm (Spodoptera litura), beet armyworm (Spodoptera exigua), black cutworm (Agrotis ipsilon), turnip moth (Agrotis segetum), semilooper (Autographa nigrisigna), cabbage looper (Trichoplusia ni) and the like; HEMIPTERA such as aster leafhopper (Macrosteles fascifrons), green rice leafhopper (Nephotettix cincticeps), brown rice planthopper (Nilaparvata lugens), small brown planthopper (Laodelphax striatellus), whitebacked rice

planthopper (Sogatella furcifera), citrus psylla (Diaphorina citri), grape whitefly (Aleurolobus taonabae), silverleaf whitfly (Bermisia argentifolii), sweetpotato whitefly (Bemisia tabaci), greenhouse whitefly (Trialeurodes vaporariorum), turnip aphid (Lipaphis erysimi), cotton melon aphid (Aphis gossypii), spirea aphid (Aphis Citricola), green peach aphid (Myzus persicae), Indian wax scale (Ceroplastes ceriferus), comstock mealybug (Pseudococcus comstocki), Japanese mealybug (Planococcus kraunhiae), cottony citrus scale (Pulvinaria aurantii), camphor scale (Pseudaonidia duplex), San Jose scale (Comstockaspis perniciosa), arrowhead scale (Unaspis yanonensis), brown-winged green bug (Plautia Stali), brown marmorated stink bug (Halyomorpha mista) and the like; COLEOPTERA such as soybean beetle (Anomala rufocuprea), Japanese beetle (Popillia japonica), tobacco beetle (Lasioderma serricorne), powderpost beetle (Lyctus brunneus), twenty-eight-spotted ladybird (Epilachna vigintioctopunctata), adzuki bean weevil (Callosobruchus chinensis), vegetable weevil (Listroderes costirostris), maize weevil (Sitophilus zeamais), boll weevil (Anthonomus grandis grandis), rice water weevil (Lissorhoptrus oryzophilus), cucurbit leaf beetle (Aulacophora femoralis), rice leaf beetle (Oulema oryzae), striped flea beetle (Phyllotreta striolata), pine shoot beetle (Tomicus piniperda), Colorado potate beetle (Leptinotarsa decemlineata), Mexican beetle (Epilachna varivestis), corn rootworm (Diabrotica sp.), yellow-spotted longicorn beetle (Psacothea hilaris), white-spotted longicorn beetle (Anoplophora malasiaca) and the like; DIPTERA such as oriental fruit fly (Dacus(Bactrocera) dorsalis), rice leafminer (Agromyza oryzae), onion maggot (Delia antiqua), seedcorn maggot (Delia platura), soybean pod gall midge (Asphondylia sp.), muscid fly (Musca domestica), leafminer (Chromatomyia horticola), serpentine leafminer (Liriomyza trifolii), tomato leafminer (Liriomyza bryoniae), house mosquito (Culex pipiens) and the like; TYLENCHIDA such as coffee root-lesion nematode (Pratylenchus coffeae), lesion nematode (Pratylenchus sp.), potato cyst nematode (Globodera rostochiensis), root-knot nematode (Meloidogyne sp.), citrus nematode (Tylenchulus semipenetrans), phagous nematode (Aphelenchus avenae), chrysanthemum foliar nematode (Aphelenchoides ritzemabosi) and the like; THYSANOPTERA such as melon thrip (Thrips palmi), western flower thrip (Frankliniella occidentalis), yellow tea thrip (Scirtothrips dorsalis), yellow flower thrip (Thrips flavus), onion thrip (Thrips tabaci) and the like; and ORTHOPTERA such as German cockroach (Blattella germanica), American cockroach (Periplaneta americana), rice grasshopper (Oxya yezoensis) and the like.

The insecticide comprising the compound represented by the general formula (1) of the present invention as an active ingredient has a remarkable control effect on the above-exemplified insect pests which are injurious to paddy field crops, upland crops, fruit trees, vegetables, other crops, flowers and the like. Therefore, the desired effect as an insecticide of the present invention can be obtained by applying the insecticide to the paddy field, upland, paddy field water, stalks and leaves of fruit trees, vegetables, other crops, flowers and the like, or soil at a season at which the insect pests are expected to appear, before their appearance or at the time when their appearance is confirmed.

The insecticide of the present invention is generally prepared into conveniently usable forms according to an ordinary manner for preparation of agricultural and horticultural chemicals. That is, the compound represented by the general formula (1) and, optionally, an adjuvant may be blended with a suitable inert carrier in a proper proportion and prepared into a suitable preparation form such as a suspension, emulsifiable concentrate, soluble concentrate, wettable powder, granules, dust, tablets or the like through dissolution, separation, suspension, mixing, impregnation, adsorption or adhering.

The inert carrier which can be used in the present invention may be either solid or liquid. Such a material which can be an inert solid carrier includes, for example, soybean flour, cereal flour, wood flour, bark flour, saw dust, powdered tobacco stalks, powdered walnut shells, bran, powdered cellulose, extraction residue of vegetables, synthetic polymers such as powdered synthetic resins, inorganic mineral powder such as clays (for example, kaolin, bentonite, acid clay and the like), talcs (for example, talc, pyrophyllite and the like), silica powders or flakes (for example, diatomaceous earth, silica sand, mica, white carbon [synthetic, high-dispersion silicic acid, also called finely divided hydrated silicon, hydrated silicic acid, some of commercially available products contain calcium silicate as the major component]), activated carbon, powdered sulfur, pumice stone, calcined diatomite, brick groats, fly ash, sand, calcium carbonate, calcium phosphate and the like, chemical fertilizers (for example, ammonium sulfate, ammonium phosphate, ammonium nitrate, urea, ammonium chloride and the like), compost and the like. These carriers can be used singly or in combination of two or more kinds.

A material which can be the inert liquid carrier is selected from such a material which itself has solvency or which does not have such solvency but is capable of dispersing an effective ingredient compound with the aid of an adjuvant. The following are typical examples of the carrier and can be used singly or in combination of two or more kinds. Examples thereof include water, alcohols (for example, methanol, ethanol, isopropanol, butanol, ethylene glycol and the like), ketones (for example, acetone, methylethyl ketone, methyl isobutyl ketone, diisobutyl ketone, cyclohexanone and the like), ethers (for example, diethyl ether, dioxane, cellosolve, diisopropyl ether, tetrahydrofuran and the like), aliphatic hydrocarbons (for example, kerosene, mineral oil and the like), aromatic hydrocarbons (for example, benzene, toluene, xylene, solvent naphtha, alkyl naphthalene and the like), halogenated hydrocarbons (for example, dichloromethane, chloroform, carbon tetrachloride, chlorobenzene and the like), esters (for example, ethyl acetate, butyl acetate, ethyl propionate, diisobutyl phthalate, dibutyl phthalate, dioctyl phthalate and the like), amides (for example, dimethylformamide, diethylformamide, dimethylacetamide and the like), nitriles (for example, acetonitrile and the like).

The following typical adjuvants can be exemplified. These adjuvants can be used depending on purposes and used singly or in combination of two or more kinds or need not to be used at all in some cases.

To emulsify, disperse, dissolve and/or wet a compound as an active ingredient, a surfactant is used. Examples thereof include sufactants such as polyoxyethylene alkyl ethers, polyoxyethylene alkylaryl ethers, polyoxyethylene higher fatty acid esters, polyoxyethylene resinates, polyoxyethylene sorbitan monolaurate, polyoxyethylene sorbitan mono-nooleate, alkylarylsulfonates, naphthalenesulfonates, lignin sulfonates, higher alcohol sulfate esters and the like.

Furthermore, to stabilize the dispersion of a compound as an active ingredient, adhere it and/or bind it, the following adjuvants can be used. Examples thereof include casein, gelatin, starch, methyl cellulose, carboxymethyl cellulose, gum Arabic, polyvinyl alcohols, pine oil, bran oil, bentonite, Xanthan gum, lignin sulfonates and the like.

In order to improve fluidity of a solid product, the following adjuvants can be used. Examples thereof include waxes, stearates, alkyl phosphates and the like. Adjuvants such as naphthalenesulfonic acid condensation products, polycondensates of phosphates and the like can be used as a peptizer for suspendible products. As a defoaming agent, adjuvants such as silicon oils and the like can also be used.

Incidentally, the compound represented by the general formula (1) of the present invention is stable to light, heat, oxidation and the like. However, an anti-oxidant or an ultraviolet absorber including a phenol derivative, for example, BHT (2,6-di-t-butyl-4-methylphenol) and BHA (butylated hydroxyanisole), a bisphenol derivative or arylamines such as phenyl-α-naphthylamine, phenyl-β-naphthylamine, condensates of phenetidine and acetone and the like, or a benzophenone-based compound is added as a stabilizer in a proper amount when necessary, whereby a composition with much stabilized effect can be obtained.

The amount of the active ingredient of the compound represented by the general formula (1) of the present invention is usually from 0.5 weight % to 20 weight % for dust formulation, from 5 weight % to 50 weight % for emulsifiable concentrate, from 10 weight % to 90 weight % for wettable powder, from 0.1 weight % to 20 weight % for granule, and from 10 weight % to 90 weight % for flowable formulation. On the other hand, the amount of the carrier in each formulation is usually from 60 weight % to 99 weight % for dust formulation, from 40 weight % to 95 weight % for emulsifiable concentrate, from 10 weight % to 90 weight % for wettable powder, from 80 weight % to 99 weight % for granule, and from 10 weight % to 90 weight % for flowable formulation. Meanwhile, the amount of the adjuvant is usually from 0.1 weight % to 20 weight % for dust formulation, from 1 weight % to 20 weight % for emulsifiable concentrate, from 0.1 weight % to 20 weight % for wettable powder, from 0.1 weight % to 20 weight % for granule, and from 0.1 weight % to 20 weight % for flowable formulation.

In order to control various kinds of insect pests, the compound of the present invention may be suitably applied to crops which are expected to create insect pests or places where such creation is not desired in an amount effective in controlling insect pests as intact, as appropriately diluted with water or the like, or as suspended, and used accordingly. The amount thereof is varied according to various factors such as purpose, target insect pests, reared status of crops, occurrence trend of insect pests, weather, environmental conditions, the type of formulation, method of application, place of application, time of application and the like. However, usually, the amount is at a concentration of 0.0001 ppm to 5000 ppm and preferably at a concentration of 0.01 ppm to 1000 ppm as an active ingredient. Furthermore, the application amount is generally from 1 to 300 g per 10 are as an active ingredient.

The insecticide comprising the compound represented by the general formula (1) of the present invention as an active ingredient may be used singly for preventing insect pests such as various agricultural, forest and horticultural insect pests, stored grain insect pests, hygienic insect pests, nematodes or the like which are injurious to paddy rice, fruit trees, vegetables, other crops, flowers and the like. Also, it may be used in combination of one or more kinds of other insecticides and/or fungicides in order to obtain a much higher control effect on controlling various diseases and insect pests occurring at the same time.

When the compound represented by the general formula (1) of the present invention is used in combination with one or more other insecticides and/or fungicides, the compound represented by the general formula (1) may be used as a mixed solution with other insecticides and/or fungicides, or the compound represented by the general formula (1) may be used as a mixture with other insecticides and/or fungicides at the time of application of the agrochemicals.

In addition, the compound represented by the general formula (1) can be used as a mixture with a plant protection agent such as a herbicide, a fertilizer, a soil conditioner, a plant growth regulator and the like, or a material, whereby a multi-purpose composition with an excellent effect can be prepared, or a composition which can expect an additive effect or synergistic effect can also be prepared.

EXAMPLES

The representative examples of the present invention are now more specifically illustrated below with reference to the following Examples. However, the present invention is not restricted to these Examples.

Example 1-1

Preparation of 4-heptafluoroisopropyl-2-(trifluoromethylthio)aniline

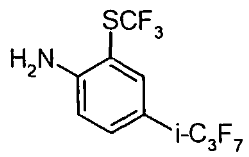

10.0 g of 2-(trifluoromethylthio)aniline, 45.8 g of 2-iodoheptafluoropropane, 10.8 g of sodium hydrosulphite, 5.2 g of sodium hydrogen carbonate and 2.1 g of tetra-n-butylammonium hydrogensulfate were added to a mixed solvent of 200 ml of t-butylmethyl ether and 200 ml of water, and the resulting mixture was strongly stirred at room temperature for 10 hours. The organic phase was separated, washed with aqueous saturated sodium hydrogen carbonate solution, and then evaporated under a reduced pressure to remove the solvent. The resulting residue was purified by silica gel column chromatography (n-hexane : ethyl acetate = 20:1) to obtain 6.5 g of the desired title product (Yield: 35%) as orange oil.
$^1$H-NMR($CDCl_3$,ppm) δ 4.80(2H, broad-s), 6.84(1H, d, J = 8.3Hz), 7.45(1H, d, J = 8.3Hz), 7.70(1H, s).
In the samemethod, the following anilines were prepared.

4-heptafluoroisopropyl-2-(pentafluoroethylthio)aniline

$^1$H-NMR($CDCl_3$,ppm) δ 4.79(2H, broad-s), 6.83(1H, d, J = 8.8Hz), 7.45(1H, dd, J =2.0Hz and 8.8Hz), 7.67(1H, d, J = 2.0Hz).
Orange oil

4-heptafluoroisopropyl-2-(heptafluoro-n-propylthio)aniline

$^1$H-NMR($CDCl_3$,ppm) δ 4.78(2H, broad-s), 6.84(1H, d, J = 8.8Hz), 7.46(1H, dd, J = 2.0Hz and 8.8Hz), 7.67(1H, d, J = 2.0Hz).
Yellow oil

4-(heptafluoro-n-propyl)-2-(trifluoromethylthio)aniline

$^1$H-NMR ($CDCl_3$, ppm) δ 4.20 (2H, broad), 6.85 (1H, d, J = 8.3Hz.), 7.44 (1H, dd, J=2.0,8.3Hz), 7. 69 (1H, d, J = 2.0Hz) .
Yellow oil

Example 1-2

Preparation of 2-bromo-4-heptafluoroisopropyl-6-(trifluoromethylthio)aniline

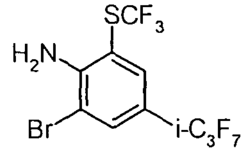

To a solution of 3.30 g of 4-heptafluoroisopropyl-2-(trifluoromethylthio)aniline added to 10 ml of N,N-dimethylformamide was introduced dropwise 1.62 g of N-bromosuccinimide dissolved in 5 ml of N,N-dimethylformamide. The

reaction solution was stirred at room temperature for 2 hours, and then ethyl acetate and water were added thereto. The organic phase was separated, washed with 50 ml of water, and then dried over anhydrous magnesium sulfate. Anhydrous magnesium sulfate was filtered off to obtain a solution and the resulting solution was concentrated under a reduced pressure. The resulting residue was purified by silica gel column chromatography (n-hexane : ethyl acetate = 15:1) to obtain 2.92 g of the desired title product (Yield: 73%) as light brown oil.
$^1$H-NMR($CDCl_3$,ppm) δ 5.29(2H, broad-s), 7.68(1H, s), 7.74(1H, d, J = 2.0Hz).

In the same method, the following anilines were prepared.

2-bromo-4-heptafluoroisopropyl-6-(pentafluoroethylthio)aniline

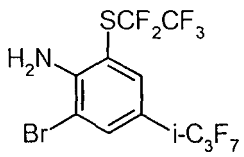

$^1$H-NMR($CDCl_3$,ppm) δ 5.28(2H, broad-s), 7.65(1H, s), 7.75(1H, d, J = 2.0Hz).
Orange oil

2-bromo-4-(heptafluoro-n-propyl)-6-(trifluoromethylthio)aniline

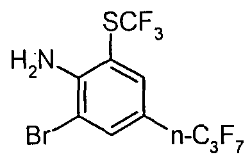

$^1$H-NMR ($CDCl_3$, ppm) δ 5.36(2H, broad-s), 7.67 (1H, s), 7.72 (1H, d, J = 2.0Hz).
Orange oil

Furthermore, using N-chlorosuccinic acid imide instead of N-bromosuccinimide, 2-chloro-4-heptafluoroisopropyl-6-(trifluoromethylthio)aniline was prepared.

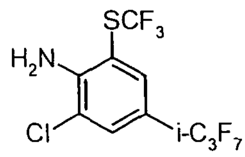

$^1$H-NMR ($CDCl_3$, ppm) δ 5.23(2H, broad-s), 7.60(1H, s), 7. 64 (1H, s).
Red oil

Example 1-3

Preparation of N-(2-bromo-4-heptafluoroisopropyl-6-trifluoromethylthio)phenyl 3-nitrobenzamide

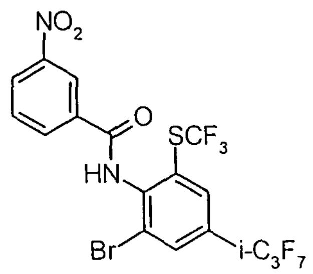

7.47 g of 3-nitrobenzoyl chloride and 7.56 g of 2-bromo-4-heptafluoroisopropyl-6-(trifluoromethylthio) aniline were added to 50 ml of pyridine, and the resulting mixture was stirred at 90°C for 10 hours. The reaction solution was poured into ice water and the pH value of the solution was adjusted to 1 by addition of 2N hydrochloric acid. Then, the reaction solution was extracted with ethyl acetate. The solvent was removed under a reduced pressure to obtain a residue. The resulting residue was added to a mixed solvent of 30 ml of tetrahydrofuran and 20 ml of water, and the resulting mixture was stirred under an ice-water bath. To the solution was added 1.2 g of sodium hydroxide, and the resultant was stirred for 1.5 hour under an ice-water bath and then stirred at room temperature for 20 hours. To the reaction solution were added ethyl acetate and water for separating the organic phase. The organic phase was washed with saturated salt water one time and dried over anhydrous magnesium sulfate, and then the solvent was removed under a reduced pressure to obtain a residue. The resulting residue was purified by silica gel column chromatography (n-hexane: ethyl acetate = 6:1) to obtain 6.85 g of the desired title product (Yield: 68%) as a white solid.
$^1$H-NMR ($CDCl_3$, ppm) δ 7 .80 (1H, t, J = 7.8Hz), 8.05 (2H, s), 8.16(1H, s), 8.30-8.34(1H, m), 8.50-8.53(1H, m), 8.82 (1H, t, J = 2.0Hz).

Example 1-4

Preparation of N-(2-bromo-4-heptafluoroisopropyl-6-trifluoromethylthio)phenyl 3-aminobenzamide

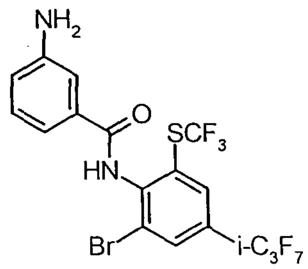

To a solution of 6.85 g of N-(2-bromo-4-heptafluoroisopropyl-6-trifluoromethylthio)phenyl 3-nitrobenzamide and 7.61 g of tin(II) chloride (anhydride) added to 50 ml of ethanol was added 5 ml of concentrated hydrochloric acid, and the resulting mixture was stirred at 60°C for 3 hours. The reaction solution was returned to room temperature, poured into ice water, and neutralized with potassium carbonate. The insoluble substance was filtered off and then the solution was extracted with ethyl acetate, and the solvent was removed under a reduced pressure. The precipitated solid was washed with n-hexane to obtain 5.13 g of the desired title product (Yield: 79%) as a white solid.
$^1$H-NMR(DMSO-$d_6$, ppm) δ 5.40(2H, broad-s), 6.78-6.81(1H, m), 7.13-7.21(3H, m), 7.99(1H, s), 8.25(1H, d, J = 2.0Hz), 10.64(1H, s).
In the same method, the following compounds were prepared.

N-(2-bromo-4-heptafluoroisopropyl-6-pentafluoroethylthio)phenyl 3-aminobenzamide

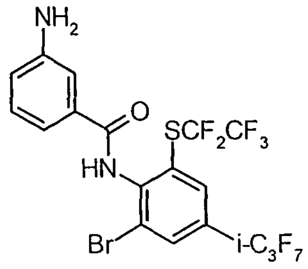

$^1$H-NMR($CDCl_3$,ppm) δ 3.91(2H, broad-s), 6.91-6.94(1H, m), 7.25-7.34(3H, m), 8.01 (2H, s), 8.06(1H, s).
White solid

N-[2-bromo-4-heptafluoroisopropyl-6-(heptafluoro-n-propylthio)]phenyl 3-aminobenzamide

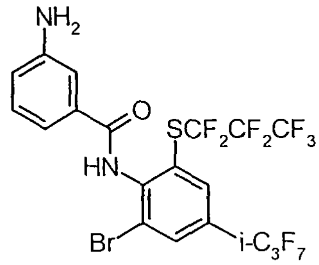

$^1$H-NMR (DMSO-$d_6$, ppm) δ 5.40 (2H, broad-s), 6.80(1H, d, J = 7.8Hz), 7.14-7.22(3H, m), 8.00(1H, s), 8.29 (1H, s), 10.67 (1H, s).
White solid

N-(2-chloro-4-heptafluoroisopropyl-6-trifluoromethylthio)phenyl 3-aminobenzamide

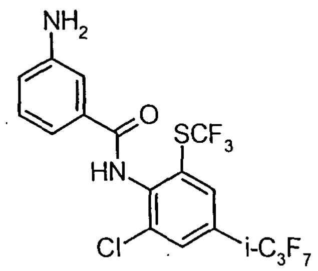

$^1$H-NMR($CDCl_3$,ppm) δ 3.91(2H, broad-s), 6.91-6.94(1H, m), 7.26-7. 32 (3H, m), 7.84 (1H, d, J = 2.4Hz), 7.97 (1H, s), 8.09(1H, s).
White solid

Example 1-5

Preparation of N-(2-bromo-4-heptafluoroisopropyl-6-trifluoromethylthio)phenyl 3-[(2-chloropyridin-3-yl)carbonylamino] benzamide (Compound No. 1-158)

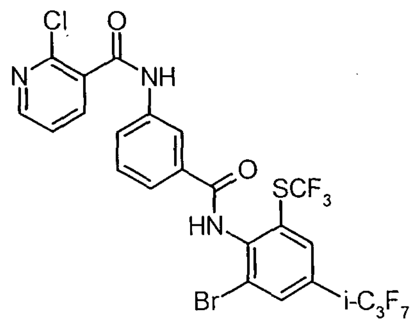

To a solution of 200 mg of N-(2-bromo-4-heptafluoroisopropyl-6-trifluoromethylthio)phenyl 3-aminobenzamide and 56 mg of pyridine added to 3 ml of tetrahydrofuran was introduced dropwise 63 mg of 2-chloronicotinoyl chloride dissolved in 1 ml of tetrahydrofuran at room temperature. The reaction solution was stirred at room temperature for 2 hours, and then ethyl acetate and water were added thereto for separating the organic phase. The organic phase was washed with 1N hydrochloric acid and aqueous saturated sodium hydrogen carbonate solution respectively one time each, and evaporated under a reduced pressure to remove the solvent. The precipitated solid was washed with n-hexane to obtain 210 mg of the desired title product (Yield: 84%) as a white solid.
$^1$H-NMR ($CDCl_3$, ppm) δ 7.42-7.46(1H, m), 7 . 60 (1H, t, J = 7.8Hz), 7.80(1H, d, J = 7.8Hz), 7.94(1H, d, J = 7.8Hz), 8.02 (1H, s), 8.03(1H, s), 8.22-8.28(2H, m), 8.33(1H, s), 8.41(1H, s), 8.55(1H, dd, J = 2.0Hz and 2.9Hz).

Example 2-1

Preparation of N-(2-bromo-4-heptafluoroisopropyl-6-trifluoromethylthio)phenyl 3-(methylamino)benzamide

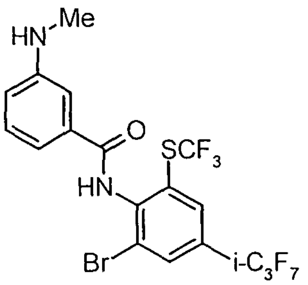

5 ml of 98% sulfuric acid was cooled to 0°C to 5°C to stir and 1.0 g of N-(2-bromo-4-heptafluoroisopropyl-6-trifluoromethylthio)phenyl 3-aminobenzamide was added thereto. The resulting mixture was stirred for 10 minutes and then 5 ml of aqueous 37% formaldehyde solution was added dropwise thereto. The resulting solution was kept at between 0°C and 5°C and stirred for 3 hours. To the reaction solution maintained in a cooled state was added 28% ammonia water for neutralization and further added ethyl acetate for separating the organic phase. The organic phase was dried over anhydrous magnesium sulfate and evaporated under a reduced pressure to remove the solvent to obtain a residue. The resulting residue was purified by silica gel column chromatography (n-hexane : ethyl acetate = 6:1) to obtain 0.74 g of the desired title product (Yield: 72%) as a white solid.
$^1$H-NMR($CDCl_3$,ppm) δ 2.91(3H, s), 4.00(1H, broad), 6. 83-6. 86 (1H, m), 7.17-7.24 (2H, m), 7.34(1H, t, J= 7.8Hz), 7.98-8.01(2H, m), 8.11(1H, s).
In the same method, the following compounds were prepared.

N-(2-bromo-4-heptafluoroisopropyl-6-pentafluoroethylthio)phenyl 3-(methylamino)benzamide

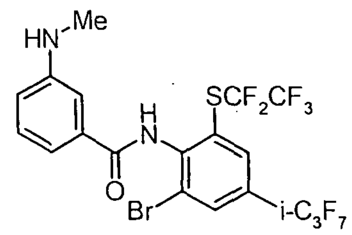

$^1$H-NMR(CDCl$_3$,ppm) δ 2.91 (3H, s), 3.99 (1H, broad), 6.83-6.86 (1H, m), 7.17-7.22 (2H, m), 7. 34 (1H, t, J = 7.8Hz), 8.01 (2H, s), 8.08 (1H, s).
White solid

N-(2-bromo-4-heptafluoroisopropyl-6-trifluoromethoxy)phenyl 3-(methylamino)benzamide

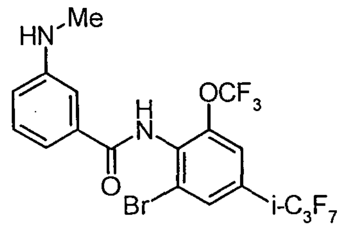

$^1$H-NMR (CDCl$_3$, ppm) δ 2. 90 (3H, s), 3. 97 (1H, broad), 6.81-6.84 (1H, m), 7.16-7.18(2H, m), 7.32 (1H, t, J = 7.8Hz), 7.56(2H, s), 7.85(1H, d, J = 2.0Hz).
White solid

Example 2-2

Preparation of N-(2-bromo-4-heptafluoroisopropyl-6-trifluoromethylthio)phenyl 3-[(N'-(2,6-difluorobenzoyl)-N'-methyl) amino]benzamide(Compound No. 2-151)

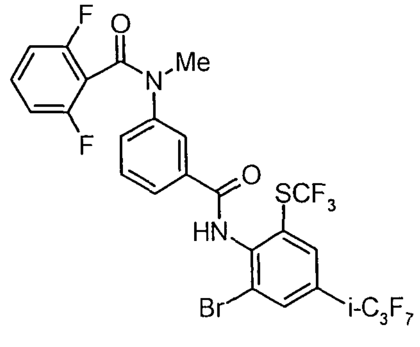

To a solution of 170 mg of N-(2-bromo-4-heptafluoroisopropyl-6-trifluoromethylthio)phenyl 3-(methylamino) benzamide and 28 mg of pyridine added to 3 ml of tetrahydrofuran was introduced dropwise 52 mg of 2,6-difluorobenzoyl chloride dissolved in 1 ml of tetrahydrofuran. The reaction solution was stirred at room temperature for 2 hours, and then ethyl acetate and water were added thereto for separating the organic phase. The organic phase was washed with 1N hydrochloric acid and aqueous saturated sodium hydrogen carbonate solution respectively one time each, and evaporated under a reduced pressure to remove the solvent. The precipitated solid was washed with n-hexane to obtain 148 mg of the desired title product (Yield: 70%) as a white solid.

$^1$H-NMR($CDCl_3$,ppm) δ 3.56(3H, s), 6.69-6.74(2H, m), 7.13-7.18(1H, m), 7.43-7.47(2H, m), 7.72(1H, s), 7.77-7.81(1H, m), 7.91(1H, s), 8.01(2H, s).

Example 3-1

Preparation of 3-(benzoylamino)benzoic acid

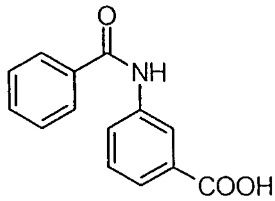

To 100 ml of water were added 5.90 g of sodium hydroxide and 10.0 g of 3-aminobenzoic acid, and the resulting mixture was stirred at room temperature and 10.3 g of benzoyl chloride was subsequently introduced dropwise thereto such that the internal temperature is maintained at 25°C to 35°C. The, the solution was stirred at room temperature for 6 hours and then 25 ml of 6N hydrochloric acid was introduced dropwise thereto. The precipitated solid was filtered off and collected, the filtered product was washed with 100 ml of water two times and dried at 50°C under a reduced pressure to obtain 13.9 g of the desired title product (Yield: 79%) as a white solid.
$^1$H-NMR($CDCl_3$,ppm) δ 7.40-7.56(5H, m), 7.78(1H, d, J=7.8Hz), 8.00(2H, d, J=8.3Hz), 8.15(1H, d, J=7.8Hz), 8.35(1H, t, J=2.0Hz), 9.89(1H, s).

Example 3-2

Preparation of 3-(N-benzoyl-N-methylamino)benzoic acid

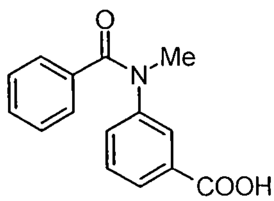

To 70 ml of acetone were added 5.0 g of 3-(benzoylamino)benzoic acid and 2.95 g of 95% potassium hydroxide (powder) and the resulting mixture was stirred at room temperature, and then 6.4 g of 98% dimethyl sulfate was introduced dropwise thereto. After completion of the introduction, the temperature of resulting mixture was elevated to a reflux condition and the resulting mixture was stired for 4 hours and cooled to room temperature. While the insoluble substance was filtered off, the solvent was removed under a reduced pressure. To the resulting residue were introduced 5 ml of aqueous 50% potassium hydroxide solution and 20 ml of ethanol, and the resultant was stirred at room temperature for 2 hours. The solvent was removed under a reduced pressure to obtain a residue. To the resulting residue were added 50 ml of ethyl acetate and 50 ml of water for separating the aqueous phase. To the aqueous phase was added concentrated hydrochloric acid to give an acidic solution. Then, 100 ml of ethyl acetate was added thereto and extracted. While the organic phase was dried over anhydrous magnesium sulfate, the solvent was removed under a reduced pressure. The precipitated solid was washed with a mixed solvent of n-hexane and ethyl acetate (3:1) and dried under a reduced pressure to obtain 3.80 g of the desired title product (Yield: 72%) as a white solid.
$^1$H-NMR(DMSO-$d_6$,ppm) δ 3.39 (3H, s), 7.21-7.31 (5H, m), 7.35-7.43(2H, m), 7.70-7.73(2H, m), 13.07(1H, broad-s).

Example 3-3

Preparation of N-(2-bromo-4-heptafluoroisopropyl-6-trifluoromethylthio)phenyl 3-[(N'-benzoyl-N'-methyl)amino]benzamide (Compound No. 2-141)

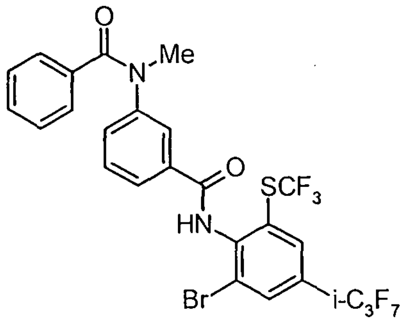

7.66 g of 3-(N-benzoyl-N-methylamino)benzoic acid, 4.28 g of thionyl chloride and 0.1 ml of N,N-dimethylformamide were added to 40 ml of toluene, and the resulting mixture was stirred at 90 °C for 2 hours. The reaction solution was concentrated under a reduced pressure to obtain light brown oil. The oil was introduced to 30 ml of pyridine with 4.40 g of 2-bromo-4-heptafluoroisopropyl-6-(trifluoromethylthio)aniline dissolved therein, and the resulting mixture was stirred at 90°C for 8 hours. The reaction solution was diluted with ethyl acetate and the organic phase was washed with 2N hydrochloric acid and saturated sodium hydrogen carbonate solution respectively one time each, and then concentrated under a reduced pressure to obtain brown oil. The oil was added to a mixed solvent of 40 ml of THF and 20 ml of water, and then 4.0 g of sodium hydroxide was introduced thereto, and the resulting mixture was stirred at room temperature for 6 hours. To the reaction solution were added ethyl acetate and water, and the organic phase was separated and then washed with water one time. While the organic phase was dried over anhydrous magnesium sulfate, the solvent was removed under a reduced pressure to obtain a residue. The resulting residue was purified by silica gel column chromatography (n-hexane : ethyl acetate 9:1 --> 2:1) to obtain 4.06 g of the desired title product (Yield: 60%) as a white solid.
$^1$H-NMR ($CDCl_3$,ppm) $\delta$ 3.55(3H, s), 7.18-7.22(2H, m), 7.27-7.35(4H, m), 7.42(1H, t, J = 7.8Hz), 7.62(1H, s), 7.73(1H, d, J = 7.8Hz), 7.97(1H, s), 7.99(1H, s), 8.10(1H, s) .

Example 4

Preparation of N-(2-bromo-4-heptafluoroisopropyl-6-trifluoromethylsulfinyl)phenyl 3-[(N'-benzoyl-N'-methyl)amino]benzamide (Compound No. 2-176)

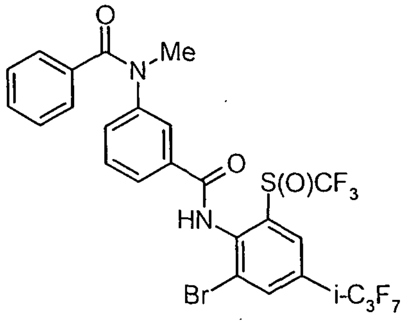

To 12 ml of dichloromethane was added 0.50 g of N-(2-bromo-4-heptafluoroisopropyl-6-trifluoromethylthio) phenyl 3-[(N'-benzoyl-N'=methyl)amino]benzamide, and the resulting mixture was stirred at room temperature. Then,

0.26 g of m-chloroperbenzoic acid was introduced thereto and stirred at room temperature for 15 hours. To the reaction solution was added aqueous sodium thiosulfate solution, the remaining peroxide was disappeared, and then the organic phase was separated. The organic phase was washed with water one time, dried over anhydrous magnesium sulfate, and the solvent was removed under a reduced pressure to obtain a residue. The resulting residue was purified by silica gel column chromatography (n-hexane : ethyl acetate = 7:3) to obtain 0.27 g of the desired title product (Yield: 53%) as a white amorphous substance.
$^1$H-NMR($CDCl_3$,ppm) 5 3.57(3H, s), 7.20-7.33(5H, m), 7.40(1H, d, J = 7.8Hz), 7.47(1H, t, J = 7.8Hz), 7.57(1H, s), 7.71 (1H, d, J = 7.8Hz), 8.03-8.05 (1H, m), 8.11 (1H, s), 8.26 (1H, s).

Example 5

Preparation of N-(2-bromo-4-heptafluoroisopropyl-6-trifluoromethylsulfonyl)phenyl 3-[(N'-benzoyl-N'-methyl)amino] benzamide (Compound No. 2-191)

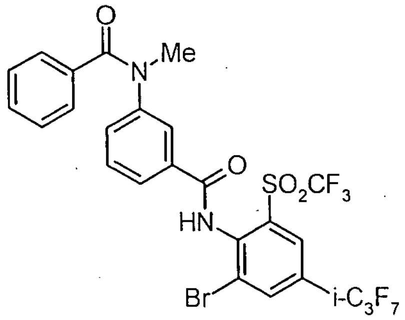

To a mixed solution of 2 ml of dichloromethane, 2 ml of acetonitrile and 4 ml of water were added 130 mg of N-(2-bromo-4-heptafluoroisopropyl-6-trifluoromethylthio)phenyl 3-[(N'-benzoyl-N'-methyl)amino]benzamide and 120 mg of sodium periodate, and the resulting mixture was stirred at room temperature. Then, 10 mg of ruthenium (III) chloride was added thereto and stirred at room temperature for 5 hours. To the reaction solution was added aqueous sodium thiosulfate solution, the remaining peroxide was disappeared, and then 30 ml of ethyl acetate was introduced for separating the organic phase. The organic phase was dried over anhydrous magnesium sulfate and then the solvent was removed under a reduced pressure to obtain a residue. The resulting residue was purified by silica gel column chromatography (n-hexane : ethyl acetate = 5:3) to obtain 82 mg of the desired title product (Yield: 60%) as a white amorphous substance.
$^1$H-NMR($CDCl_3$,ppm) δ 3.57(3H, s), 7.18-7.36(6H, m), 7.43(1H, t, J = 7.8Hz), 7.61(1H, d, J = 2.0Hz), 7.68(1H, d, J = 7.8Hz), 8.28 (1H, d, J=2.0Hz), 8.32 (1H, d, J=2.0Hz), 8.76(1H, s).

Example 6-1

Preparation of N-(2-bromo-4-heptafluoroisopropyl-6-trifluoromethylthio)phenyl 3-(benzoylamino)benzamide

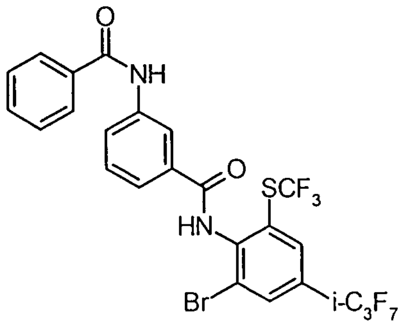

To a solution of 300 mg of N-(2-bromo-4-heptafluoroisopropyl-6-trifluoromethylthio)phenyl 3-aminobenzamide prepared in Example 1-4 and 85 mg of pyridine added to 3 ml of tetrahydrofuran was introduced dropwise 75 mg of benzoyl chloride dissolved in 1 ml of tetrahydrofuran at room temperature. The resulting mixture was stirred at room temperature for 2 hours, and then ethyl acetate and water were added thereto for separating the organic phase. The organic phase was washed with 1N hydrochloric acid and saturated sodium hydrogen carbonate solution respectively one time each, and the solvent was removed under a reduced pressure to precipitate a solid. The resulting solid was washed with n-hexane to obtain 345 mg of the desired title product (Yield: 97%) as a white solid.
$^1$H-NMR (DMSO-$d_6$,ppm) δ 7.53-7.64(4H, m), 7.81(1H, d, J = 7.8Hz), 8.00-8.05(3H, m), 8.11 (1H, d, J = 7. 8Hz), 8.31 (1H, d, J = 1.5Hz), 8.41(1H, s), 10.52(1H, s), 10.93(1H, s).

Example 6-2

Preparation of N-(2-bromo-4-heptafluoroisopropyl-6-trifluoromethylsulfonyl)phenyl 3-(benzoylamino)benzamide(Compound No. 1-191)

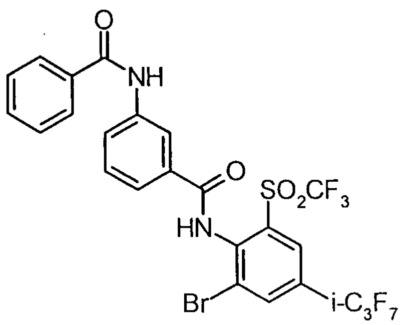

0.34 g of N-(2-bromo-4-heptafluoroisopropyl-6-trifluoromethylthio)phenyl 3-(benzoylamino)benzamide and 0.33 g of sodium periodate were added to a mixed solution of 2.5 ml of dichloromethane, 2.5 ml of acetonitrile and 5 ml of water, and the resulting mixture was stirred at room temperature. Subsequently, 10 mg of ruthenium (III) chloride was added thereto and stirred at room temperature for 7 hours. To the reaction solution was added sodium bisulfite and peroxide was dissolved thereon, and then ethyl acetate was added thereto for separating the organic phase. The organic phase was dried over anhydrous magnesium sulfate and the solvent was removed under a reduced pressure to obtain a residue. The resulting residue was purified by silica gel column chromatography (n-hexane : ethyl acetate = 2:1) to obtain 0.23 g of the desired title product (Yield: 65%) as a white solid.
$^1$H-NMR(DMSO-$d_6$,ppm) δ 7.53-7.63(4H, m), 7.74(1H, d, J = 7.8Hz), 7.98-8.01(2H, m), 8.08(1H, d, J= 8.3Hz), 8.24(1H, s), 8.38 (1H, t, J = 1.5Hz), 8.81 (1H, s), 10.51 (1H, s), 10.82(1H, s).

Example 7

Preparation of 4-heptafluoroisopropyl-2-methyl-6-(trifluoromethylthio)aniline

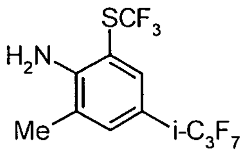

0.92 g of 2-bromo-4-heptafluoroisopropyl-6-(trifluoromethylthio)aniline, 0.13 g of trimethylboroxin and 1.44 g of potassium carbonate were added to a mixed solvent of 10 ml of toluene, 5 ml of ethanol and 5 ml of water, and the resulting mixture was stirred in a nitrogen atmosphere at room temperature. Subsequently, while 0.2 g of tetrakis(triphenylphosphine)palladium (0) was introduced thereto, the resulting solution was elevated to 80°C and stirred for 8 hours. The solution was returned to room temperature, the insoluble substance was filtered off, and then ethyl acetate and water were added thereto for separating the organic phase. The organic phase was dried over anhydrous magnesium sulfate and the solvent was removed under a reduced pressure to obtain a residue. The resulting residue was purified by silica gel column chromatography (n-hexane : ethyl acetate = 20:1) to obtain 0.31 g of the desired title product (Yield: 40%) as light yellow oil.
$^1$H-NMR($CDCl_3$,ppm) δ 2.24 (3H, s), 4.77 (2H, broad-s), 7.34 (1H, s), 7.60 (1H, s).

Example 8-1

Preparation of N-(2-chloro-4-heptafluoroisopropyl-6-trifluoromethoxy)phenyl 3-nitrobenzamide

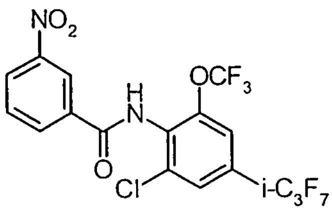

1.17 g of 3-nitrobenzoyl chloride and 1.20 g of (2-chloro-4-heptafluoroisopropyl-6-trifluoromethoxy)aniline were added to 8 ml of pyridine, and the resulting mixture was stirred at 90°C for 6 hours. To the reaction solution were added ethyl acetate and 1N hydrochloric acid, the organic phase was separated in a state that the aqueous phase became acidic, and then the organic phase was washed with saturated sodium hydrogen carbonate solution two times. The organic phase was dried over anhydrous magnesium sulfate and the solvent was removed under a reduced pressure to obtain a residue. The resulting residue was added to a mixed solution of 10 ml of tetrahydrofuran and 5 ml of water. To the solution was added 0.32 g of sodium hydroxide, and the resulting mixture was stirred at room temperature for 1 day. To the reaction solution were added ethyl acetate and water for separating the organic phase, and then the solvent was removed under a reduced pressure to obtain a residue. The resulting residue was purified by silica gel column chromatography (n-hexane : ethyl acetate = 4:1) to obtain 1.20 g of the desired title product (Yield: 72%) as a white solid.
$^1$H-NMR($CDCl_3$,ppm) δ 7.56(1H, s), 7.71-7.79(3H, m), 8.30(1H, d, J = 7.8Hz), 8.48-8.51(1H, m), 8.77(1H, t, J = 2.0Hz).

Example 8-2

Preparation of N-(2-chloro-4-heptafluoroisopropyl-6-trifluoromethoxy)phenyl 3-aminobenzamide

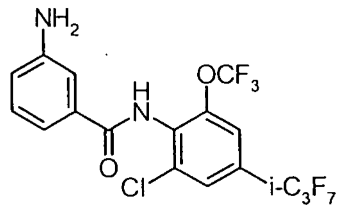

Using N-(2-chloro-4-heptafluoroisopropyl-6-trifluoromethoxy)phenyl 3-nitrobenzamide as a starting raw material, the desired title product was prepared according to the conditions as described in Example 1-4. White solid. Yield: 86%.

$^1$H-NMR($CDCl_3$,ppm) δ 3. 39 (2H, broad-s), 6. 89-6. 92 (1H, m), 7.22-7.32(3H, m), 7.52-7.53(2H, m), 7.69(1H, d, J = 1.5Hz).

Example 8-3

Preparation of N-(2-chloro-4-heptafluoroisopropyl-6-trifluoromethoxy)phenyl 3-(benzoylamino)benzamide (Compound No. 1-241)

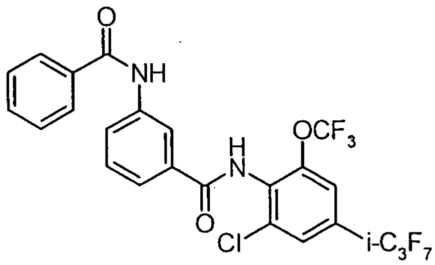

Using N-(2-chloro-4-heptafluoroisopropyl-6-trifluoromethoxy)phenyl 3-aminobenzamide as a starting raw material, the desired title product was prepared according to the conditions as described in Example 1-5. White solid. Yield: 80%.

$^1$H-NMR ($CDCl_3$,ppm) δ 7.36-7.43(3H, m), 7.47-7.53 (2H, m), 7.63-7.65(2H, m), 7.81-7.87(3H, m), 8.20(1H, s), 8.28(1H, s), 8.39 (1H, s).

Example 9-1

Preparation of 2-(2,2,2-trifluoroethoxy)nitrobenzene

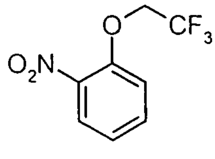

A solution of 1.70 g of 60% sodium hydride added to 20 ml of N,N-dimethylformamide was stirred at 5°C, and 4.25 g of 2,2,2-trifluoroethanol dissolved in 5 ml of N,N-dimethylformamide was introduced dropwise thereinto. The resulting solution was returned to room temperature and stirred for 1 hour, and then 5.0 g of 2-fluoronitrobenzene dissolved in 5 ml of N,N-dimethylformamide was introduced dropwise thereinto. Then, the reaction solution was stirred at room temperature for 2 hours and diluted with ethyl acetate, and then water was introduced thereinto for separating the organic phase. The organic phase was dried over anhydrous magnesium sulfate, and the solvent was removed

under a reduced pressure to obtain a residue. The resulting residue was purified by silica gel column chromatography (n-hexane : ethyl acetate = 6: 1) to obtain 8.14 g of the desired title product (Yield: 90%) as yellow oil.
$^{1}$H-NMR($CDCl_3$,ppm) δ 4.50(2H, q, J = 7.8Hz), 7.13-7.22(2H, m), 7.57-7.62 (1H, m), 7.88 (1H, dd, J = 2.0,8.3Hz).

Example 9-2

Preparation of 2-(2,2,2-trifluoroethoxy)aniline

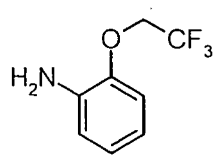

3.50 g of 2-(2,2,2-trifluoroethoxy)nitrobenzene and 0.15 g of 10% palladium carbon were added to 25 ml of methanol, and the resulting mixture was stirred under an atmospheric pressure in a hydrogen atmosphere at room temperature for 3 hours. The insoluble substance was filtered off and then the filtrate was concentrated under a reduced pressure to obtain 2.86 g of the desired title product (Yield: 95%) as yellow oil.
$^{1}$H-NMR($CDCl_3$,ppm) δ 3.83(2H, broad-s), 7.34(2H, q, J = 8.3Hz), 6.68-6.78(3H, m), 6.85-6.90(1H, m).

Example 9-3

Preparation of 4-heptafluoroisopropyl-2-(2,2,2-trifluoroethoxy)aniline

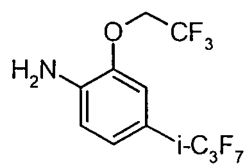

2. 85 g of 2-(2,2,2-trifluoroethoxy)aniline, 6.62 g of 2-iodoheptafluoropropane, 3.11 g of sodium hydrosulphite, 1.50 g of sodium hydrogen carbonate and 0.61 g of tetra-n-butylammonium hydrogensulfate were added to a mixed solvent of 30 ml of t-butylmethyl ether and 30 ml of water, and the resulting mixture was strongly stirred at room temperature for 12 hours. The organic phase was separated and washed with saturated- sodium hydrogen carbonate solution, and then evaporated under a reduced pressure to remove the solvent. The resulting residue was purified by silica gel column chromatography (n-hexane : ethyl acetate = 9:1) to obtain 13.99 g of the desired title product (Yield: 74%) as light yellow oil.
$^{1}$H-NMR ($CDCl_3$, ppm) δ 4.14 (2H, broad-s), 4.39 (2H, q, J = 7.8Hz), 6.79(1H, d, J = 8.3Hz), 6.95(1H, s), 7.11(1H, d, J = 8.3Hz).

Example 9-4

Preparation of 2-bromo-4-heptafluoroisopropyl-6-(2,2,2-trifluoroethoxy)aniline

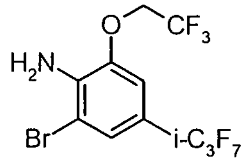

Using 4-heptafluoroisopropyl-2-(2,2,2-trifluoroethoxy)aniline as a starting raw material, the desired title product was prepared according to the conditions as described in Example 1-2. Brown oil. Yield: 88%.
$^1$H-NMR($CDCl_3$,ppm) δ 4.41(2H, q, J = 7.8Hz), 4.58(2H, broad-s), 6.90(1H, s), 7.39(1H, s).

Example 9-5

Preparation of N-[2-bromo-4-heptafluoroisopropyl-6-(2,2,2-trifluoroethoxy)]phenyl 3-[(N'-benzoyl-N'-methyl)amino]benzamide (Compound No. 2-269)

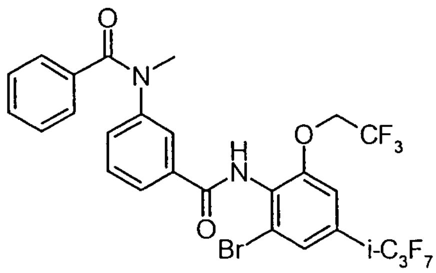

Using 2-bromo-4-heptafluoroisopropyl-6-(2,2,2-trifluoroethoxy)aniline as a starting raw material, the desired title product was prepared according to the conditions as described in Example 3-3. White amorphous substance. Yield: 69%.
$^1$H-NMR($CDCl_3$,ppm) δ 3.54(3H, s), 4.42(2H, q, J = 7.8Hz), 7.14-7.21(3H, m), 7.24-7.31(4H, m), 7.37(1H, t, J = 7.8Hz), 7.46(1H, s), 7.60-7.62(2H, m), 7.66-7.69(1H, m).

Example 10-1

Preparation of 4-[1-hydroxy-2,2,2-trifluoro-1-(trifluoromethyl)ethyl]-2-(trifluoromethylthio)aniline

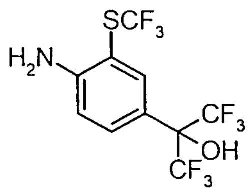

While 5.0 g of 2-trifluoromethylthio aniline and 6.5 g of hexafluoroacetone hydrate were mixed at room temperature, 0.1 g of p-toluenesulfonic acid monohydrate was added thereto, and the reaction solution was stirred at 100 °C for 20 hours. After disappearance of the starting raw material was confirmed by means of TLC, to the reaction solution were added ethyl acetate and saturated sodium hydrogen carbonate solution for solution separation and extraction. To the organic phase was added anhydrous magnesium sulfate, the organic phase was dried and filtered off. The filtrate was concentrated under a reduced pressure to obtain a residue. The resulting residue was purified by silica gel column chromatography (n-hexane : ethyl acetate = 4:1) to obtain 4.74 g of the desired title product (Yield: 51%) as dark brown oil.

$^1H$-NMR($CDCl_3$,ppm) δ 4.71(2H, broad), 6.81(1H, d, J = 8.8Hz), 7.58(1H, d, J = 8.8Hz), 7.84(1H, d, J = 1.5Hz).

Example 10-2

Preparation of 2-bromo-4-[1-hydroxy-2,2,2-trifluoro-1-(trifluoromethyl)ethyl]-6-(trifluoromethylthio) aniline

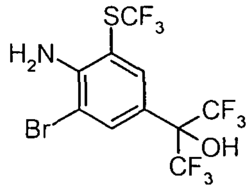

Using 4-[1-hydroxy-2,2,2-trifluoro-1-(trifluoromethyl)ethyl]-2- (trifluoromethylthio) aniline as a starting raw material-, the desired title product was prepared according to the conditions as described in Example 1-2. Red oil. Yield: 81%.

Example 10-3

Preparation of N-[2-bromo-4-{1-hydroxy-2,2,2-trifluoro-1-(trifluoromethyl)ethyl}-6-(trifluoromethylthio) phenyl] 3-nitrobenzamide

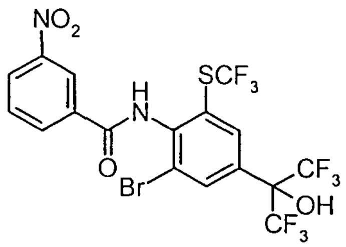

Using 2-bromo-4-[1-hydroxy-2,2,2-trifluoro-1-(trifluoromethyl)ethyl]-6-(trifluoromethylthio) aniline as a starting raw material, the desired title product was prepared according to the conditions as described in Example 1-3. White solid. Yield: 70%.

Example 10-4

Preparation of' N-[2-bromo-4-{1-hdyroxy-2,2,2-trifluoro-1-(trifluoromethyl)ethyl}-6-(trifluoromethylthio) phenyl] 3-aminobenzamide

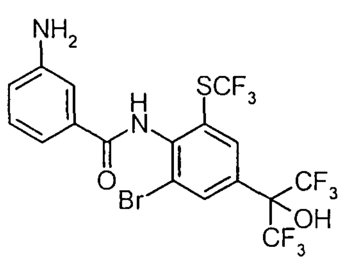

Using N-[2-bromo-4-{1-hydroxy-2,2,2-trifluoro-1-(trifluoromethyl)ethyl}-6-(trifluoromethylthio) phenyl] 3-nitrobenzamide as a starting raw material, the desired title product was prepared according to the conditions as described in Example 1-4. White solid. Yield: 90%.

Example 10-5

Preparation of N-[2-bromo-4-{1-hydroxy-2,2,2-trifluoro-1-(trifluoromethyl)ethyl}-6-(trifluoromethylthio) phenyl] 3-[(2-fluorobenzoyl)amino]benzamide

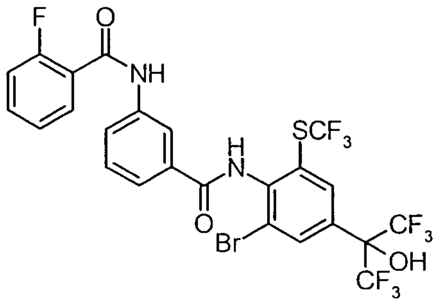

Using N-[2-bromo-4-{1-hydroxy-2,2,2-trifluoro-1-(trifluoromethyl)ethyl}-6-(trifluoromethylthio) phenyl] 3-aminobenzamide and 2-fluorobenzoyl chloride as starting raw materials, the desired title product was prepared according to the conditions as described in Example 1-5. White solid. Yield: 84%.

Example 10-6

Preparation of N-[2-bromo-4-{1-chloro-2,2,2-trifluoro-1-(trifluoromethyl)ethyl}-6-(trifluoromethylthio) phenyl] 3-[(2-fluorobenzoyl)amino]benzamide

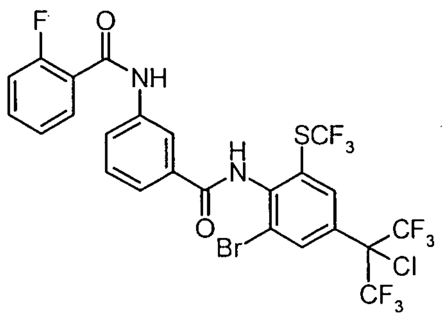

At room temperature, 1.0g of N-[2-bromo-4-{1-hydroxy-2,2,2-trifluoro-1-(trifluoromethyl)ethyl}-6-(trifluoromethylthio) phenyl] 3-[(2-fluorobenzoyl)amino]benzamide and 0.2 g of pyridine were introduced to 10 ml of thionyl chloride. Then, the temparature of resulting solution was elevated and the resulting solution was stirred under a reflux condition. After disappearance of the raw material was confirmed by means of TLC, the reaction solution was cooled and then concentrated under a reduced pressure. The resulting residue was purified by silica gel chromatography (n-hexane : ethyl acetate = 3:1) to obtain 0.77 g of the desired title product (Yield: 75%) as a white solid.

Example10-7

Preparation of N-(2-bromo-4-heptafluoroisopropyl-6-trifluoromethylthio)phenyl 3-[(2-fluorobenzoyl)amino]benzamide (Compound No. 1-143)

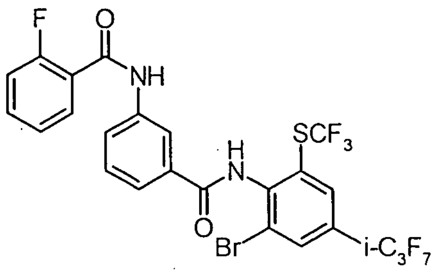

At room temperature, 400 mg of N-[2-bromo-4-{1-chloro-2,2,2-trifluoro-1-(trifluoromethyl)ethyl}-6-(trifluoromethylthio) phenyl] 3-[(2-fluorobenzoyl)amino]benzamide and 166 mg of potassium fluoride were introduced to 10 ml of N,N-dimethylformamide. Then, the resulting solution was elevated to 120°C and stirred for 5 hours. The reaction solution was cooled to room temperature, and then ethyl acetate and water were added thereto for separating the organic phase. To the organic phase was added anhydrous magnesium sulfate, and the organic phase was dried and filtered off. The filtrate was concentrated under a reduced pressure. To the resulting residue was added diisopropyl ether for washing. The filtered product obtained by filtering a suspension was vacuum-dried at room temperature to obtain 281 mg of the desired title product (Yield: 72%).
$^1$H-NMR(DMSO-$d_6$,ppm) δ 7.33-7.90 (2H, m), 7.57-7.62(2H, m), 7.68-7.73(1H, m), 7.81(1H, d, J = 7.8Hz), 8.01(1H, d, J= 7.8Hz), 8.04(1H, s), 8.30(1H, d, J= 2.0Hz), 8.37(1H, s), 10.70(1H, s), 10.95(1H, s).

Example 10-8

Preparation of N-(2-bromo-4-heptafluoroisopropyl-6-trifluoromethylthio)phenyl 3-[(2-fluorobenzoyl)amino]benzamide (Compound No. 1-143)

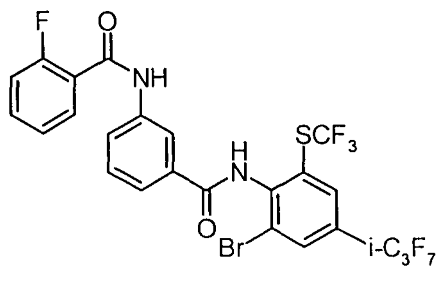

At room temperature, 300 mg of N-[2-bromo-4-{1-hydroxy-2,2,2-trifluoro-1-(trifluoromethyl)ethyl}-6-(trifluoromethylthio) phenyl] 3-[(2-fluorobenzoyl)amino]benzamide was introduced to 20 ml of methylene chloride. Subsequently, 470 mg of 2,2-difluoro-1,3-dimethyl-2-imidazolidinone were added dropwise thereto and stirred at room temperature for 8 hours. To the reaction solution was added water for separating the organic phase. To the organic phase was added anhydrous magnesium sulfate, and the organic phase was dried and filtered off. The resulting filtrate was evaporated to be dried to obtain a solid. The resulting solid was pulverized to obtain 181 mg of the desired product (Yield: 60%) in the form of powder.

The physical properties were described in Example 10-7.

Example 11-1

Preparation of 2-trifluoromethoxy-4-[1-hydroxy-2,2,2-trifluoro-1-(trifluoromethyl)ethyl]aniline

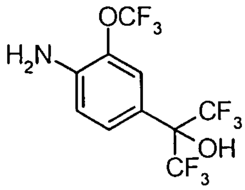

While 3.38 g of 2-trifluoromethoxyaniline and 4.75 g of hexafluoroacetone hydrate were mixed at room temperature, 0.1 g of p-toluenesulfonic acid monohydrate was added thereto, and the reaction solution was stirred at 100°C for 20 hours. After disappearance of the starting raw material was confirmed by means of TLC, to the reaction solution were added ethyl acetate and saturated sodium hydrogen carbonate solution for solution separation and extraction. To the organic phase was added anhydrous magnesium sulfate, the organic phase was dried and filtered off. The filtrate was concentrated under a reduced pressure to obtain a residue. The resulting residue was purified by silica gel column chromatography (n-hexane : ethyl acetate = 4:1) to obtain 3.60 g of the desired title product (Yield: 55%) as dark brown oil. $^{1}$H-NMR ($CDCl_3$,ppm) δ 3.37 (1H, broad-s), 4.10(2H, broad-s), 6.83(1H, d, J = 8.8Hz), 7.39(1H, d, J = 8.8Hz), 7.50(1H, s).

Example 11-2

Preparation of 2-bromo-4-[1-hydroxy-2,2,2-trifluoro-1-(trifluoromethyl)ethyl]-6-trifluoromethoxyaniline

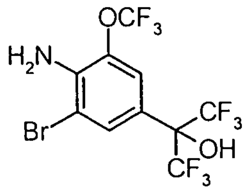

Using 2-trifluoromethoxy-4-[1-hdyroxy-2,2,2-trifluoro-1-(trifluoromethyl) ethyl] aniline as a starting raw material, the desired title product was prepared according to the conditions as described in Example 1-2. Red oil. Yield: 92%. $^{1}$H-NMR($CDCl_3$,ppm) δ 3.98(1H, t, J = 2.4Hz), 4.55(2H, broad-s), 7.47(1H, s),-7.71(1H, d, J = 1.5Hz).

Example 11-3

Preparation of N-(2-bromo-4-heptafluoroisopropyl-6-trifluoromethoxy) phenyl 3-(benzoylamino)benzamide (Compound No. 1-243)

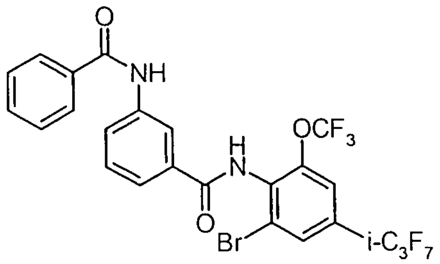

Using 2-bromo-4-[1-hydroxy-2,2,2-trifluoro-1-(trifluoromethyl)ethyl]-6-trifluoromethoxyaniline as a starting raw material, the desired title product was prepared according to the conditions as described in Examples 10-3 to 10-7. White solid.

$^1$H-NMR(DMSO-$d_6$,ppm) δ 7.48-7. 67 (4H, m), 7.73-7.81(2H, m), 7.94-8.14 (4H, m), 8.38 (1H, s), 10.51 (1H, s), 10.63 (1H, s).

Example 12-1

Preparation of N-(2-bromo-4-heptafluoroisopropyl-6-trifluoromethylsulfinyl)phenyl 3-nitrobenzamide

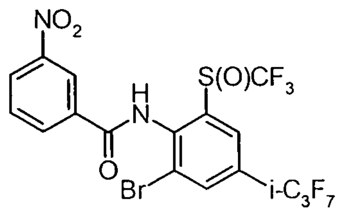

9.56 g of N-[2-bromo-4-heptafluoroisopropyl-6-(trifluoromethyl)thio]phenyl 3-nitrobenzamide prepared in Example 1-3 and 2.80 g of m-chloroperbenzoic acid were added to 100 ml of dichloromethane, and the resulting mixture was stirred at room temperature overnight. To the reaction solution was added 2.70 g of metachloroperbenzoic acid and the resulting mixture was further stirred at room temperature for 96 hours. To the reaction solution was added aqueous saturated sodium thiosulfate solution for separating the organic phase. The organic phase was washed with water and then dried over anhydrous magnesium sulfate. The solvent was removed under a reduced pressure. The resulting residue was purified by silica gel column chromatography (n-hexane : ethyl acetate = 10:1) to obtain 5.80 g of the desired product (Yield: 59%) as a white solid.

$^1$H-NMR ($CDCl_3$, ppm) δ 7.80(1H, t, J = 7.8Hz), 8.18(1H, d, J = 2.0Hz), 8.24(1H, s), 8.30(1H, dd, J = 2.0,7.8Hz), 8.46 (1H, s), 8.53(1H, dd, J = 2.0,7.8Hz), 8.80(1H, t, J = 2.0Hz).

Example 12-2

Preparation of N-[2-bromo-4-heptafluoroisopropyl-6-(trifluoromethyl)sulfinyl]phenyl 3-aminobenzamide

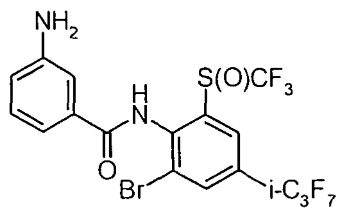

1.02 g of N-[2-bromo-4-heptafluoroisopropyl-6-(trifluoromethyl)sulfinyl]phenyl 3-nitrobenzamide and 0.99 g of tin(II) chloride (anhydride) were added to 10 ml of ethanol, and 1 ml of concentrated hydrochloric acid was subsequently introduced dropwise thereto. After the introduction, the resulting solution was elevated to 60°C and stirred for 4 hours. The reaction solution was neutralized with sodium hydroxide under ice-cooling. The resulting precipitated insoluble substance was filtered off through celite. The filtered product on celite was washed with ethyl acetate. The organic phase of the filtrate was washed with aqueous 20% sodium hydroxide solution and saturated salt water, and then dried over anhydrous magnesium sulfate. The solvent was removed under a reduced pressure to obtain a residue. The resulting residue was purified by silica gel column chromatography (n-hexane : ethyl acetate = 3:1) to obtain 0.83g of the desired product (Yield: 85%) as a white solid.
$^1$H-NMR($CDCl_3$,ppm) δ 3. 95 (2H, broad), 6. 92 (1H, dd, J = 2.0, 7.3Hz), 7.23 (1H, s), 7.24 (1H, d, J = 7.3Hz), 7.31 (1H, t, J= 7.3Hz), 8.11 (1H, d, J= 7.3Hz), 8.17 (1H, s), 8.30 (1H, s) .

Example 12-3

Preparation of N-[2-bromo-4-heptafluoroisopropyl-6-(trifluoromethyl)sulfinyl]phenyl 3-(methylamino)benzamide

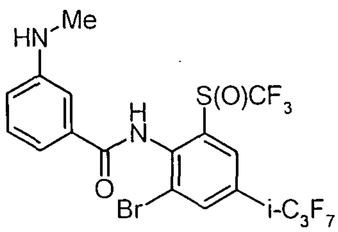

To 10 ml of concentrated sulfuric acid was added 1.96 g of N-[2-bromo-4-heptafluoroisopropyl-6-(trifluoromethyl) sulfinyl]phenyl 3-aminobenzamide and the resulting mixture was stirred. Subsequently, 5.6 ml of aqueous 37% formaldehyde solution was added dropwise thereto while the solution temperature was maintained at 30 to 40°, and the resulting solution was stirred at room temperature for 8 hours. The reaction solution was poured into ice water and extracted with ethyl acetate. The organic phase was washed with aqueous 20% sodium hydroxide solution and water, and then dried over anhydrous magnesium sulfate. The solvent was removed under a reduced pressure to obtain a residue. The resulting residue was washed with diisopropyl ether to obtain 1.30 g of the desired product (Yield: 65%) as a white solid.
$^1$H-NMR(DMSO-$d_6$,ppm) δ 2.80(3H, s), 4.99(1H, broad), 7.07-7.08(1H, m), 7.28(1H, d, J = 7.3Hz), 7.48-7.49(1H, m), 7.51(1H, s), 8.13(1H, d, J = 2.0Hz), 8.24(1H, s), 10.12(1H, broad).

Example 12-4

Preparation of N-[2-bromo-4-heptafluoroisopropyl-6-(trifluoromethyl)sulfinyl]phenyl 3-{[N'-(2-chloropyridin-3-yl)carbonyl-N'-methyl]amino}benzamide (Compound No. 2-180)

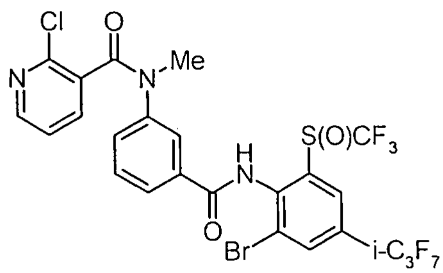

Using N-[2-bromo-4-heptafluoroisopropyl-6-(trifluoromethyl)sulfinyl]phenyl 3-(methylamino)benzamide as a

starting raw material and 2-chloronicotinoyl chloride, the desired title product was prepared according to the conditions as described in Example 1-5. White amorphous substance. Yield: 70%.
$^1$H-NMR($CDCl_3$,ppm) δ 3.58 (3H, s), 7.15 (1H, dd, J = 4.9,7.3Hz), 7.42-7.45 (2H, m), 7.60 (1H, d, J = 7.3Hz), 7.71-7.72 (2H, m), 8.13 (1H, s), 8.19-8.29 (3H, m).

Example 12-5

Preparation of N-[2-bromo-4-heptafluoroisopropyl-6-(trifluoromethyl)sulfinyl]phenyl 3-{[N'-(pyrimidin-5-yl)carbonyl-N'-methyl]amino}benzamide (Compound No. 2-281)

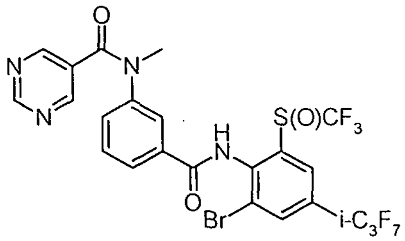

Using N-[2-bromo-4-heptafluoroisopropyl-6-(trifluoromethyl)sulfinyl]phenyl 3-aminobenzamide prepared in Example 12-3 as a starting raw material and pyrimidin-5-carboxyl chloride, the desired title product was prepared according to the conditions as described in Example 1-5. Light yellow solid. Yield: 53%.
$^1$H-NMR($CDCl_3$,ppm) δ 3.59 (3H, s), 7.34 (1H, d, J =8.3Hz), 7.51 (1H, t, J =7.8Hz), 7.78 (2H, dd, J=3.9, 1.5Hz), 8.14 (1H, d, J =2.0Hz), 8.24 (1H, s), 8.35 (1H, s), 8.67 (2H, s), 9.11 (1H, s).

Next, formulation examples including the compound represented by the general formula (1) of the present invention as an active ingredient will be illustrated. However, the present invention is not restricted to these formulation examples. Incidentally, in the formulation examples, the term "part(s)"means "part(s) by weight".

Formulation Example 1

20 parts of the compound represented by the general formula (1) of the present invention, 10 parts of Sorpol 355S (a surface active agent, a product of TOHO Chemical Industry, Co., Ltd.) and 70 parts of xylene were uniformly stirred and mixed to obtain an emulsifiable formulation.

Formulation Example 2

10 parts of the compound represented by the general formula (1) of the present invention, 2 parts of sodium alkylnaphthalene sulfonate, 1 part of sodium lignin sulfonate, 5 parts of white carbon and 82 parts of diatomaceous earth were uniformly stirred and mixed to obtain a wettable powder.

Formulation Example 3

0.3 part of the compound represented by the general formula (1) of the present invention and 0.3 part of white carbon were uniformly mixed, and 99.2 parts of clay and 0.2 part of Driless A (a product of Sankyo Co., Ltd.) were added thereto. The resulting mixture was uniformly pulverized and mixed to obtain a dust formulation.

Formulation Example 4

2 parts of the compound represented by the general formula (1) of the present invention, 2 parts of white carbon, 2 parts of sodium lignin sulfonate and 94 parts of bentonite were uniformly pulverized and mixed, and then water was added thereto. The resulting mixture was kneaded, granulated and dried to obtain a granule.

Formulation Example 5

20 parts of the compound represented by the general formula (1) of the present invention and 5 parts of 20%

aqueous solution of polyvinyl alcohol were fully stirred and mixed, and then 75 parts of 0.8% aqueous solution of Xanthan gum was added thereto. The resulting mixture was stirred and mixed again to obtain a flowable formulation.

Furthermore, to make sure that the compound represented by the general formula (1) of the present invention has an excellent insecticidal activity, the following test examples are illustrated. However, the present invention is not restricted to these test examples.

Test Example 1

Insecticidal Test on Common Cutworm (Spodoptera litura)

A piece of cabbage leaf was immersed for 30 seconds in a liquid chemical prepared by diluting a test compound to a prescribed concentration. After air-drying, the piece was put into a 7-cm polyethylene cup and second-instar larvae of common cutworms were released thereinto. The polyethylene cups were set in an isothermal chamber thermostated at 25°C. From the release 6 days later, the dead and alive were counted. The test was carried out with two replications of 5 insects per a plot.

As a result of the above test, at a treatment concentration of 100 ppm, the following compounds showed 70% mortality or more: Compound Nos. 1-1, 1-8, 1-94, 1-143, 1-145, 1-146, 1-148, 1-149, 1-150, 1-151, 1-152, 1-158, 1-166, 1-176, 1-180, 1-191, 1-195, 1-201, 1-202, 1-203, 1-204, 1-205, 1-207, 1-241, 1-242, 1-243, 1-244, 1-245, 1-246, 1-247, 1-248, 1-249, 1-253, 1-254, 1-255, 1-256, 1-257, 1-258, 1-259, 1-260, 1-261, 1-262, 1-263, 1-264, 1-265, 1-266, 1-269, 1-270, 2-141, 2-143, 2-145, 2-148, 2-151, 2-152, 2-158, 2-172, 2-173, 2-174, 2-175, 2-176, 2-177, 2-178, 2-179, 2-180, 2-181, 2-191, 2-195, 2-201, 2-203, 2-207, 2-243, 2-260, 2-261, 2-262, 2-269, 2-270, 2-281, 3-49 and 3-51.

Test Example 2

Insecticidal Test on Diamondback Moth (Plutella xylostella)

A piece of cabbage leaf was immersed for 30 seconds in a liquid chemical prepared by diluting a test compound to a prescribed concentration. After air-drying, the piece was put into a 7-cm polyethylene cup and second-instar larvae of diamondback moths were released thereinto. The polyethylene cups were set in an isothermal chamber thermostated at 25°C. From the release 6 days later, the dead and alive were counted. The test was carried out with two replications of 5 insects per a plot.

As a result of the above test, at a treatment concentration of 100 ppm, the following compounds showed 70% mortality or more: Compound Nos. 1-1, 1-8, 1-94, 1-143, 1-145, 1-146, 1-148, 1-149, 1-150, 1-151, 1-152, 1-158, 1-166, 1-176, 1-180, 1-191, 1-195, 1-201, 1-202, 1-203, 1-204, 1-205, 1-207, 1-241, 1-242, 1-243, 1-244, 1-245, 1-246, 1-247, 1-248, 1-249, 1-253, 1-254, 1-255, 1-256, 1-257, 1-258, 1-259, 1-260, 1-261, 1-262, 1-263, 1-264, 1-265, 1-266, 1-269, 1-270, 2-141, 2-143, 2-145, 2-148, 2-151, 2-152, 2-158, 2-172, 2-173, 2-174, 2-175, 2-176, 2-177, 2-178, 2-179, 2-180, 2-181, 2-191, 2-195, 2-201, 2-203, 2-207, 2-243, 2-260, 2-261, 2-262, 2-269, 2-270, 2-281, 3-49 and 3-51.

Test Example 3

Insecticidal Test on Western Flower Thrip (Franklinella occidentalis)

Into a plastic cup (diameter: 5 cm, height: 5 cm) was poured 1% agar gel, and the reverse side of a primary leaf of common bean cut with a diameter of 4 . 5 cm was placed upward to prepare leaf disc. 3 adult female insects that were already mated were released thereinto and the resulting material was lidded to oviposit for 2 days. Then, adult female insects were taken away and 4 days thereafter, the number of larvae on the leaf disc was counted and a chemical to the prescribed concentration was dispersed thereon using a vertical sprayer. 3 days after the dispersion, the surviving insects were counted. The adjusted mortality was calculated according to the following equation.

$$\text{Adjusted Mortality} = 100 \times Ta \times Cb / (Tb \times Ca)$$

Ta: the number of surviving insects after the dispersion in a treated plot
Tb: the number of surviving insects before the dispersion in a treated plot
Ca: the number of surviving insects after the dispersion in an untreated plot
Cb: the number of surviving insects before the dispersion in an untreated plot

As a result of the above test, at a treatment concentration of 300 ppm, the following compounds showed 30% adjusted mortality or less: Compound Nos. 1-143, 1-145, 1-148, 1-158, 1-176, 1-180, 1-195, 1-243, 1-244, 1-245, 2-141, 2-143, 2-158, 2-176, 2-177, 2-178, 2-179, 2-180, 2-181 2-243 and 2-281.

Test Example 4

Insecticidal Test on Melon Thrip (Thrips palmi)

Into a plastic cup (diameter: 5 cm, height: 5 cm) was poured 1% agar gel, and the reverse side of a cucumber leaf cut with a diameter of 4.5 cm was placed upward to prepare leaf disc. A chemical to the prescribed concentration was dispersed thereon using a vertical sprayer. After air-drying, 5 adult melon thrips were released thereinto and the resulting material was lidded. 3 days thereafter, the number of surviving insects was counted and the mortality was calculated.
As a result of the above test, at a treatment concentration of 300 ppm, the following compounds showed 70% mortality or more: Compound Nos. 1-143, 1-145, 1-158, 2-141 and 2-158.

Test Example 5

Insecticidal Test on Onion Thrip (Thrips tabaci)

A chemical to the prescribed concentration was dispersed on onion seedlings, air-dried, and then both the seedlings and 5 adult insects of onion thrips were put into a glass test tube (diameter; 3 cm, height: 10 cm). The resulting material was lidded. 3 days thereafter, the number of surviving insects was counted and the mortality was calculated.
As a result of the above test, at a treatment concentration of 300 ppm, the following compounds showed 70% mortality or more: Compound Nos. 1-143, 1-145, 1-148, 1-158, 1-243, 1-245, 2-141, 2-143 and 2-158.

Comparative Example 1

Insecticidal Test using N-(4-heptafluoroisopropyl-2-methyl)phenyl 3-(2-iodobenzoylamino)benzamide (Compound A) and N-(2,6-dimethyl-4-trifluoromethyl)phenyl 3-(benzoylamino)benzamide (Compound B)
The title compounds A and B were provided as chemicals for use in Test Examples 1 to 5, but insecticidal activity under the same conditions could not be confirmed.

## Claims

1. A compound represented by the general formula (1),

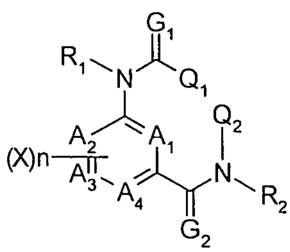

(1)

wherein, in the formula, $A_1$, $A_2$, $A_3$ and $A_4$ each independently represents a carbon atom, a nitrogen atom or an oxidized nitrogen atom;

$R_1$ and $R_2$ are each independently a hydrogen atom, a C1-C4 alkyl group or a C1-C4 alkylcarbonyl group;
$G_1$ and $G_2$ are each independently an oxygen atom or a sulfur atom;
Xs are each independently a hydrogen atom, a halogen atom or a trifluoromethyl group;
n is an integer of 0 to 4;
$Q_1$ is a phenyl group,
a substituted phenyl group having one or more substituents which may be the same or different (the substituents selected from a halogen atom, a C1-C4 alkyl group, a C1-C4 haloalkyl group, a C2-C4 alkenyl group, a C2-C4

haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group,- a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group),
a heterocyclic group (The heterocyclic group herein represents a pyridyl group, a pyridine N-oxide group, a pyrimidinyl group, a pyridazyl group, a pyrazyl group, a furyl group, a thienyl group, an oxazolyl group, an isoxazolyl group, an oxadiazolyl group, a thiazolyl group, an isothiazolyl group, an imidazolyl group, a triazolyl group, a pyrrole group, a pyrazolyl group or a tetrazolyl group.), or
a substituted heterocyclic group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group) (The heterocyclic group represents the same as those described above.); and
$Q_2$ is represented by the general formula (2) or (3),

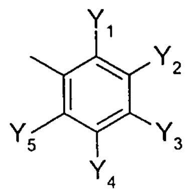
(2)

wherein, in the formula, $Y_1$ represents a C1-C4 alkoxy group, a C1-C4 haloalkoxy group, a C1-C3 haloalkylthio group, a C1-C3 haloalkylsulfinyl group or a C1-C3 haloalkylsulfonyl group; $Y_5$ represents a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_3$ represents a C2-C6 perfluoroalkyl group, a C1-C6 perfluoroalkylthio group, a C1-C6 perfluoroalkylsulfinyl group or a C1-C6 perfluoroalkylsulfonyl group; and $Y_2$ and $Y_4$ each independently represents a hydrogen atom, a halogen atom or a C1-C4 alkyl group; or

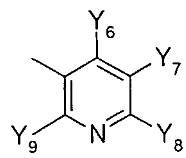
(3)

wherein, in the formula, $Y_6$ and $Y_9$ each independently represents a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_8$ represents a C1-C4 haloalkoxy group, a C2-C6 perfluoroalkyl group, a C1-C6 perfluoroalkylthio group, a C1-C6 perfluoroalkylsulfinyl group or a C1-C6 perfluoroalkylsulfonyl group; and $Y_7$ represents a hydrogen atom, a halogen atom or a C1-C4 alkyl group (Herein, at least one of $Y_6$ and $Y_9$ must represent a C1-C4 alkoxy group, a C1-C4 haloalkoxy group, a C1-C3 haloalkylthio group, a C1-C3 haloalkylsulfinyl group or a C1-C3 haloalkylsulfonyl group.).

**2.** A compound represented by the general formula (1),

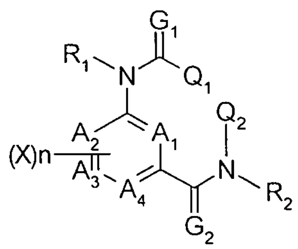 (1)

wherein, in the formula, $A_1$, $A_2$, $A_3$ and $A_4$ each independently represents a carbon atom, a nitrogen atom or an oxidized nitrogen atom;

$R_1$ and $R_2$ are each independently a hydrogen atom, a C1-C4 alkyl group or a C1-C4 alkylcarbonyl group;
$G_1$ and $G_2$ are each independently an oxygen atom or a sulfur atom;
Xs are each independently a hydrogen atom, a halogen atom or a trifluoromethyl group;
n represents an integer of 0 to 4;
$Q_1$ is a phenyl group,
a substituted phenyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group),
a heterocyclic group (The heterocyclic group herein represents a pyridyl group, a pyridine N-oxide group, a pyrimidinyl group, a pyridazyl group, a pyrazyl group, a furyl group, a thienyl group, an oxazolyl group, an isoxazolyl group, an oxadiazolyl group, a thiazolyl group, an isothiazolyl group, an imidazolyl group, a triazolyl group, a pyrrole group, a pyrazolyl group or a tetrazolyl group.), or
a substituted heterocyclic group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group) (The heterocyclic group represents the same as those described above.); and
$Q_2$ is represented by the general formula (2) or (3),

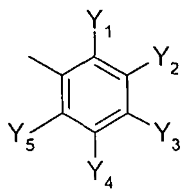 (2)

wherein, in the formula, $Y_1$ represents a C1-C4 alkoxy group, a C1-C4 haloalkoxy group, a C1-C3 haloalkylthio group, a C1-C3 haloalkylsulfinyl group or a C1-C3 haloalkylsulfonyl group; $Y_5$ represents a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_3$ represents a C2-C6 perfluoroalkyl group, a C1-C6 perfluoroalkylthio group, a C1-C6 perfluoroalkylsulfinyl group or a C1-C6 perfluoroalkylsulfonyl group; and $Y_2$ and $Y_4$ each independently represent a hydrogen atom, a halogen atom or a C1-C4 alkyl group, or

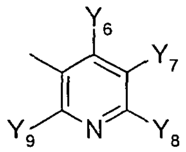

(3)

wherein, in the formula, $Y_6$ and $Y_9$ each independently represents a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_8$ represents a C1-C4 haloalkoxy group, a C2-C6 perfluoroalkyl group, a C1-C6 perfluoroalkylthio group, a C1-C6 perfluoroalkylsulfinyl group or a C1-C6 perfluoroalkylsulfonyl group; and $Y_7$ represents a hydrogen atom, a halogen atom or a C1-C4 alkyl group (Herein, at least one of $Y_6$ and $Y_9$ must represent a C1-C4 alkoxy group, a C1-C4 haloalkoxy group, a C1-C3 haloalkylthio group, a C1-C3 haloalkylsulfinyl group or a C1-C3 haloalkylsulfonyl group.),
whereas such compounds are excluded, wherein $Y_1$ represents a trifluoromethylthio group; $Y_2$ and $Y_4$ represent hydrogen atoms; $Y_3$ represents a heptafluoroisopropyl group; $Y_5$ represents a bromine atom; X represents a hydrogen atom; $G_1$ and $G_2$ represent oxygen atoms; $R_1$ and $R_2$ represent hydrogen atoms; and $Q_1$ represents an unsubstituted phenyl group.

**3.** The compound according to claim 1 or 2, represented by the general formula (1a) in which $A_1$, $A_2$, $A_3$ and $A_4$ in the general formula (1) are all carbon atoms,

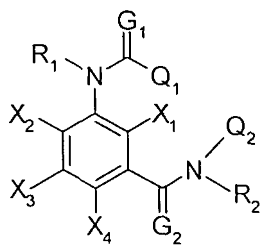

(1a)

wherein, in the formula, when any one of $R_1$ and $R_2$ is a hydrogen atom, the other one is a C1-C4 alkyl group or a C1-C4 alkylcarbonyl group, or both of $R_1$ and $R_2$ are C1-C4 alkyl groups or C1-C4 alkylcarbonyl groups;

$G_1$ and $G_2$ are each independently an oxygen atom or a sulfur atom;
$X_1$, $X_2$, $X_3$ and $X_4$ are each independently a hydrogen atom, a halogen atom or a trifluoromethyl group;
$Q_1$ is a phenyl group,
a substituted phenyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group),
a heterocyclic group (The heterocyclic group herein represents a pyridyl group, a pyridine N-oxide group, a pyrimidinyl group, a pyridazyl group, a pyrazyl group, a furyl group, a thienyl group, an oxazolyl group, an isoxazolyl group, an oxadiazolyl group, a thiazolyl group, an isothiazolyl group, an imidazolyl group, a triazolyl group, a pyrrole group, a pyrazolyl group or a tetrazolyl group.), or
a substituted heterocyclic group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino

group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group) (The heterocyclic group represents the same as those described above.); and

$Q_2$ is represented by the general formula (2) or (3),

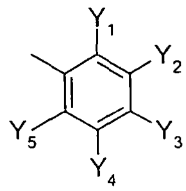

(2)

wherein, in the formula, $Y_1$ represents a C1-C3 haloalkylthio group, a C1-C3 haloalkylsulfinyl group or a C1-C3 haloalkylsulfonyl group; $Y_5$ represents a halogen atom, a C1-C4 alkyl group, a C1-C4 haloalkyl group, a C1-alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_3$ represents a C2-C6 perfluoroalkyl group, a C1-C6 perfluoroalkylthio group, a C1-C6 perfluoroalkylsulfinyl group or a C1-C6 perfluoroalkylsulfonyl group; and $Y_2$ and $Y_4$ each independently represents a hydrogen atom, a halogen atom or a C1-C4 alkyl group, or

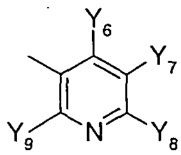

(3)

wherein, in the formula, $Y_6$ and $Y_9$ each independently represent a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_8$ represents a C1-C4 haloalkoxy group, a C2-C6 perfluoroalkyl group, a C1-C6 perfluoroalkylthio group, a C1-C6 perfluoroalkylsulfinyl group or a C1-C6 perfluoroalkylsulfonyl group; and $Y_7$ represents a hydrogen atom, a halogen atom or a C1-C4 alkyl group (Herein, at least one of $Y_6$ and $Y_9$ must represent a C1-C3 haloalkylthio group, a C1-C3 haloalkylsulfinyl group or a C1-C3 haloalkylsulfonyl group.).

**4.** The compound according to claim 3, wherein $Q_2$ is represented by the general formula (2),

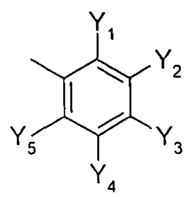

(2)

wherein, in the formula, $Y_1$ represents a C1-C3 haloalkylthio group, a C1-C3 haloalkylsulfinyl group or a C1-C3 haloalkylsulfonyl group; $Y_5$ represents a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_3$ represents a C2-C6 perfluoroalkyl group, a C1-C6 perfluoroalkylthio group, a C1-C6 perfluoroalkylsulfinyl group or a C1-C6 perfluoroalkylsulfonyl group; and $Y_2$ and $Y_4$ represent each independently a hydrogen atom, a halogen atom or a C1-C4 alkyl group.

**5.** The compound according to claim 4, wherein $Q_1$ represents a phenyl group,

a substituted phenyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group),

a pyridyl group, or

a substituted pyridyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group).

**6.** The compound according to claim 1 or 2, represented by the general formula (1a) in which $A_1$, $A_2$, $A_3$ and $A_4$ in the general formula (1) are all carbon atoms,

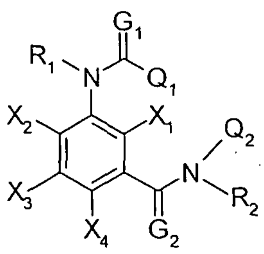

(1a)

wherein, in the formula, $R_1$ and $R_2$ are each independently a hydrogen atom, a C1-C4 alkyl group or a C1-C4 alkylcarbonyl group;

$G_1$ and $G_2$ are each independently an oxygen atom or a sulfur atom;

$X_1$, $X_2$, $X_3$ and $X_4$ are each independently a hydrogen atom, a halogen atom or a trifluoromethyl group;

$Q_1$ is a substituted phenyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group),

a heterocyclic group (The heterocyclic group herein represents a pyridyl group, a pyridine N-oxide group, a pyrimidinyl group, a pyridazyl group, a pyrazyl group, a furyl group, a thienyl group, an oxazolyl group, an isoxazolyl group, an oxadiazolyl group, a thiazolyl group, an isothiazolyl group, an imidazolyl group, a triazolyl group, a pyrrole group, a pyrazolyl group or a tetrazolyl group.), or

a substituted heterocyclic group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group) (The heterocyclic group represents the same as those described above.); and

$Q_2$ is represented by the general formula (2) or (3),

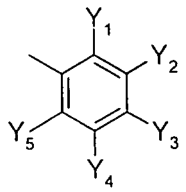

(2)

wherein, in the formula, $Y_1$ represents a C1-C3 haloalkylthio group, a C1-C3 haloalkylsulfinyl group or a C1-C3 haloalkylsulfonyl group; $Y_5$ represents a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_3$ represents a C2-C6 perfluoroalkyl group, a C1-C6 perfluoroalkylthio group, a C1-C6 perfluoroalkylsulfinyl group or a C1-C6 perfluoroalkylsulfonyl group; and $Y_2$ and $Y_4$ each independently represents a hydrogen atom, a halogen atom or a C1-C4 alkyl group, or

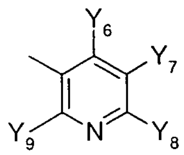

(3)

wherein, in the formula, $Y_6$ and $Y_9$ each independently represent a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_8$ represents a C1-C4 haloalkoxy group, a C2-C6 perfluoroalkyl group, a C1-C6 perfluoroalkylthio group, a C1-C6 perfluoroalkylsulfinyl group or a C1-C6 perfluoroalkylsulfonyl group; and $Y_7$ represents a hydrogen atom, a halogen atom or a C1-C4 alkyl group (Herein, at least one of $Y_6$ and $Y_9$ must represent a C1-C3 haloalkylthio group, a C1-C3 haloalkylsulfinyl group or a C1-C3 haloalkylsulfonyl group.).

**7.** The compound according to claim 6, wherein $Q_1$ represents a substituted phenyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group),

a pyridyl group, or
a pyridyl group having one or more substituents which may be the same or different (the substituents selected a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group); and
$Q_2$ is represented by the general formula (2),

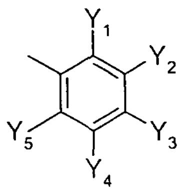

(2)

wherein, in the formula, $Y_1$ represents a C1-C3 haloalkylthio group, a C1-C3 haloalkylsulfinyl group or a C1-C3 haloalkylsulfonyl group; $Y_5$ represents a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_3$ represents a C2-C6 perfluoroalkyl group, a C1-C6 perfluoroalkylthio group, a C1-C6 perfluoroalkylsulfinyl group or a C1-C6 perfluoroalkylsulfonyl group; and $Y_2$ and $Y_4$ each independently represents a hydrogen atom, a halogen atom or a C1-C4 alkyl group.

**8.** The compound according to claim 1 or 2; represented by the general formula (1a) in which $A_1$, $A_2$, $A_3$ and $A_4$ in the general formula (1) are all carbon atoms,

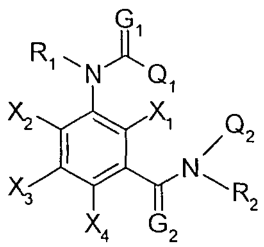

(1a)

wherein, in the formula, $R_1$ and $R_2$ are each independently a hydrogen atom, a C1-C4 alkyl group or a C1-C4 alkylcarbonyl group;

$G_1$ and $G_2$ are each independently an oxygen atom or a sulfur atom;
$X_1$, $X_2$, $X_3$ and $X_4$ are each independently a hydrogen atom, a halogen atom or a trifluoromethyl group;
$Q_1$ is a phenyl group,
a substituted phenyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group),
a heterocyclic group (The heterocyclic group herein represents a pyridyl group, a pyridine N-oxide group, a pyrimidinyl group, a pyridazyl group, a pyrazyl group, a furyl group, a thienyl group, an oxazolyl group, an isoxazolyl group, an oxadiazolyl group, a thiazolyl group, an isothiazolyl group, an imidazolyl group, a triazolyl group, a pyrrole group, a pyrazolyl group or a tetrazolyl group.), or
a substituted heterocyclic group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group) (The heterocyclic group represents the same as those described above.); and
$Q_2$ is represented by the general formula (2) or (3),

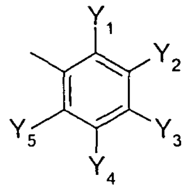

(2)

wherein, in the formula, $Y_1$ represents a C1-C3 haloalkylthio group, a C1-C3 haloalkylsulfinyl group or a C1-C3 haloalkylsulfonyl group; $Y_5$ represents a fluorine atom, a chlorine atom, an iodine atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_3$ represents a C2-C6 perfluoroalkyl group, a C1-C6 perfluoroalkylthio group, a C1-C6 perfluoroalkylsulfinyl group or a C1-C6 perfluoroalkylsulfonyl group; and $Y_2$ and $Y_4$ each independently represents a hydrogen atom, a halogen atom or a C1-C4 alkyl group, or

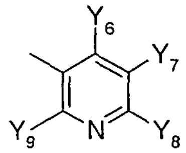

(3)

wherein, in the formula, $Y_6$ and $Y_9$ each independently representa halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_8$ represents a C1-C4 haloalkoxy group, a C2-C6 perfluoroalkyl group, a C1-C6 perfluoroalkylthio group, a C1-C6 perfluoroalkylsulfinyl group or a C1-C6 perfluoroalkylsulfonyl group; and $Y_7$ represents a hydrogen atom, a halogen atom or a C1-C4 alkyl group (Herein, at least one of $Y_6$ and $Y_9$ must represent a C1-C3 haloalkylthio group, a C1-C3 haloalkylsulfinyl group or a C1-C3 haloalkylsulfonyl group.).

**9.** The compound according to claim 8, wherein $Q_1$ represents a phenyl group,

a substituted phenyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a Cl-C4 alkoxycarbonyl group, an acetylamino group and a phenyl group),
a pyridyl group, or
a substituted pyridyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group); and
$Q_2$ is represented by the general formula (2),

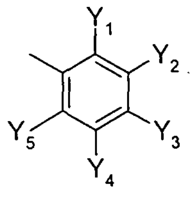

(2)

wherein, in the formula, $Y_1$ represents a C1-C3 haloalkylthio group, a C1-C3 haloalkylsulfinyl group or a C1-C3 haloalkylsulfonyl group; $Y_5$ represents a fluorine atom, a chlorine atom, an iodine atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_3$ represents a C2-C6 perfluoroalkyl group, a C1-C6 perfluoroalkylthio group, a C1-C6 perfluoroalkylsulfinyl group or a C1-C6 perfluoroalkylsulfonyl group; and $Y_2$ and $Y_4$ each independently represent a hydrogen atom, a halogen atom or a C1-C4 alkyl group.

**10.** The compound according to claim 1 or 2, represented by the general formula (1a) in which $A_1$, $A_2$, $A_3$ and $A_4$ in the general formula (1) are all carbon atoms,

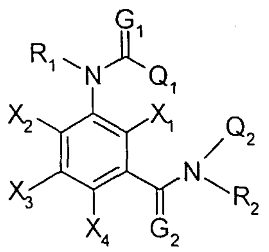

(1a)

wherein, in the formula, $R_1$ and $R_2$ are each independently a hydrogen atom, a C1-C4 alkyl group or a C1-C4 alkylcarbonyl group;

$G_1$ and $G_2$ are each independently an oxygen atom or a sulfur atom;
$X_1$, $X_2$, $X_3$ and $X_4$ are each independently a hydrogen atom, a halogen atom or a trifluoromethyl group;
$Q_1$ is a phenyl group,
a substituted phenyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group),
a heterocyclic group (The heterocyclic group herein represents a pyridyl group, a pyridine N-oxide group, a pyrimidinyl group, a pyridazyl group, a pyrazyl group, a furyl,group, a thienyl group, an oxazolyl group, an isoxazolyl group, an oxadiazolyl group, a thiazolyl group, an isothiazolyl group, an imidazolyl group, a triazolyl group, a pyrrole group, a pyrazolyl group or a tetrazolyl group.), or
a substituted heterocyclic group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group) (The heterocyclic group represents the same as those described above.); and

$Q_2$ is represented by the general formula (2) or (3),

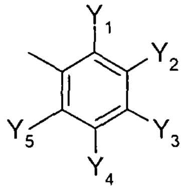

(2)

wherein, in the formula, $Y_1$ represents a C2-C3 haloalkylthio group, a C1-C3 haloalkylsulfinyl group or a C1-C3 haloalkylsulfonyl group; $Y_5$ represents a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_3$ represents a C2-C6 perfluoroalkyl group, a C1-C6 perfluoroalkylthio group, a C1-C6 perfluoroalkylsulfinyl group or a C1-C6 perfluoroalkylsulfonyl group; and $Y_2$ and $Y_4$ each independently represent a hydrogen atom, a halogen atom or a C1-C4 alkyl group; or

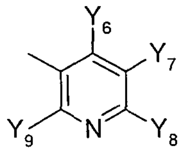

(3)

wherein, in the formula, $Y_6$ and $Y_9$ each independently represents a halogen atom, a C1-C4 alkyl group, a C1-C4 haloalkyl group, a C1-C4 alkoxy group, a C1-C4 haloalkoxy group, a C1-C3 alkylthio group, a C2-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_8$ represents a C1-C4 haloalkoxy group, a C2-C6 perfluoroalkyl group, a C1-C6 perfluoroalkylthio group, a C1-C6 perfluoroalkylsulfinyl group or a C1-C6 perfluoroalkylsulfonyl group; and $Y_7$ represents a hydrogen atom, a halogen atom or a C1-C4 alkyl group. (Herein, at least one of $Y_6$ and $Y_9$ must represent a C2-C3 haloalkylthio group, a C1-C3 haloalkylsulfinyl group or a C1-C3 haloalkylsulfonyl group.).

**11.** The compound according to claim 10, wherein $Q_1$ represents a phenyl group,

a substituted phenyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group),
a pyridyl group, or
a pyridyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group); and
$Q_2$ is represented by the general formula (2),

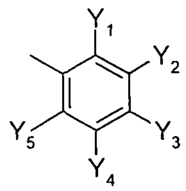

(2)

wherein, in the formula, $Y_1$ represents a C2-C3 haloalkylthio group, a C1-C3 haloalkylsulfinyl group or a C1-C3 haloalkylsulfonyl group; $Y_5$ represents a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_3$ represents a C2-C6 perfluoroalkyl group, a C1-C6 perfluoroalkylthio group, a C1-C6 perfluoroalkylsulfinyl group or a C1-C6 perfluoroalkylsulfonyl group; and $Y_2$ and $Y_4$ each independently represents a hydrogen atom, a halogen atom or a C1-C4 alkyl group.

**12.** The compound according to claim 1, represented by the general formula (1),

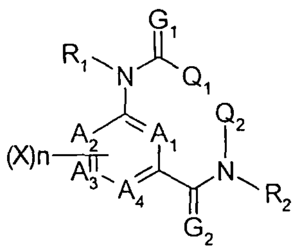

(1)

wherein, in the formula, $A_1$, $A_2$, $A_3$ and $A_4$ each independently represents a carbon atom, a nitrogen atom or an oxidized nitrogen atom;
when any one of $R_1$ and $R_2$ is a hydrogen atom, the other one is a C1-C4 alkyl group or a C1-C4 alkylcarbonyl group, or both of $R_1$ and $R_2$ are C1-C4 alkyl groups or C1-C4 alkylcarbonyl groups;

$G_1$ and $G_2$ are each independently an oxygen atom or a sulfur atom;
Xs are each independently a hydrogen atom, a halogen atom or a trifluoromethyl group;
n represents an integer of 0 to 4;
$Q_1$ is a phenyl group,
a substituted phenyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group),
a heterocyclic group (The heterocyclic group herein represents a pyridyl group, a pyridine N-oxide group, a pyrimidinyl group, a pyridazyl group, a pyrazyl group, a furyl group, a thienyl group, an oxazolyl group, an isoxazolyl group, an oxadiazolyl group, a thiazolyl group, an isothiazolyl group, an imidazolyl group, a triazolyl group, a pyrrole group, a pyrazolyl group or a tetrazolyl group.), or
a substituted heterocyclic group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group) (The heterocyclic group

represents the same as those described above.); and
$Q_2$ is represented by the general formula (2) or (3),

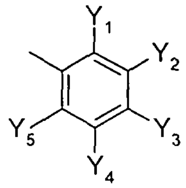
(2)

wherein, in the formula, $Y_1$ represents a C1-C4 alkoxy group or a C1-C4 haloalkoxy group; $Y_5$ represents a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_3$ represents a C2-C6 perfluoroalkyl group, a C1-C6 perfluoroalkylthio group, a C1-C6 perfluoroalkylsulfinyl group or a C1-C6 perfluoroalkylsulfonyl group; and $Y_2$ and $Y_4$ each independently represents a hydrogen atom, a halogen atom or a C1-C4 alkyl group, or

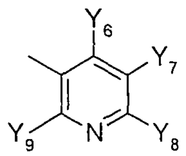
(3)

wherein, in the formula, $Y_6$ and $Y_9$ each independently represents a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_8$ represents a C1-C4 haloalkoxy group, a C2-C6 perfluoroalkyl group, a C1-C6 perfluoroalkylthio group, a C1-C6 perfluoroalkylsulfinyl group or a C1-C6 perfluoroalkylsulfonyl group; and $Y_7$ represents a hydrogen atom, a halogen atom or a C1-C4 alkyl group (Herein, at least one of $Y_6$ and $Y_9$ must represent a C1-C4 alkoxy group or a C1-C4 haloalkoxy group.).

**13.** The compound according to claim 12, represented by the general formula (1a) in which $A_1$, $A_2$, $A_3$ and $A_4$ in the general formula (1) are all carbon atoms,

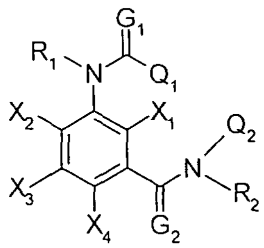
(1a)

wherein, in the formula, when any one of $R_1$ and $R_2$ is a hydrogen atom, the other one is a C1-C4 alkyl group or a C1-C4 alkylcarbonyl group, or both $R_1$ and $R_2$ are C1-C4 alkyl groups or C1-C4 alkylcarbonyl groups;

$G_1$ and $G_2$ are each independently an oxygen atom or a sulfur atom;
$X_1$, $X_2$, $X_3$ and $X_4$ are each independently a hydrogen atom, a halogen atom or a trifluoromethyl group;
$Q_1$ is a phenyl group,
a substituted phenyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3

haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group),
a heterocyclic group (The heterocyclic group herein represents a pyridyl group, a pyridine N-oxide group, a pyrimidinyl group, a pyridazyl group, a pyrazyl group, a furyl group, a thienyl group, an oxazolyl group, an isoxazolyl group, an oxadiazolyl group, a thiazolyl group, an isothiazolyl group, an imidazolyl group, a triazolyl group, a pyrrole group, a pyrazolyl group or a tetrazolyl group.), or
a substituted heterocyclic group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a Cl-C3 alkylsulfonyl group, a C1-C3 haloalkylsulfonyl group, an amino group, a Cl-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group) (The heterocyclic group represents the same as those described above.); and
$Q_2$ is represented by the general formula (2),

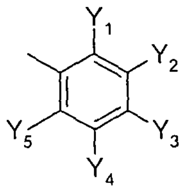
(2)

wherein, in the formula, $Y_1$ represents a C1-C4 haloalkoxy group; $Y_5$ represents a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_3$ represents a C2-C6 perfluoroalkyl group, a C1-C6 perfluoroalkylthio group, a C1-C6 perfluoroalkylsulfinyl group or a C1-C6 perfluoroalkylsulfonyl group; and $Y_2$ and $Y_4$ each independently represents a hydrogen atom, a halogen atom or a C1-C4 alkyl group.

**14.** The compound according to claim 13, wherein $Q_1$ represents a phenyl group,

a substituted phenyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group),
a pyridyl group, or
a substituted pyridyl group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group).

**15.** A compound represented by the general formula (4),

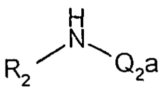 (4)

wherein, in the formula, $R_2$ represents a hydrogen atom, a C1-C4 alkyl group or a C1-C4 alkylcarbonyl group; and

$Q_2$a is represented by the general formula (2), (3) or (5),

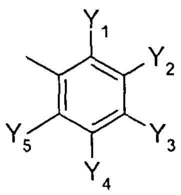 (2)

wherein, in the formula, $Y_1$ represents a C1-C4 alkoxy group, a C1-C4 haloalkoxy group, a C1-C3 haloalkylthio group, a C1-C3 haloalkylsulfinyl group or a C1-C3 haloalkylsulfonyl group; $Y_5$ represents a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_3$ represents a C2-C6 perfluoroalkyl group, a C1-C6 perfluoroalkylthio group, a C1-C6 perfluoroalkylsulfinyl group or a C1-C6 perfluoroalkylsulfonyl group; and $Y_2$ and $Y_4$ each independently represents a hydrogen atom, a halogen atom or a C1-C4 alkyl group;

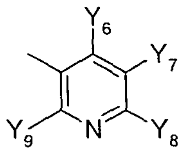 (3)

wherein, in the formula, $Y_6$ and $Y_9$ each independently represents a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_8$ represents a C1-C4 haloalkoxy group, a C2-C6 perfluoroalkyl group, a C1-C6 perfluoroalkylthio group, a C1-C6 perfluoroalkylsulfinyl group or a C1-C6 perfluoroalkylsulfonyl group; and $Y_7$ represents a hydrogen atom, a halogen atom or a C1-C4 alkyl group (Herein, at least one of $Y_6$ and $Y_9$ must represent a C1-C4 alkoxy group, a C1-C4 haloalkoxy group, a C1-C3 haloalkylthio group, a C1-C3 haloalkylsulfinyl group or a C1-C3 haloalkylsulfonyl group.); or

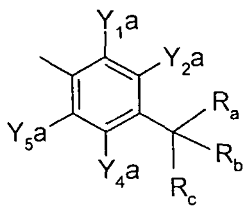 (5)

wherein, in the formula, $R_a$ and $R_b$ are each independently a fluorine atom or a C1-C4 perfluoroalkyl group; $R_c$ is a hydroxy group, -O-$R_d$ ($R_d$ represents a C1-C3 alkyl group, a C1-C3 haloalkyl group, a C1-C3 alkylsulfonyl group, a C1-C3 haloalkylsulfonyl group, an arylsulfonyl group, a C1-C4 alkylcarbonyl group or a C1-C4 haloalkylcarbonyl group.), a chlorine atom, a bromine atom or an iodine atom; $Y_1$a represents a C1-C4 alkoxy group, a C1-C4 haloalkoxy group, a C1-C3 haloalkylthio group, a C1-C3 haloalkylsulfinyl group or a C1-C3 haloalkylsulfonyl group; $Y_5$a represents a hydrogen atom, a halogen atom, a C1-C4 alkyl group, a C1-C4 haloalkyl group,

a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; and $Y_2$a and $Y_4$a each independently represents a hydrogen atom, a halogen atom or a C1-C4 alkyl group.

**16.** A compound represented by the general formula (7),

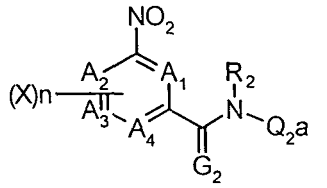

(7)

wherein, in the formula, $A_1$, $A_2$, $A_3$ and $A_4$ each independently represents a carbon atom, a nitrogen atom or an oxidized nitrogen atom;

$R_2$ represents a hydrogen atom, a C1-C4 alkyl group or a C1-C4 alkylcarbonyl group;
$G_2$ represents an oxygen atom or a sulfur atom;
X represents a hydrogen atom, a halogen atom or a trifluoromethyl group;
n represents an integer of 0 to 4; and
$Q_2$a is represented by the general formula (2), (3) or (5),

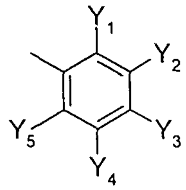

(2)

wherein, in the formula, $Y_1$ represents a C1-C4 alkoxy group, a C1-C4 haloalkoxy group, a C1-C3 haloalkylthio group, a C1-C3 haloalkylsulfinyl group or a C1-C3 haloalkylsulfonyl group; $Y_5$ represents a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_3$ represents a C2-C6 perfluoroalkyl group, a C1-C6 perfluoroalkylthio group, a C1-C6 perfluoroalkylsulfinyl group or a C1-C6 perfluoroalkylsulfonyl group; and $Y_2$ and $Y_4$ each independently represents a hydrogen atom, a halogen atom or a C1-C4 alkyl group;

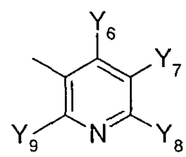

(3)

wherein, in the formula, $Y_6$ and $Y_9$ each independently represents a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_8$ represents a C1-C4 haloalkoxy group, a C2-C6 perfluoroalkyl group, a C1-C6 perfluoroalkylthio group, a C1-C6 perfluoroalkylsulfinyl group or a C1-C6 perfluoroalkylsulfonyl group; and $Y_7$ represents a hydrogen atom, a halogen atom or a C1-C4 alkyl group (Herein, at least one of $Y_6$ and $Y_9$ must represent a C1-C4 alkoxy group, a C1-C4 haloalkoxy group, a C1-C3 haloalkylthio group, a C1-C3 haloalkylsulfinyl group or a C1-C3 haloalkylsulfonyl group.); or

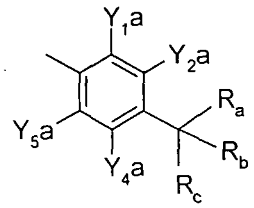

(5)

wherein, in the formula, $R_a$ and $R_b$ are each independently a fluorine atom or a C1-C4 perfluoroalkyl group; $R_c$ is a hydroxy group, -O-$R_d$ ($R_d$ represents a C1-C3 alkyl group, a C1-C3 haloalkyl group, a C1-C3 alkylsulfonyl group, a C1-C3 haloalkylsulfonyl group, an arylsulfonyl group, a C1-C4 alkylcarbonyl group or a C1-C4 haloalkylcarbonyl group.), a chlorine atom, a bromine atom or an iodine atom; $Y_1$a represents a C1-C4 alkoxy group, a C1-C4 haloalkoxy group, a C1-C3 haloalkylthio group, a C1-C3 haloalkylsulfinyl group or a C1-C3 haloalkylsulfonyl group; $Y_5$a represents a hydrogen atom, a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; and $Y_2$a and $Y_4$a each independently represents a hydrogen atom, a halogen atom or a C1-C4 alkyl group.

**17.** A compound represented by the general formula (9),

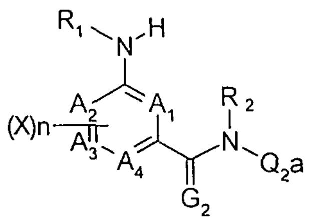

(9)

wherein, in the formula, $A_1$, $A_2$, $A_3$ and $A_4$ each independently represents a carbon atom, a nitrogen atom or an oxidized nitrogen atom;

$R_1$ and $R_2$ are each independently a hydrogen atom, a C1-C4 alkyl group or a C1-C4 alkylcarbonyl group;
$G_2$ is an oxygen atom or a sulfur atom;
X is a hydrogen atom, a halogen atom or a trifluoromethyl group;
n represents an integer of 0 to 4; and
$Q_2$a is represented by the general formula (2), (3) or (5),

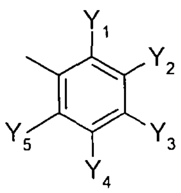

(2)

wherein, in the formula, $Y_1$ represents a C1-C4 alkoxy group, a C1-C4 haloalkoxy group, a C1-C3 haloalkylthio group, a C1-C3 haloalkylsulfinyl group or a C1-C3 haloalkylsulfonyl group; $Y_5$ represents a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_3$ represents a C2-C6 perfluoroalkyl group, a C1-C6 perfluoroalkylthio group, a C1-C6 perfluoroalkylsulfinyl group or a C1-C6 perfluoroalkylsulfonyl group; and $Y_2$ and $Y_4$ each independently represents a hydrogen atom, a halogen atom or a C1-C4 alkyl group;

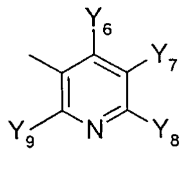

(3)

wherein, in the formula, $Y_6$ and $Y_9$ each independently represents a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; $Y_8$ represents a C1-C4 haloalkoxy group, a C2-C6 perfluoroalkyl group, a C1-C6 perfluoroalkylthio group, a C1-C6 perfluoroalkylsulfinyl group or a C1-C6 perfluoroalkylsulfonyl group; and $Y_7$ represents a hydrogen atom, a halogen atom or a C1-C4 alkyl group (Herein, at least one of $Y_6$ and $Y_9$ must represent a C1-C4 alkoxy group, a C1-C4 haloalkoxy group, a C1-C3 haloalkylthio group, a C1-C3 haloalkylsulfinyl group or a C1-C3 haloalkylsulfonyl group.); or

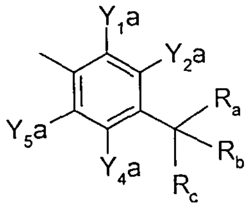

(5)

wherein, in the formula, $R_a$ and $R_b$ are each independently a fluorine atom or a C1-C4 perfluoroalkyl group; $R_c$ is a hydroxy group, $-O-R_d$ ($R_d$ represents a C1-C3 alkyl group, a C1-C3 haloalkyl group, a C1-C3 alkylsulfonyl group, a C1-C3 haloalkylsulfonyl group, an arylsulfonyl group, a C1-C4 alkylcarbonyl group or a C1-C4 haloalkylcarbonyl group.), a chlorine atom, a bromine atom or an iodine atom; $Y_1$a represents a C1-C4 alkoxy group, a C1-C4 haloalkoxy group, a C1-C3 haloalkylthio group, a C1-C3 haloalkylsulfinyl group or a C1-C3 haloalkylsulfonyl group; $Y_5$a represents a hydrogen atom, a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; and $Y_2$a and $Y_4$a each independently represents a hydrogen atom, a halogen atom or a C1-C4 alkyl group.

**18.** A compound represented by the general formula (10),

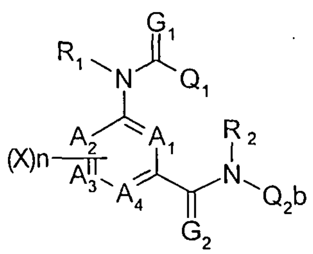

(10)

wherein, in the formula, $A_1$, $A_2$, $A_3$ and $A_4$ each independently represent a carbon atom, a nitrogen atom or an oxidized nitrogen atom;

$R_1$ and $R_2$ are each independently a hydrogen atom, a C1-C4 alkyl group or a C1-C4 alkylcarbonyl group;
$G_1$ and $G_2$ are each independently an oxygen atom or a sulfur atom;
X is a hydrogen atom, a halogen atom or a trifluoromethyl group;
n represents an integer of 0 to 4;
$Q_1$ is a phenyl group,
a substituted phenyl group having one or more substituents which may be the same or different (the substituents selected from a halogen atom, a C1-C4 alkyl group, a C1-C4 haloalkyl group, a C2-C4 alkenyl group, a C2-C4

haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group),

a heterocyclic group (The heterocyclic group herein represents a pyridyl group, a pyridine N-oxide group, a pyrimidinyl group, a pyridazyl group, a pyrazyl group, a furyl group, a thienyl group, an oxazolyl group, an isoxazolyl group, an oxadiazolyl group, a thiazolyl group, an isothiazolyl group, an imidazolyl group, a triazolyl group, a pyrrole group, a pyrazolyl group or a tetrazolyl group.), or

a substituted heterocyclic group having one or more substituents which may be the same or different (the substituents selected from a halogen atom, a C1-C4 alkyl group, a C1-C4 haloalkyl group, a C2-C4 alkenyl group, a C2-C4 haloalkenyl group, a C2-C4 alkynyl group, a C2-C4 haloalkynyl group, a C3-C6 cycloalkyl group, a C3-C6 halocycloalkyl group, a C1-C3 alkoxy group, a C1-C3 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group, an amino group, a C1-C4 alkylamino group, a di C1-C4 alkylamino group, a cyano group, a nitro group, a hydroxy group, a C1-C4 alkylcarbonyl group, a C1-C4 alkylcarbonyloxy group, a C1-C4 alkoxycarbonyl group, an acetylamino group and a phenyl group) (The heterocyclic group represents the same as those described above.); and

$Q_2b$ is represented by the general formula (5),

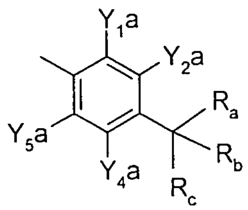
(5)

wherein, in the formula, $R_a$ and $R_b$ are each independently a fluorine atom or a C1-C4 perfluoroalkyl group; $R_c$ is a hydroxy group, -O-$R_d$ ($R_d$ represents a C1-C3 alkyl group, a C1-C3 haloalkyl group, a C1-C3 alkylsulfonyl group, a C1-C3 haloalkylsulfonyl group, an arylsulfonyl group, a C1-C4 alkylcarbonyl group or a C1-C4 haloalkylcarbonyl group.), a chlorine atom, a bromine atom or an iodine atom; $Y_1a$ represents a C1-C4 alkoxy group, a C1-C4 haloalkoxy group, a C1-C3 haloalkylthio group, a C1-C3 haloalkylsulfinylgroup or a C1-C3 haloalkylsulfonyl group; $Y_5a$ represents a hydrogen atom, a halogen atom, a C1-C4 alkyl group, a C1-C4 haloalkyl group, a C1-C4 alkoxy group, a C1-C4 haloalkoxy group, a C1-C3 alkylthio group, a C1-C3 haloalkylthio group, a C1-C3 alkylsulfinyl group, a C1-C3 haloalkylsulfinyl group, a C1-C3 alkylsulfonyl group, a C1-C3 haloalkylsulfonyl group or a cyano group; and $Y_2a$ and $Y_4a$ each independently represent a hydrogen atom, a halogen atom or a C1-C4 alkyl group.

**19.** An insecticide comprising the compound as described in any one of claims 1 to 14 as an active ingredient.

**20.** A method for application of a chemical, which comprises applying an effective amount of the compound as described in any one of claims 1 to 14 on useful crops or soil for the purpose of protecting useful crops from harmful organisms.

**INTERNATIONAL SEARCH REPORT**

International application No.
PCT/JP2006/312318

A. CLASSIFICATION OF SUBJECT MATTER
*C07C237/42*(2006.01)i, *A01N37/24*(2006.01)i, *A01N41/10*(2006.01)i, *A61P7/04* (2006.01)i, *C07C317/40*(2006.01)i, *C07C323/42*(2006.01)i, *C07D213/82* (2006.01)i

According to International Patent Classification (IPC) or to both national classification and IPC

B. FIELDS SEARCHED

Minimum documentation searched (classification system followed by classification symbols)
C07C237/42, A01N37/24, A01N41/10, A61P7/04, C07C317/40, C07C323/42, C07D213/82

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | WO 2005/021488 A1 (Mitsui Chemicals, Inc.), 10 March, 2005 (10.03.05), Claims; page 26 | 15-17<br>1-14,18-20 |
| Y | JP 2003-528070 A (E.I. Du Pont De Nemours & Co.), 24 September, 2003 (24.09.03), Claims | 1-14,18-20 |
| A | JP 2005-504084 A (E.I. Du Pont De Nemours & Co.), 10 February, 2005 (10.02.05), Claims | 1-20 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 01 September, 2006 (01.09.06) | 12 September, 2006 (12.09.06) |
| Name and mailing address of the ISA/ Japanese Patent Office | Authorized officer |
| Facsimile No. | Telephone No. |

Form PCT/ISA/210 (second sheet) (April 2005)

**INTERNATIONAL SEARCH REPORT**
Information on patent family members

International application No.
PCT/JP2006/312318

| | | |
|---|---|---|
| WO 2005/021488 A1 | 2005.03.10 | (Family: none) |
| JP 2003-528070 A | 2003.09.24 | WO 01/70671 A2<br>AU 200150946 A<br>EP 1265850 A2<br>BR 200109757 A<br>KR 2002084234 A<br>HU 200300263 A2<br>CN 1419537 A<br>NZ 520728 A<br>US 2003/0229050 A1<br>MX 2002009207 A1<br>ZA 200206148 A<br>US 6747047 B2<br>US 2004/0142984 A1<br>IN 200201167 P3<br>AU 2001250946 B2<br>US 6995178 B2<br>US 2006/0079561 A1 |
| JP 2005-504084 A | 2005.02.10 | WO 03/026415 A2<br>EP 1427705 A2<br>BR 200212799 A<br>AU 2002334581 A1<br>US 2004/0235959 A1<br>MX 2004002649 A1<br>CN 1555364 A<br>IN 200400088 P3 |

Form PCT/ISA/210 (patent family annex) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 200055120 A
- US 6548514 B
- WO 20007980 A
- US 2002032238 A


### Non-patent literature cited in the description


- *J. Am. Chem. Soc.,* 1967, 5012
- *Tetrahedron Lett.,* 1994, 4955